(19) Europäisches Patentamt / European Patent Office / Office européen des brevets

(11) **EP 2 578 200 B1**

(12) # EUROPÄISCHE PATENTSCHRIFT

(45) Veröffentlichungstag und Bekanntmachung des Hinweises auf die Patenterteilung:
**28.03.2018 Patentblatt 2018/13**

(51) Int Cl.:
***A61K 6/00*** $^{(2006.01)}$ ***A61B 5/00*** $^{(2006.01)}$

(21) Anmeldenummer: **11183887.6**

(22) Anmeldetag: **04.10.2011**

(54) **Zusammensetzungen zum Infiltrieren und/oder Versiegeln von Zahnhartsubstanz und entsprechende Verfahren**

Compounds for infiltrating and/or sealing of dental hard substance and method

Compositions pour l'infiltration et/ou le scellement de la matière dentaire dure et procédé correspondant

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**10.04.2013 Patentblatt 2013/15**

(73) Patentinhaber: **VOCO GmbH**
**27472 Cuxhaven (DE)**

(72) Erfinder:
- **Plaumann, Manfred Thomas**
  **27472 Cuxhaven (DE)**
- **Maletz, Reinhard Dr.,**
  **27472 Cuxhaven (DE)**
- **Stepputtis, Manfred Dr.,**
  **27449 Kutenholz (DE)**
- **Barg, Andree**
  **21762 Otterndorf (DE)**
- **Blömker, Tobias Dr.,**
  **22527 Hamburg (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**EP-B1- 0 969 789** **WO-A2-2007/028159**
**DE-A1- 3 522 006** **DE-A1- 19 825 021**
**US-A1- 2010 016 464** **US-B1- 6 670 499**

- **FELIX KRAUSE ET AL: "Effects of composite fissure sealants on IR laser fluorescence measurements", LASERS IN MEDICAL SCIENCE, SPRINGER-VERLAG, LO, vol. 23, no. 2, 23 May 2007 (2007-05-23), pages 133-139, XP019566752, ISSN: 1435-604X**

Bemerkungen:
Die Akte enthält technische Angaben, die nach dem Eingang der Anmeldung eingereicht wurden und die nicht in dieser Patentschrift enthalten sind.

**EP 2 578 200 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Zusammensetzungen, die ein organisches Bindemittelsystem und darin dispergiert ein Füllstoffsystem umfassen. Diese Zusammensetzungen finden erfindungsgemäß Anwendung in einem Verfahren zum Infiltrieren und/oder Versiegeln von Zahnhartsubstanz und zur Detektion von Karies im infiltrierten Bereich bzw. unter der Versiegelung mittels Fluoreszenz sowie zur Bestimmung von Veränderungen des Infiltrationszustands bzw. der Versiegelung mittels Fluoreszenz. Ein weiterer Aspekt der vorliegenden Erfindung betrifft ein Verfahren zum Erfassen von Daten einer Fluoreszenzmessung an einem Zahn, wobei eine Zahnfläche in entsprechender Weise infiltriert und/oder versiegelt ist. Die vorliegende Erfindung betrifft auch ein Kit umfassend eine erfindungsgemäße Zusammensetzung. Beschrieben werden auch Verfahren zur Kariestherapie und Verfahren zur Kariesdiagnostik sowie zum Kariesmonitoring unter einer versiegelten Zahnfläche.

Die vorliegende Erfindung betrifft insbesondere dentale Zusammensetzungen, die als dentales Versiegelungsmaterial (Fissurenversiegler bzw. Versiegelungsmaterial zur Komplettversiegelung von Zähnen; auch als lichthärtender Desensibilisierungslack) eingesetzt werden können und es dabei beispielsweise gestatten, den Aktivitätszustand initialkariöser Schmelzläsionen mittels Fluoreszenzmessung (insbesondere IR-Laserfluoreszenzmessung) zu bestimmen. Die vorliegende Erfindung betrifft somit insbesondere Zusammensetzungen, die sowohl für Licht der zur Anregung der Fluoreszenz an einem Zahn erforderlichen Wellenlängen als auch für die nach Anregung emittierten Fluoreszenzstrahlen transparent sind.

Die unter Menschen am weitesten verbreitete Infektionskrankheit Karies entsteht in Form kariöser Läsionen, wenn Plaquebakterien, primär Mutans-Streptokokken und Laktobazillen, organische Säuren durch einen anaeroben Stoffwechselmechanismus vergärbarer Kohlenhydrate bilden. Die Säuren diffundieren in die Zahnhartsubstanz und zerstören die kristallinen Phasen des Schmelzes und des Dentins. Durch die Auflösung dieser Mineralien werden irreversible strukturelle Veränderungen im Zahnhartgewebe verursacht. Diese Demineralisation findet ab einem pH-Wert von weniger als 5,5 statt. Darüber hinaus kommt es zu einer fortwährenden Ausfällung bestimmter Ionen, wie Kalzium- und Phosphationen, aus dem Speichel. Die Gegenwart dieser Ionen im Mundraum führt zu einer Remineralisation. Ist Fluorid zugegen, wird die Remineralisation zusätzlich gefördert, indem sich säureresistenter Fluorhydroxylapatit bildet. Im dynamischen Milieu der Mundhöhle wechseln sich Demineralisation und Remineralisation ab. Besteht ein Gleichgewicht zwischen diesen Vorgängen, entsteht keine Karies. Wird das Gleichgewicht jedoch gestört, wird durch zunehmenden Verlust an anorganischen Bestandteilen des Schmelzes die Bildung einer kariösen Läsion gefördert.

Die Kariesfrüherkennung spielt im Kampf gegen diese Krankheit eine entscheidende Rolle, da es heute Stand des Wissens ist, dass eine Kariesprogression in jedem Stadium arretiert werden kann. Erkennt man eine Läsion im Frühstadium, so ist die Wahrscheinlichkeit einer erfolgreichen Remineralisation deutlich erhöht. Eine (minimal)invasive Versorgung kann so unter Umständen vermieden werden.

In der Regel erfolgt die Diagnose auf kariöse Läsion anhand einer visuellen/taktilen Inspektion der jeweiligen Zahnsituation. Handelt es sich, wie in den meisten Fällen, um Okklusalkaries, so stellt die korrekte Diagnose eine enorme klinische Herausforderung an den Zahnarzt, da es äußerst schwierig ist, selbst unter Zuhilfenahme einer Lupe/Sonde, eine Läsion in Fissuren und Grübchen sicher zu erkennen. Die Schwierigkeit der okklusalen Kariesdiagnostik resultiert aus der speziellen Morphologie von Fissuren und Grübchen. Aus diesem Grund haben sich alternative diagnostische Verfahren im Markt bereits weit entwickelt.

Beispielhaft genannt seien an dieser Stelle Verfahren zum Nachweis und zur kontrollierten Überwachung von Veränderungen im mineralisierten Gewebe durch optische Verfahren. So schlägt die US 7,796,243 B2 den Einsatz der optischen Kohärenztomographie in Kombination mit der Ramanspektroskopie vor. In US 2010/0227296 A1 werden Verfahren zur Bewertung von Risikofaktoren bezüglich der oralen Gesundheit angegeben, die u.a. auf physikalischen Phänomenen wie Lumineszenz, Fluoreszenz und thermischen Emissionen beruhen. US 2007/0021670 A1 betrifft Geräte zur Erkennung von Karies, Demineralisation und Remineralisation, die auf IR-photothermaler Radiometrie und Lumineszenz basieren.

Als besonders geeignet zum Erkennen von insbesondere Karies, aber auch von Plaque, Konkrementen, bakteriellem Befall an Zähnen etc. haben sich berührungslose Verfahren und Vorrichtungen erwiesen, die auf die Eigenschaft von gesunder und kariöser Zahnhartsubstanz zurückgreifen, natürlicherweise zu fluoreszieren. Durch die Bestrahlung mit energiereichem Laserlicht bestimmter Wellenlänge können beispielsweise kariöse Bereiche zu einer stärkeren Fluoreszenz als gesunde Zahnhartsubstanz angeregt werden.

In DE 30 31 249 C2 wird eine Untersuchungsmethode zum Nachweis von Karies beschrieben, wobei der Zahn mit nahezu monochromatischem Licht bestrahlt wird. Die Lichtstrahlung regt an dem Zahn eine Fluoreszenzstrahlung an. Das vom Zahn dann emittierte Fluoreszenzspektrum zeigt deutliche Unterschiede zwischen kariösen und gesunden Zahnbereichen. So ist im roten Spektralbereich des Fluoreszenzspektrums des Zahns, zwischen 550 und 650 nm, die Intensität deutlich höher als bei einem gesunden Zahn, bezogen auf eingestrahltes Licht einer Wellenlänge von 410 nm. Demgegenüber ist im blauen Spektralbereich des Fluoreszenzspektrums des Zahns, zwischen 350 und 450 nm, die Intensität der Fluoreszenzstrahlung für kariöse Bereiche und gesunde Bereiche des Zahns fast identisch. Man schlug

daher vor, den Zahn mit einer Wellenlänge von 410 nm zu bestrahlen und mittels zweier Filter die Fluoreszenzstrahlung des Zahns für eine erste Wellenlänge von 450 nm sowie eine zweite Wellenlänge von 610 nm, also im blauen und roten Spektralbereich, beispielsweise mit Hilfe von Photodetektoren zu erfassen. Die durch diese Anordnung erfassten Fluoreszenzstrahlungsintensitäten werden subtrahiert, so dass aufgrund der dadurch gewonnenen Differenzintensität ein gesunder Zahnbereich eindeutig von einem kariösen Zahnbereich unterschieden werden kann.

An anderer Stelle (S. Albin et al., "Laser Induced Fluorescence of Dental Caries", Proc SPIE 907, 96-98, 1988) wird eine Anregungswellenlänge von 488 nm angegeben.

In DE 42 00 741 C2 wird beschrieben, dass eine Vorrichtung zum Erkennen von Karies durch eine Anregungsstrahlung mit einer Wellenlänge im Bereich von 360 nm bis 580 nm ausgestattet ist und die am bestrahlten Zahn hervorgerufene Fluoreszenzstrahlung im Wellenlängenbereich zwischen 620 nm und 720 nm herausgefiltert wird. Durch diese Maßnahme sei der Abstand zwischen der Wellenlänge der Anregungsstrahlung und der abgegebenen Fluoreszenzstrahlung ausreichend groß, so dass die Anregungsstrahlung nicht die Auswertungsergebnisse durch Überlagerung der Fluoreszenzstrahlung verfälschen kann.

In E. de Josselin de Jong et al. "A new Method for in vivo Quantification of Changes in Initial Enamel Caries with Laser Fluorescence", Caries Research, 2-7, 1995 wird beschrieben, den Zahn mit Laserlicht einer Wellenlänge von 488 nm zu bestrahlen, die Fluoreszenzstrahlung des Zahns für Wellenlängen ab 520 nm über eine CCD-Kamera zu erfassen und die Daten mathematisch auszuwerten, um so einen kariösen Zahnbereich feststellen zu können.

DE 93 17 984 U1 offenbart eine Vorrichtung zum Erkennen von Karies, wobei die Anregungsstrahlung nicht kontinuierlich, sondern gepulst während eines Anregungsintervalls erzeugt wird.

US 7,596,253 offenbart Verfahren und Vorrichtungen zur Detektion von Karies, wobei Fluoreszenz-Bilddaten erzeugt und verarbeitet werden.

In DE 297 04 185 U1 wird eine Vorrichtung zum Erkennen von Karies vorgeschlagen, mit der ein zu untersuchender Zahn homogener bestrahlt und das angeregte Fluoreszenzspektrum des Zahns genauer erfasst werden soll. Die Emissions- und Erfassungseinrichtung dieser Vorrichtung weist eine Vielzahl von einzelnen Emissionsfasern und Detektionsfasern auf, die abwechselnd zueinander angeordnet sein können. Insbesondere soll eine Emissionsfaser von mehreren Detektionsfasern koaxial umgeben sein.

DE 195 41 686 B4 gibt eine Vorrichtung zum Erkennen von Karies, Plaque oder bakteriellem Befall an Zähnen an, die verbesserte Eigenschaften aufweisen soll. Hierbei beträgt die Wellenlänge der von einer Lichtquelle erzeugten Anregungsstrahlung zwischen 600 nm und 670 nm.

DE 198 25 021 A1 beschreibt Verfahren und Vorrichtungen zum Erkennen von Karies, Plaque oder bakteriellem Befall an Zähnen, wobei die Fluoreszenzstrahlung mit Wellenlängen oberhalb von ca. 800 nm ausgewertet wird. Mit dieser Maßnahme soll sich eine besonders empfindliche Erfassung von versteckter Karies, wie in Fissuren oder im approximalen Zahnbereich erreichen lassen, da in diesem Wellenlängenbereich der Anteil von kariesspezifischen Fluorophoren und anderen Ablagerungen besonders hoch ist, aber gesunder Zahnschmelz oder Dentin nicht oder nur gering fluoreszieren.

Weitere Verfahren und/oder Vorrichtungen zum Erkennen von bakteriellem Befall von Zähnen, die auf optischen Prinzipien beruhen, sind den Druckschriften DE 197 09 500 C1, DE 202 09 441 U1, DE 102 27 128 A1, DE 603 16 699 T2, DE 196 19 067, DE 10 2005 052 294 A1, DE 94 17 470 U1 und DE 101 33 451 A1 entnehmbar.

Den nunmehr technisch weit entwickelten Diagnoseverfahren zur Kariesfrüherkennung müssen selbstverständlich geeignete therapeutische Verfahren zur Seite gestellt werden, die bewirken, dass der Kariesbefall nicht weiter fortschreitet und zum Stillstand kommt, möglichst unter Vermeidung eines invasiven Eingriffs durch den Zahnarzt. Bewährt hat sich insoweit die Zahnversiegelung (insbesondere die Versiegelung von Fissuren und Grübchen, aber auch im Sinne einer Komplettversiegelung des Zahnes) mit mechanisch hochbeständigen, retentionsfesten, abrasionsresistenten und leicht fließfähigen Kunststoffzusammensetzungen.

Unter der "Versiegelung von Fissuren und Grübchen" (nachfolgend auch zusammenfassend "Fissurenversiegelung" genannt) versteht man das Auffüllen der zum Teil sehr tiefen Grübchen, rauen Furchen und Rinnen auf der Oberfläche von Zähnen mit einem gut fließenden Kunststoffmaterial. Die oftmals engen Einziehungen auf den Kauflächen eines Zahnes werden Fissuren genannt. Bei Kindern und Jugendlichen, aber auch bei Erwachsenen, entwickelt sich häufig Karies zuerst in den Fissuren der Kauflächen, wobei die okklusalen Flächen der Seitenzähne die höchste Kariesanfälligkeit zeigen. Ein stark ausgebildetes und zerklüftetes Relief befindet sich beispielsweise auch an der Innenseite der Frontzähne, so dass auch hier eine kariesprophylaktische Versiegelung sinnvoll sein kann. Zusätzlich fördert ein deutlich ausgeprägtes Fissurenrelief auch die Plaqueanheftung. Plaqueakkumulation wurde auch in engen Fissuren und an steilen Höckerabhängen beobachtet.

Die verschiedenen Fissurentypen (beispielsweise ampullenförmig, I-förmig, U-förmig, V-förmig) können Speisereste aufnehmen und somit auch für Karies verursachende Bakterien einen idealen und geschützten Lebensraum bieten, da mundhygienische Maßnahmen wie das Putzen der Zähne hier nicht greifen: Die Borsten der Zahnbürsten sind im allgemeinen zu breit, um den Boden der Fissur reinigen zu können. Die Morphologie der Fissuren macht daher eine mechanische Reinigung der Grübchen fast unmöglich. Sollte in der Fissur eine Schmelzkaries entstehen, dann

wird sie sich mit hoher Wahrscheinlichkeit sehr schnell ins Dentin ausweiten, da die Schmelzdicke im Bereich der Fissur, insbesondere am Fissurengrund, in der Regel sehr dünn ist. Die große Anfälligkeit der Fissuren für einen kariösen Befall wird somit hauptsächlich durch ihre spezielle Morphologie erklärt.

Die Kariesgefahr in Fissuren ist im Vergleich mit glatten Zahnflächen auch bei Durchführung einer Fluoridprophylaxe deutlich erhöht, da in den Fissuren eine Fluoridprophylaxe nicht die gewohnte Wirksamkeit entfalten kann.

Durch eine Versiegelung der Fissuren wird das Zahnrelief flacher, der Zahn ist leichter zu reinigen und die Entstehung einer Karies kann so verhindert werden. Die Fissurenversiegelung ist heutzutage als bewährte und empfohlene, effektive prophylaktische Maßnahme anerkannt und findet in der täglichen zahnärztlichen Praxis eine immer breitere Anwendung. Es gilt die Regel, dass alle kariesgefährdeten Fissuren und Grübchen bereits vorsorglich versiegelt werden sollten. Man konnte nachweisen, dass ein solches Vorgehen zu einer deutlichen Abnahme kariogener Mikroorganismen in der Fissur unterhalb der Versiegelung führt.

Wie oben bereits ausgeführt, betrifft die vorliegende Erfindung insbesondere Zusammensetzungen zur Anwendung in einem Verfahren zum Infiltrieren und/oder Versiegeln von Zahnhartsubstanz (gesund oder kariös) und in einem Verfahren zur Detektion von Karies unter der Versiegelung mittels Fluoreszenz sowie in einem Verfahren zum Bestimmen von Veränderungen des Infiltrationszustands bzw. einer Versiegelung mittels Fluoreszenz. Die Zahnhartsubstanz kann dabei gesund oder kariös sein.

Das Infiltrieren kariöser Zahnhartsubstanz kann insbesondere ein Infiltrieren einer initialen kariösen Läsion sein, wobei es sich um Zahnschmelzareale mit erhöhtem Porenvolumen handelt. Diese Poren stellen Diffusionswege für eine fortschreitende Auflösung der Zahnschmelzstruktur dar, weswegen bereits vorgeschlagen wurde, durch Infiltration mit härtbaren Materialien sowohl einen Verschluss der Diffusionswege als auch eine Stabilisierung der geschädigten Schmelzstruktur zu erreichen. Es wurde nachgewiesen, dass niedrigviskose Kunststoffzusammensetzungen in kariöse Läsionen infiltrieren (penetrieren) und nach Aushärtung eine weitere Demineralisation des Zahnschmelzes verhindern können.

In einem Verfahren zum Infiltrieren von gesunder Zahnhartsubstanz wird unter Verwendung eines niederviskosen Kunststoffmaterials Zahnhartsubstanz infiltriert (beispielsweise dort, wo es aufgrund des Rückgangs des Zahnfleisches oder aufgrund des Abbaus von Zahnschmelz nicht durch eine Zahnschmelzschicht geschützt ist), indem das Kunststoffmaterial in die Zahnhartsubstanz eindringt.

In einem Verfahren zum Versiegeln von Zahnhartsubstanz kann es gleichzeitig zum Infiltrieren der Zahnhartsubstanz kommen. Unter "versiegelter Zahnhartsubstanz" wird im Rahmen des vorliegenden Textes eine Zahnhartsubstanz verstanden, auf deren Oberfläche eine ausgehärtete Schicht eines Kunststoffmaterials (Versiegelungsmaterials) gebildet ist. In der dentalen Praxis werden insbesondere Fissuren und Grübchen eines Zahnes versiegelt. In Einzelfällen wird jedoch eine "Komplettversiegelung" von Zähnen durchgeführt, um eine verbesserte prophylaktische Wirkung zu erzielen. Im Rahmen der Komplettversiegelung hat es sich jedoch bislang als nachteilig herausgestellt, dass die eingesetzten Versiegelungsmaterialen an glatten Zahnoberflächen nur unzureichend anhaften.

Auch die Anhaftung von üblichen Versiegelungsmaterialen an Zahnoberflächen im Bereich von Fissuren und Grübchen wird als noch nicht ausreichend angesehen; dies gilt insbesondere hinsichtlich transparenter Versiegelungsmaterialen, welche die Detektion von Karies unter der Versiegelung mittels Floreszenz ermöglichen.

In der dentalen Praxis werden als Versiegelungsmaterialen üblicherweise Zusammensetzungen eingesetzt, welche ein organisches Bindemittelsystem auf Basis von Dimethacrylat umfassen; diese Versiegelungsmaterialen sind entweder nicht oder im Vergleich zu Füllungskompositen geringer gefüllt. Solche nicht oder nur gering gefüllten Versiegelungsmaterialien zeigen in der dentalen Praxis das günstigste Retentioinsverhalten. Aufgrund ihrer geringen Viskosität dringen derartige Versiegelungsmaterialen im Vergleich zu Füllungskompositen besser in die Tiefe einer Fissur oder in Poren einer Läsion bzw. die Tubuli von freiliegendem Dentin ein. Nachteiligerweise sind derartige Versiegelungsmaterialien jedoch aufgrund des geringeren Füllstoffanteils weniger abrasions- und biegefest als übliche dentale Füllungsmaterialien.

Versiegelungsmaterialen werden in der Praxis beispielsweise nach der Art ihrer Aushärtung unterschieden, die durch Licht- und/oder Autopolymerisation erfolgen kann. Lichthärtbare Versieglungsmaterialien, die in Form von Einkomponentenmaterialien vorliegen, sind im Vergleich zu zweikomponentigen autopolymerisierbaren Versiegelungsmaterialien weniger verarbeitungsanfällig, da es keine Blasenbildung während des Anmischens geben kann. Lichthärtbare Versiegelungsmaterialen sind somit üblicherweise bevorzugt. Neben lichthärtbaren und zweikomponentigen autopolymerisierbaren Versiegelungsmaterialien sind auch sogenannten dualhärtende Systeme bekannt, welche durch Lichthärtung und chemische Härtung ausgehärtet werden können.

Ein weiteres Unterscheidungskriterium zur Unterscheidung von Versiegelungsmaterialen betrifft ihr Erscheinungsbild. So wird unterschieden zwischen transparenten und eingefärbten Produkten. Transparente Versiegelungsmaterialen sind entweder ungefüllt (d. h. sie enthalten kein Füllstoffsystem umfassend Füllstoffpartikel) oder sind mit nanoskaligen Füllstoffteilchen versetzt, deren Größe kleiner ist als die Wellenlänge des sichtbaren Lichts. Transparente Versiegelungsmaterialen erlauben es dem Zahnarzt, beispielsweise nach der Versiegelung einer Fissur, ein mögliches Entstehen oder Fortschreiten von Karies in der Tiefe der Fissur zu erkennen.

Viele Varianten dentaler Versiegelungsmaterialien werden auch als fluoridabgebende Materialien angeboten.

In DE 23 01 067 werden Dentalfissurenversiegelungsmittel vorgeschlagen, die stark verbesserte Handhabungseigenschaften und ein vorzügliches Vermögen aufweisen sollen, sich entwickelnde Löcher und Fissuren in Zähnen vollständig zu füllen und zu versiegeln, wobei ihre Zusammensetzungen als hauptsächliche härtbare Monomerkomponente Glykoldimethacrylate, wie beispielsweise Ethylenglycoldimethacrylat, Diethylenglykoldimethacrylat, etc. enthalten. Es wird erläutert, dass die Verwendung aromatischer Dimethacrylate zum Versiegeln von Fissuren ungeeignet ist, da ihre Viskosität zum Einfließen in die Löcher und Fissuren zu hoch sei und mit ihnen keine vollständige Versiegelung und gute Haftung zu erzielen sei. Die beschriebenen Zusammensetzungen sind zweikomponentig ausgelegt und werden chemisch ausgehärtet.

Es ist zu erwarten, dass Zusammensetzungen, wie sie in der DE 23 01 067 beschrieben sind, aufgrund der relativ großen Menge an reaktiver Monomerkomponente viel Wasser aufnehmen werden. Die im Monomer vorhandenen Ethergruppen $-CH_2-CH_2-O-$ zeigen freie, ungehinderte Rotation und bilden hochflexible Molekülketten. Die Packung der Ketten im Polymerisat ist somit nicht starr und fixiert, sondern beweglich. Hierdurch wird das polymere Netzwerk für einen Eintritt von Wasser geöffnet. Gelangen große Mengen an Wasser ins Polymerisat, wird dieses zusätzlich aufweitet, irreversibel hydrolytisch spalten und abbauen.

In der US 6,573,312 B2 wird eine Zusammensetzung zum Versiegeln/Füllen von Fissuren und Grübchen angegeben, die ein chemisch modifiziertes Bis-GMA (2,2-Bis[4-(2-hydroxy-3-methacryloyl-oxypropoxy)phenyl)propan) enthält. Die Zusammensetzung soll verbesserte physikalische und mechanische Eigenschaften des Polymerisats erzielen und die Umsetzung der Polymerisation soll bei Photohärtung in höheren Raten erfolgen. Die Modifizierung des Bis-GMA erfolgt durch sukzessive Reaktion der sekundären Hydroxylgruppen des Bis-GMA mit Methacrylsäurechlorid in Gegenwart eines organischen Amins. Im ersten Syntheseschritt wird das Dimethacrylat so zu einem Trimethacrylat (Tri-GMA) und in einem zweiten Schritt zu einem tetrafunktionalisierten Methacrylat (Tetra-GMA) abreagiert. Die Zusammensetzungen bestehen aus Bis-GMA und Tri-GMA sowie aus Bis-GMA, Tri-GMA und Tetra-GMA, wobei die Zusammensetzungen ferner Füllstoffe, Photoinitiatoren, Additive und Verdünnungsmonomere enthalten. Als Verdünnungsmonomere werden Methylmethacrylat und Glykoldimethacrylate vorgeschlagen.

Die besonderen Eigenschaften dentaler Zusammensetzungen basierend auf Bis-GMA beruhen auf der Möglichkeit des Moleküls, neben der Verknüpfung über die Methacrylatgruppen, über Wasserstoffbrückenbindungen, ausgehend von den freien Hydroxylgruppen, zusätzliche Struktureinheiten, sogenannte Überstrukturen oder Sekundär/Tertiärstrukturen, auszubilden. Der Aufbau derartiger Überstrukturen kann ebenfalls durch Carbamat-, Amid- oder ähnlich zusammengesetzte Gruppen, nicht jedoch durch Estergruppen befördert werden. Zudem bedingt durch die starre Konformation des Bisphenol-A-Strukturmotivs weist das Polymerisat enge, dicht gepackte und über mehrere Bindungstypen miteinander wechselwirkende Molekülverbände auf. Diese strukturellen Voraussetzungen begründen die guten mechanischen und physikalischen Eigenschaften von Bis-GMA Polymerisaten.

Werden diese zusätzlichen Wechselwirkungen durch Funktionalisierung der freien Hydroxylgruppen blockiert, so wie es gemäß US 6573312 B2 der Fall ist, sollte die Ausbildung der Überstrukturen behindert und ein Abfall der physikalischen und mechanischen Werte der ausgehärteten Zusammensetzungen die Folge sein. Die in US 6,573,312 B2 aufgeführten Werte der physikalischen Eigenschaften sind lediglich geschätzt. Relevante Unterschiede zwischen den Werten der Vergleichsbeispiele und denen der erfindungsgemäßen Beispiele gemäß US 6,573,312 B2 sind nicht ersichtlich.

US 2005/0288387 A1 beschreibt dentale Zusammensetzungen, die zur Beschichtung von Zähnen oder als dentale Versiegelungsmaterialien einsetzbar sein sollen. Die Zusammensetzungen enthalten eine Multiacrylatverbindung, einen Initiator und einen Alkohol. Die Multiacrylatverbindung enthält wenigstens 3 Acrylateinheiten pro Molekül, beispielsweise Dipentaerythritolpentaacrylat, Di-trimethylolpropantetraacrylat, Trimethylolpropantriacrylat, etc. Der Initiator ist bevorzugt ein Photoinitiator.

US 2009/0047633 A1 betrifft ein dentales Versiegelungs- und/oder Beschichtungssystem, das an Zahnhartsubstanz selbst haftet. Die Zusammensetzung enthält 10 bis 60 Gew.-% einer polymerisierbaren Verbindung, umfassend Bis-GMA und Urethanverbindungen oder deren Mischungen, 1 bis 40 Gew.-% einer polymerisierbaren sauren Verbindung, 3 bis 60 Gew.-% eines Kieselsäurefüllstoffs mit einer durchschnittlichen Teilchengröße von 1 bis 100 nm, 1 bis 30 Gew.-% Wasser und 10 bis 60 Gew.-% Lösungsmittel.

EP 0 969 789 B1 beschreibt dentale Versiegelungs- und Beschichtungsmaterialien, die mindestens 10 Gew.-% eines polymerisierbaren Materials, 0,01 bis 20 Gew.-% eines Füllstoffs im Nanobereich, der eine primäre Teilchengröße zwischen 1 und 100 nm aufweist, wobei die Oberfläche des Füllstoffs im Nanobereich durch eine chemische Oberflächenbehandlung mit einem Silanisierungsmittel modifiziert ist, und mindestens 10 Gew.-% eines organischen Lösungsmittel mit einem Siedepunkt, der unterhalb von Wasser liegt, umfassen.

US 6,899,948 B2 offenbart dentale Versieglungsmaterialien, enthaltend neben härtbaren Monomeren nicht-aggregierte, oberflächenmodifizierte Kieselsäureteilchen mit durchschnittlichen Teilchengrößen von kleiner 200 nm.

US 6,572,693 B1 offenbart Dentalmaterialien, die auch zur Versiegelung eingesetzt werden können. Neben einer härtbaren Harzmatrix enthalten diese Zusammensetzungen Cluster nanoskaliger Teilchen sowie nicht-agglomerierte nanoskalige Partikel. Die nanoskaligen Partikel können Kieselsäureteilchen sein, während die Cluster Schwermetalloxide enthalten, um die Radioopazität des Materials sicherzustellen.

WO 2007/028159 A2 betrifft klare, transparente und opake dentale Versiegelungszusammensetzungen, bei denen kolloidale Kieselsäureteilchen mit einer durchschnittlichen Teilchengröße von 10 bis 100 nm im Methacrylat(Acrylat)harz dispergiert sind. Die Zusammensetzungen sind photochemisch härtbar und sollen harte, glatte und glänzende Beschichtungen auf dem Zahn ergeben. Die nicht-agglomerierten Kieselsäureteilchen sollen homogen innerhalb der Harzmatrix verteilt sein, so dass die Dispersion eine niedrige Viskosität aufweist. Der Gehalt an Kieselsäure in der Dispersion beträgt bis zu 60 Gew.-%. Die Polymerisate sollen gute mechanische Werte wie Abriebfestigkeit aufweisen. Bevorzugt werden die Zusammensetzungen zum Versiegeln von Fissuren und Grübchen auf der Oberfläche eines Zahns oder einer Restauration benutzt.

WO 01/30307 A1 offenbart dentale Zusammensetzungen mit einer visuellen Opazität bei einem Wert von kleiner als 0,25. Da die reziproke Eigenschaft der Transluzenz die Opazität darstellt, wird hier eine Zusammensetzung beschrieben mit Werten der Transluzenz, die größer als 0,75 sind. Diese Zusammensetzungen weisen, neben einer härtbaren Harzzusammensetzung, Kieselsäureteilchen mit einem durchschnittlichen Durchmesser von weniger als 200 nm auf.

Eine temporäre, transparente, dentale Lackzusammensetzung zur Beschichtung von Zahnoberflächen wird in US 2006/0063853 A1 offenbart. Hier wird Transparenz durch den Zusatz von Glashohlkugeln zur Zusammensetzung bewirkt. Um diesen Effekt hervorzubringen ist, muss der Brechungsindex des Bindemittels geringer als der der Glashohlkugeln.

Sowohl US 2007/0166450 A1 als auch US 2010/0016464 A1 beschreiben dentale Beschichtungs- und Versiegelungsmaterialien, die Fluoreszenzmittel enthalten.

WO 2005/094757 A1 offenbart eine dentale Zusammensetzung, die für einen Einsatz als Fissuren- und Grübchenversiegeler geeignet ist und ein mittels Wärme aushärtbares Polymerisationssystem umfasst, das einer Stufenwachstumspolymerisation unterliegt.

In EP 1 307 173 B1 wird ein Fluoridlack und Fissurenversiegelungsmaterial auf Silikonbasis offenbart.

Die Druckschriften DE 20 2006 020 483 U1, DE 20 2006 020 480 U1, DE 20 2006 020 479 U1, DE 20 2006 020 477 U1, DE 20 2006 020 476 U1, EP 2 023 884 A1, EP 1 854 445 A1, EP 2 145 613 A1 und EP 2 151 229 A2 betreffen Zusammensetzungen zur Infiltration von Zahnschmelz bei der Behandlung oder Prävention von Kariesläsionen.

Nanoskalige Teilchen in dentalen Zusammensetzungen, die allerdings nicht zur Zahnversiegelung vorgesehen sind, werden zudem in US 5,936,006 A, US 6,194,481 und US 6,593,395 B2 beschrieben.

In "Effects of composite fissure sealants on IR laser fluorescence measurements", Lasers Med Sci, 23, 133-139, 2008 wurde das Konzept einer Schmelzversiegelung als therapeutische Maßnahme, den Kariesbefall zu arretieren, mit dem einer fluoreszenzbasierten optischen Methode zur Kariesdiagnostik kombiniert. Die Untersuchungsergebnisse ergaben, dass es möglich ist, die Kariesaktivität unter ungefüllten oder transparenten, mit nanoskaligen Teilchen gefüllten, Versiegelungsmaterialien klinisch verlässlich zu verfolgen.

Nanoskalige, hochdisperse Füllstoffteilchen zeichnen sich regelmäßig dadurch aus, dass die Teilchen isoliert in der Harzmatrix in Form einer Suspension vorliegen. Die Partikel zeigen dabei im Wesentlichen weder Aggregation noch Agglomeration. Ein nanoskaliges Teilchen liegt somit regelmäßig in diskreter Form vor und ist durch geeignete physikalische Verfahren (beispielsweise durch Transmissions-Elektronenmikroskopie) ein als Individuum erkennbares Teilchen. Der Durchmesser eines nanoskaligen Teilchens und somit seine Größe liegt unter 200 nm, vorzugsweise unter 150 nm. Besonders bevorzugt sind auch für die vorliegende Erfindung Teilchengrößen von 1 nm bis 100 nm.

Unter dem Begriff "Aggregat" wird eine feste Zusammenlagerung von Primärteilchen zu Sekundär- bzw. Tertiärteilchen etc. verstanden. Unter dem Begriff "Agglomerat" wird eine nicht feste Zusammenlagerung von "Aggregaten" verstanden. Ein Aufbrechen und eine Zerteilung von "Agglomeraten" in die ihnen zugrunde liegenden "Aggregate" durch den Eintrag mechanischer Energie in die Suspension, beispielsweise durch Dispergieren, ist vergleichsweise leicht möglich. Ein weiterer Abbau in die einzelnen Primärteilchen ist dann nicht mehr ohne weiteres möglich.

Beispiele für hochdisperse, nanoskalige Füllstoffe, die in diskreter Form vorliegen, sind amorphe Kieselsäuren. Die (Primär-)Teilchen weisen im Allgemeinen Durchmesser von 1 nm bis 150 nm auf und sind in der Regel kugelförmig. Das Innere der einzelnen Partikel besteht üblicherweise aus einem Gerüst von Siloxanverbindungen, das sich aus der Verknüpfung von [$SiO_4$]- Tetraedern bzw. von Polykieselsäuren ergibt. Die Teilchen können dispergiert in einer Flüssigkeit als Kieselsol vorliegen. Man nennt die Kieselsäure dann kolloidale Kieselsäure.

Solche Substanzen können auf unterschiedliche Weise hergestellt werden.

Beispielsweise können sie mittels Flammenhydrolyse von Chlorsilanen synthetisiert werden. Bei diesem Herstellverfahren werden spezielle pyrogene Kieselsäuren erhalten. Je nach Synthesebedingungen können pyrogene Kieselsäuren unterschiedliche Primärpartikelgrößen erhalten, wobei mit Abnahme der Primärpartikelgröße die Aggregation der Primärpartikel zunimmt. So liegt beispielsweise die pyrogene Kieselsäure AEROSIL OX 50 als die mit einem Partikeldurchmesser von 40 nm grobteiligste im Handel erhältliche Kieselsäure, die nach dem Verfahren der Flammenhydrolyse hergestellt wird, weitgehend isoliert in Form sphärischer Partikel vor. Vom Hersteller wird sie bezüglich ihrer

"nicht-aggregierten" und "nicht-agglomerierten" Struktur den Kieselsäuren, die aus dem naßchemischen "Sol-Gel Verfahren" stammen, strukturell gleichgestellt (siehe Degussa, Schriftenreihe Pigmente, Zur Bedeutung und Existenz von Primärteilchen bei hochdispersen Stoffen, Nummer 60, Seite 10-11). Nimmt die Primärpartikelgröße ab, so verstärkt sich die Tendenz pyrogener Kieselsäuren, sich zu Aggregaten zusammenzulagern.

Hochdisperse, nanoskalige Füllstoffe, die in diskreter Form, also als isolierte, individuellle Partikel vorliegen, können auch mittels des "Sol-Gel"-Verfahrens hergestellt werden. Kieselsäuren werden hierbei durch gezielte Hydrolyse und Kondensation aufgebaut. Ausgehend von Sol-Gel-Vorstufen wie Metallalkoxiden, beispielsweise Tetraalkoxysilan, wird beispielsweise in Alkohol oder Wasser unter basischer Katalyse eine Hydrolyse und Kondensation durchgeführt. Diese Reaktion kann auch sauer katalysiert ablaufen. Aufgrund der großen Anzahl an Einflussparametern (Katalysator, Molverhältnis, Temperatur, Zeit, pH-Wert, Reaktionsmedium usw.) in diesem naßchemischen Verfahren können gezielt gewünschte Eigenschaftsparameter angesteuert werden. Die Anwesenheit von Hydroxylgruppen an den Oberflächen der Teilchen in Verbindung mit dem sauren oder basischen Charakter der Suspension ermöglichen die Einstellung der Stabilität einer Partikeldispersion über einen breiten pH-Bereich aufgrund von interpartikulärer elektrostatischer Abstoßung. Es ergeben sich hierbei enge Teilchengrößenverteilungen. Das vorstehend erläuterte Herstellverfahren wird auch als "Stöber-Synthese" bezeichnet.

Mit Sol-Gel-Verfahren ist auch die Herstellung nanoskaliger Metall- und Schwermetalloxide sowie von Mischoxiden möglich.

Gemäß einem bevorzugten alternativen Verfahren zur Herstellung von kolloidalem Kieselsol wird von Wasserglas ausgegangen. Hierbei wird eine wässrige Lösung von Natriumsilikat mittels eines Ionenaustauschers entionisiert, wodurch Kieselsäure gebildet wird. Diese Säure ist instabil und polymerisiert zu kleinen Partikeln, aus denen dann die nanoskaligen Kieselsäuren gebildet werden. Durch ein geeignetes Einstellen der Prozeßparameter können auch hier enge Partikelgrößenverteilungen erhalten werden.

Die anorganischen Füllstoffteilchen dentaler Zusammensetzungen werden häufig organisch oberflächenbehandelt. Eine organische Oberflächenbehandlung verbessert üblicherweise die Kompatibilität der Partikeloberfläche mit der organischen Bindemittelphase, da die an sich hydrophile Teilchenoberfläche durch die Oberflächenbehandlung hydrophobiert wird und sich dann besser mit der Kunststoffmatrix verträgt. Die Oberflächenmodifizierung wird bevorzugt so durchgeführt, dass die anorganische Oberfläche bei Aushärtung des Materials durch Copolymerisation kovalent in das entstehende Polymer eingebunden wird. Häufig wird die anorganische Oberfläche silanisiert. Hierbei werden vorhydrolysierte Methacryloxyalkyltrialkoxysilane, wie beispielsweise 3-Methacryloxypropyltrimethoxysilan, eingesetzt, bei denen die bei der Hydrolyse gebildeten Silanolgruppen mit den freien Hydroxylgruppen der Füllstoffoberfläche reagieren. Angaben über Modifizierungsreagentien und Ausführungsarten sind u.a. aus DE 24 05 578 A1, US 2002/0065337, DE 195 08 586 A1, WO 00/69392 sowie US 6,387,981 bekannt. Derartige Verfahren zum Modifizieren der Oberfläche der anorganischen Partikel sind auch im Rahmen der vorliegenden Erfindung (siehe unten) bevorzugt.

Wie die flammhydrolytisch hergestellten, pyrogenen Kieselsäuren, die kommerziell in unterschiedlichen Partikelgrößen durch die Produktserien "Aerosile" (Degussa) oder "CAB-O-SIL" (Cabot Corporation) erhältlich sind, können auch die nach alternativen Verfahren hergestellten Kieselsäuren in unterschiedlichen Größen durch die Produktserien "Highlink" (Clariant), "Nalco" (Nalco Chemical Company), "Nanocryl" (Nanoresins), "Bidzil" (Eka Chemicals), "Levasil" (Fa. H.C. Starck), "NexSil" (Nyacol) oder "Ludox" (du Pont) kommerziell erstanden werden.

Zu geeigneten Kieselsolen sei auch auf DE 600 12 775 T2, DE 10 2006 044 520 A1, EP 2 110 414 A1 sowie auf die dort zitierten Referenzen verwiesen.

Es war eine primäre Aufgabe der vorliegenden Erfindung, eine Zusammensetzung zur Anwendung in einem Verfahren zum Infiltrieren und/oder Versiegeln von Zahnhartsubstanz und zur Detektion von Karies im infiltrierten Bereich bzw. unter der Versiegelung mittels Fluoreszenz anzugeben. Die zu infiltrierende bzw. zu versiegelnde Zahnhartsubstanz sollte dabei wahlweise kariös oder gesund sein.

Um die Detektion von Karies im infiltrierten Bereich bzw. unter der Versiegelung mittels Fluoreszenz zu ermöglichen, sollte die Zusammensetzung vorzugsweise so ausgestaltet sein, dass sie zu einem Versiegelungsmaterial aushärtbar ist, das eine hohe Transmission (vorzugsweise > 70 % Transmission bei Schichtdicke von 1 mm) in dem zur Detektion benötigten Wellenlängenbereich aufweist.

Die anzugebende Zusammensetzung sollte dabei in ausgehärteter Form vorzugsweise gleichzeitig eine hervorragende Anhaftung und Retention am Zahnschmelz besitzen und dabei vorzugsweise auch gleichzeitig hervorragende mechanische Eigenschaften aufweisen (auch nach Alterung); insbesondere sollten die Biegefestigkeit und die Abrasionsresistenz besonders hoch sein.

Zudem sollte die anzugebende Zusammensetzung den behandelnden Zahnarzt in die Lage versetzen, Messungen am versiegelten bzw. infiltrierten Zahn so durchzuführen, dass er möglichst viele Informationen über den Gesundheitszustand des behandelten Zahnes (insbesondere über das Vorhandensein oder die Abwesenheit von Karies) und über die Qualität bzw. die sich mit der Zeit ändernden Eigenschaften der applizierten Versiegelung bzw. des Infiltrationszustandes zu erhalten.

Die anzugebende Zusammensetzung sollte vorzugsweise auch ein Monitoring des versiegelten bzw. infiltrierten

Zahnes und einer applizierten Versiegelung über einen längeren Zeitraum hinweg ermöglichen, und den behandelnden Zahnarzt z.B. in die Lage versetzen, einen geeigneten Zeitpunkt für die Entfernung einer ihren Zweck nicht mehr hinreichend erfüllenden Versiegelung möglichst genau und auf einfache Weise zu bestimmen.

Gemäß einem eng verwandten Aspekt war es eine entsprechende Aufgabe der vorliegenden Erfindung, ein verbessertes Verfahren zum Erfassen von Daten einer Messung an einer versiegelten bzw. infiltrierten Zahnfläche eines Zahnes anzugeben.

Es war eine weitere eng verwandte Aufgabe der vorliegenden Erfindung, ein verbessertes Verfahren zur Vorbereitung einer Kariesdiagnostik an einem Zahn eines Patienten anzugeben. Ein entsprechendes Verfahren zur Kariesdiagnostik sollte dabei an dem erfindungsgemäß anzugebenden Vorbereitungsverfahren anknüpfen.

Im Zusammenhang mit den vorstehend geschilderten (Teil-)Aufgaben, welche der vorliegenden Erfindung zugrunde liegen, ist darauf hinzuweisen, dass zwar, wie vorstehend unter Bezugnahme auf den Stand der Technik geschildert, bereits eine Vielzahl von Zusammensetzungen bekannt sind, welche zur Infiltration und/oder Versiegelung von Zahnflächen (insbesondere Fissuren und Grübchen, aber auch beispielsweise freiliegendem Dentin, siehe dazu oben) geeignet sind, dass jedoch ganz generell die Suche nach einem geeigneten Versiegelungsmaterial besondere Schwierigkeiten mit sich bringt, wenn das anzugebende Material nicht nur eine kurzzeitige Versiegelung ermöglichen, sondern gleichzeitig eine Reihe von weiteren Anforderungen erfüllen soll.

Zusammengefasst sind einige der vorstehend genannten Anforderungen an eine anzugebende Zusammensetzung die folgenden:

- Gute Retention am Zahnschmelz bzw. eine gute Haftung durch Penetration in die Dentintubuli, auch nach Alterung;
- Weitere gute mechanische Eigenschaften (insbesondere Biegefestigkeit und Abrasionsresistenz);
- Hoher Transmissionsgrad in dem für die Kariesdiagnostik verwendeten Wellenlängenbereich;
- Untersuchbarkeit einer versiegelten Zahnfläche durch das applizierte und ausgehärtete Versieglungsmaterial hindurch, ohne Zerstörung des Versiegelungsmaterials;
- Gute Überprüfbarkeit der Qualität und der sich mit der Zeit verändernden Eigenschaften der auf eine Zahnfläche applizierten Versiegelung, auch im Sinne eines Monitoring über lange Zeiträume hinweg, ohne Zerstörung des Versiegelungsmaterials. Entsprechend gute Überprüfbarkeit des Infiltrationszustands.
- Im ausgehärteten Zustand soll die Zusammensetzung eine Wasseraufnahme besitzen, die kleiner ist als 25 $\mu g/mm^3$;
- Die Zusammensetzung soll ein gutes Anfließverhalten besitzen;

Dabei sollten die Eigenschaften des zu findenden Materials bei Messbedingungen gemessen werden, wie sie weiter unten bei den "Bestimmungsmethoden" angegeben sind.

Die Erfindung wird in den beigefügten Ansprüchen definiert.

Überraschenderweise hat sich in eigenen Untersuchungen herausgestellt, dass sämtliche der vorstehend genannten (Teil-)Aufgaben gelöst werden können durch Anwendung einer erfindungsgemäßen Zusammensetzung umfassend:

(a) ein Füllstoffsystem bestehend aus oder umfassend mittels organischer Strukturelemente oberflächenmodifizierte, anorganische Füllstoffpartikel, wobei die organischen Strukturelemente mit einem organischen Bindemittelsystem unter Bildung kovalenter Bindungen umsetzbar sind,
wobei die Füllstoffpartikel der Zusammensetzung eine mittlere Teilchengröße im Bereich zwischen 1 nm und 150 nm besitzen,
(b) ein organisches Bindemittelsystem umfassend ein oder mehrere polymerisierbare Monomere,

wobei das Füllstoffsystem in dem organischen Bindemittelsystem (b) dispergiert ist.

Die erfindungsgemäße Zusammensetzung ist vorzugsweise so ausgestaltet, dass das eine Monomer bzw. eines, mehrere oder sämtliche der mehreren polymerisierbaren Monomere des organischen Bindemittelsystems (b) zumindest ein Strukturelement Z enthält, das unabhängig von etwaigen weiteren Strukturelementen Z ausgewählt ist aus der Gruppe bestehend aus

$$-O-(C{=}O)-CH{=}CH_2,\ -O-(C{=}O)-C(CH_3){=}CH_2,$$

$-(C=O)-CH=CH_2$, $-(C=O)-C(CH_3)=CH_2$

$-CH=CH_2$, $-C(CH_3)=CH_2$ und $-O-CH=CH_2$.

Die mittlere Teilchengröße wird dabei bestimmt für den Gesamtanteil an Füllstoffpartikeln in der Zusammensetzung. Sofern verschiedene Typen von Füllstoffpartikeln in der Zusammensetzung vorliegen, neben oberflächenmodifizierten, anorganischen beispielsweise organische oder nicht oberflächenmodifizierte Füllstoffpartikel, werden also sämtliche dieser Füllstoffpartikel bei der Bestimmung der mittleren Teilchengröße berücksichtigt. Zur Methode der Bestimmung der mittleren Teilchengröße siehe unten.

Durch Variation der Bestandteile und Mengenverhältnisse des Bindemittelsystems und des Füllstoffsystems lassen sich die physikalischen Eigenschaften einer erfindungsgemäßen Zusammensetzung und der durch Aushärtung daraus hervorgehenden Körper (insbesondere Schichten) individuell steuern. Der Fachmann wird ausgehend von den vorstehend und nachfolgend gegebenen Informationen und insbesondere unter Berücksichtigung der nachfolgenden Beispiele anhand einfacher systematischer Untersuchungen zu erfindungsgemäßen Zusammensetzungen gelangen, die den Erfordernissen des jeweiligen Einzelfalls entsprechen.

Vorzugsweise besteht das Füllstoffsystem aus mittels organischer Strukturelemente oberflächenmodifizierten, anorganischen Füllstoffpartikeln, wobei die organischen Strukturelemente mit den Monomeren unter Bildung kovalenter Bindungen umsetzbar sind. Die anorganischen Füllstoffpartikel, die das Füllstoffsystem bilden oder in ihm enthalten sind, besitzen vor der Oberflächenmodifizierung (siehe dazu unten) bevorzugt eine spezifische Oberfläche von > 380 $m^2/g$, bevorzugt > 450 $m^2/g$, gemessen nach dem BET-Verfahren. Zur gewählten Bestimmungsmethode siehe unten.

Eine solche Zusammensetzung ist erfindungsgemäß zur Anwendung in einem Verfahren zum Infiltrieren und/oder Versiegeln von Zahnhartsubstanz und zur Detektion von Karies im infiltrierten Bereich bzw. unter der Versiegelung mittels Fluoreszenz geeignet und bestimmt. Die Zahnhartsubstanz kann dabei kariös oder gesund sein. Bevorzugt ist eine solche Zusammensetzung zur erfindungsgemäßen Anwendung in einem therapeutischen dentalen Verfahren als Versiegelungsmaterial zur Versiegelung von Fissuren und/oder Grübchen und/oder kariösen Läsionen bestimmt.

Angesichts des genannten Anwendungszwecks versteht es sich, dass die erfindungsgemäß angegebene Zusammensetzung transparent ist für sowohl Fluoreszenzlicht, welches nach entsprechender Anregung von der gesunden bzw. kariösen Zahnhartsubstanz emittiert wird, als auch transparent ist für Licht, welches zur Anregung einer entsprechenden Fluoreszenz geeignet ist. Die erfindungsgemäße Zusammensetzung ist vorzugsweise transparent für Licht im Wellenlängenbereich > 400 nm.

Vorzugsweise ist die erfindungsgemäße Zusammensetzung insgesamt zur Anwendung in Verfahren bestimmt, welche die folgenden Maßnahmen umfassen: (a) Infiltrieren und/oder Versiegeln von Zahnhartsubstanz, (b) Detektion von Karies im infiltrierten Bereich bzw. unter der Versiegelung mittels Floreszenz und/oder (c) Bestimmung von Veränderungen der Versiegelung mittels Fluoreszenz.

Die erfindungsgemäße (transparente) Zusammensetzung wäre ohne die Erkenntnis, dass sie sich im ausgehärteten Zustand für Fluoreszenzuntersuchungen eignet, für den Zweck der Infiltration und/oder Versiegelung von Zahnhartsubstanz nicht uneingeschränkt einsetzbar, denn der behandelnde Zahnarzt ohne Fluoreszenztechnik kann nicht mit ausreichender Sicherheit die Qualität der Infiltration bzw. der Versiegelung bestimmen. Er ist erst durch die Kombination der Zusammensetzung mit dem Einsatz von Fluoreszenztechniken in die Lage versetzt, die Qualität zu überwachen und einen geeigneten Zeitpunkt für ergänzende prophylaktische Maßnahmen zu finden.

Die erfindungsgemäß anzuwendenden Zusammensetzungen umfassen vorzugsweise mittels organischer Strukturelemente oberflächenmodifizierte, nicht flammhydrolytisch hergestellte, anorganische Füllstoffpartikel, bevorzugt in einem Anteil von 50 bis 100 Gew.-%, bezogen auf die eingesetzte Gesamtmasse an Füllstoffpartikeln in der erfindungsgemäßen Zusammensetzung. Diese Füllstoffpartikel resultieren aus der Umsetzung von nicht flammhydrolytisch hergestellten, anorganischen Füllstoffpartikeln mit geeigneten Mitteln zur Oberflächenmodifizierung, üblicherweise aus der Umsetzung solcher Füllstoffpartikel mit geeigneten alkoxysilangruppenhaltigen Agentien. Solche bevorzugten erfindungsgemäßen Zusammensetzungen besitzen überraschenderweise ein hervorragendes Retentionsverhalten an Zahnhartsubstanz sowie weitere hervorragende mechanische Eigenschaften wie insbesondere eine besonders hohe Biegefestigkeit und Abrasionsresistenz. Sie behalten diese Eigenschaften auch nach Alterung weitgehend bei. In eigenen Untersuchungen haben sich solche bevorzugten erfindungsgemäßen Zusammensetzungen als überlegen gezeigt im Vergleich mit Zusammensetzungen, welche überwiegend oder ausschließlich flammhydrolytisch hergestellte Füllstoffpartikel umfassen. In eigenen Untersuchungen (siehe dazu die Beispiele unten) wurden insbesondere Zusammensetzungen verglichen, welche Füllstoffpartikel auf Basis von Kieselsäure umfassen. Der Fachmann wird diese Erkenntnis berücksichtigen, wenn er ausgehend von den weiter unten folgenden Beispielen weitere Zusammensetzungen mit den im Rahmen der vorliegenden Erfindung angestrebten Eigenschaften formulieren will.

Die erfindungsgemäße Zusammensetzung ist vorzugsweise so ausgestaltet, dass sie zu einem Versiegelungsmaterial aushärtbar ist, das eine Haftung auf Zahnschmelz von mehr als 15 MPa, bevorzugt mehr als 17 MPa aufweist.

Die erfindungsgemäße Zusammensetzung ist vorzugsweise so ausgestaltet, dass sie zu einem Versiegelungsmaterial aushärtbar ist, das eine Biegefestigkeit von mehr als 80 MPa, bevorzugt mehr als 90 MPa, besonders bevorzugt mehr als 100 MPa aufweist.

Die erfindungsgemäße Zusammensetzung ist dabei (vorzugsweise gleichzeitig) so ausgestaltet, dass sie zu einem Versiegelungsmaterial aushärtbar ist, das eine Abrasion, bestimmt nach der 3-Medien-Abrasions-Methode nach ACTA (zur Bestimmungsmethode siehe weiter unten), von weniger als 80 $\mu$m, bevorzugt weniger als 70 $\mu$m, besonders bevorzugt weniger als 60 $\mu$m aufweist.

Besonders bevorzugt ist die erfindungsgemäße Zusammensetzung sowohl so ausgestaltet, dass sie zu einem Versiegelungsmaterial aushärtbar ist, das sowohl die genannte Biegefestigkeit als auch die genannte Abrasion besitzt.

Eine besonders bevorzugte erfindungsgemäße Zusammensetzung ist so ausgestaltet, dass sie zu einem Versiegelungsmaterial aushärtbar ist, das bei einer Schichtdicke von 1 mm einen Transmissionsgrad von 70 % oder mehr, bevorzugt von 75 % oder mehr, besonders bevorzugt von 80 % oder mehr für eine senkrecht einfallende Strahlung mit einer Wellenlänge von 700 nm besitzt. 700 nm ist eine Wellenlänge, wie sie in Fluoreszenzspektrum vorhanden ist, welches bei der Fluoreszenz von kariöser und gesunder Zahnhartsubstanz entsteht, wobei die Fluoreszenzintensität für kariöse Zahnhartsubstanz bei dieser Wellenlänge signifikant größer ist als für gesunde Zahnhartsubstanz.

Bevorzugt ist die erfindungsgemäße Zusammensetzung zusätzlich so ausgestaltet, dass sie zu einem Versiegelungsmaterial aushärtbar ist, das bei einer Schichtdicke von 1 mm einen Transmissionsgrad von 70 % oder mehr, bevorzugt von 75 % oder mehr, besonders bevorzugt von 80 % oder mehr für eine senkrecht einfallende Strahlung mit einer Wellenlänge von 655 nm besitzt. Die Wellenlänge von 655 nm ist eine bevorzugte Anregungswellenlänge für Fluoreszenzuntersuchungen am Zahn.

Ganz besonders bevorzugt ist die erfindungsgemäße Zusammensetzung so ausgestaltet, dass sie zu einem Versiegelungsmaterial aushärtbar ist, das bei einer Schichtdicke von 1 mm einen Transmissionsgrad von 70 % oder mehr, bevorzugt von 75 % oder mehr, besonders bevorzugt von 80 % oder mehr für eine senkrecht einfallende Strahlung besitzt, wobei

(a) der Transmissionsgrad im gesamten Wellenlängenspektrum des visuellen Lichtes bestimmt wird, vorzugsweise mit einem Spektrokolorimeter (ColorFlex) der Firma Hunterlab (siehe dazu die Erläuterungen weiter unten), und/oder

(b) der Transmissionsgrad bei eine Wellenlänge von 500 nm bestimmt wird, bevorzugt mit einem UV/VIS-Spektrometer vorzugsweise des Typs Lambda 650 (PerkinElmer) (siehe dazu die Erläuterungen weiter unten).

Bevorzugt ist die erfindungsgemäße Zusammensetzung zusätzlich dadurch gekennzeichnet, dass der Kontaktwinkel der Zusammensetzung auf trockenem Zahnschmelz, gemessen mit einem Kontaktwinkelmessgerät der Firma Krüss (DSA 100), weniger als 60°, bevorzugt weniger als 50°, besonders bevorzugt weniger als 40° beträgt.

Bevorzugt ist die erfindungsgemäße Zusammensetzung so ausgestaltet, dass sie zu einem Versiegelungsmaterial aushärtbar ist, dessen Wasseraufnahme weniger als 15 $\mu g/mm^3$ beträgt, bevorzugt weniger als 13 $\mu g/mm^3$, besonders bevorzugt weniger als 10 $\mu g/mm^3$.

Besonders bevorzugt ist die erfindungsgemäße Zusammensetzung sowohl so ausgestaltet, dass sie zu einem Versiegelungsmaterial aushärtbar ist, das nicht nur den genannten Transmissionsgrad besitzt, sondern gleichzeitig auch (i) die genannte Biegefestigkeit und/oder (ii) die genannte Abrasion und/oder (iii) den genannten Kontaktwinkel der Zusammensetzung auf trockenem Zahnschmelz und/oder (iv) die genannte Wasseraufnahme.

Erfindungsgemäß einsetzbar sind sowohl oberflächenmodifizierte flammhydrolytisch hergestellte Kieselsäurepartikel als auch oberflächenmodifizierte nicht-flammhydrolytisch hergestellte Kieselsäurepartikel, die zu leicht unterschiedlichen Eigenschaften der erfindungsgemäß einzusetzenden Zusammensetzungen führen können. Da sowohl oberflächenmodifizierte flammhydrolytisch hergestellte Kieselsäurepartikel als auch oberflächenmodifizierte nicht-flammhydrolytisch hergestellte Kieselsäurepartikel weitgehend nicht-aggregiert und nicht-agglomeriert vorliegen können, können über die Ursache der unterschiedlichen Eigenschaften der jeweiligen Zusammensetzungen nur Vermutungen angestellt werden. Möglicherweise spielen zwei Gründe, die auf dem Unterschied im Herstellverfahren der Teilchen beruhen, eine entscheidende Rolle:

So ist zum einen bekannt, dass die Anzahl der freien Hydroxylgruppen bei flammhydrolytisch hergestellten Kieselsäureteilchen sehr viel geringer ist als die Anzahl der freien Hydroxylgruppen bei nicht-flammhydrolytisch hergestellten (insbesondere nasschemisch hergestellten) Kieselsäureteilchen. Dieser Unterschied basiert darauf, dass sich bei den flammhydrolytischen Prozess, der die Verbrennung von Siliziumtetrachlorid in einer Sauerstoff-Wasserstoff-Flamme bei hohen Temperaturen umfasst, durch direkte (thermische) Kondensation der Silanolgruppen Polysiloxanstrukturen bilden, wodurch die Anzahl der freien Hydroxylgruppen reduziert wird.

Des Weiteren ist ebenfalls bekannt, dass flammhydrolytisch hergestellte Kieselsäuren im Gegensatz zu nasschemisch erzeugten Kieselsäuren über eine nahezu porenfreie, glatte Oberfläche verfügen. Auch dieses Merkmal ist durch das Herstellverfahren verursacht.

Flammhydrolytisch hergestellte Kieselsäuren besitzen somit im Gegensatz zu nasschemisch hergestellten Kieselsäuren aufgrund der unterschiedlichen Herstellverfahren eine geringere Zahl von Hydroxylgruppen pro Flächeneinheit, wobei die Gesamtoberfläche von flammhydrolytisch hergestellten Kieselsäuren sogar noch kleiner ist als die Gesamtoberfläche von nasschemisch hergestellten Kieselsäuren. Diese Sachlage lässt es plausibel erscheinen, dass nasschemisch hergestellte Kieselsäure in bei weitem stärkeren Ausmaß funktionalisiert werden als flammhydrolytisch hergestellte Kieselsäuren gleicher Partikelgröße. Eine stärkere Oberflächenfunktionalisierung der nicht-flammhydrolytisch (vorzugsweise nasschemisch) hergestellten Kieselsäurepartikel führt jedoch zu einer höheren Vernetzungsdichte und somit zu einem besseren Einbau der Partikel in die Matrix eines organischen Bindemittelsystems. Dieser bessere Einbau der Teilchen scheint verantwortlich zu sein für die in eigenen Untersuchungen bestimmten besseren mechanischen Werte und die sehr gute Abrasionsbeständigkeit der erfindungsgemäß anzuwendenden dentalen Zusammensetzungen, welche auf nicht-flammhydrolytisch hergestellten, anorganischen Füllstoffpartikeln basieren.

Überdies verbleiben nach Oberflächenmodifizierung der nicht-flammhydrolytisch hergestellten (vorzugsweise nasschemisch hergestellten) anorganischen Füllstoffpartikel regelmäßig noch frei Hydroxylgruppen auf den Oberflächen. Es scheint so zu sein, als ob die verbleibende recht große Zahl freier Hydroxylgruppen auf nasschemisch hergestellten Füllstoffpartikeln den entsprechenden dentalen Zusammensetzungen ein gewisses Maß an Hydrophilie geben, was möglicherweise dafür verantwortlich ist, dass solche erfindungsgemäß anzuwendenden Zusammensetzungen vergleichsweise gut an geätzte Zahnhartstrukturen anfließen, diese penetrieren und so zu einer festen Mikroverzahnung des Kunststoffs an der Zahnhartsubstanz führen. Dieser Effekt scheint die hervorragenden experimentellen Haftwerte erfindungsgemäß anzuwendender Zusammensetzungen zu erklären, welche auf nicht-flammhydrolytisch hergestellten, anorganischen Füllstoffpartikeln basieren.

In eigenen Untersuchungen hat sich zudem gezeigt, dass es vorteilhaft ist, die oberflächenmodifizierten nicht-flammhydrolytisch hergestellten, anorganischen Füllstoffpartikel in Dispersion herzustellen und vor Vereinigen mit dem einzusetzenden Bindemittelsystem nicht zu trocknen. Bei anderer Vorgehensweise wurden in manchen Fällen erhebliche Unterschiede in den erhaltenen physikalischen Eigenschaften festgestellt, die möglicherweise auf eine veränderte Oberflächenstruktur der getrockneten im Vergleich mit den nicht getrockneten Partikeln zurückzuführen ist.

Aufgrund der Eigenschaften (Transmission; Biegefestigkeit; Abrasionsresistenz; Haftung auf Zahnschmelz) der erfindungsgemäß anzuwendenden Zusammensetzung lässt sich im Zusammenspiel mit Fluoreszenzdetektionsmethoden eine hervorragende und dennoch kostengünstige Kariesprophylaxe insbesondere im Bereich von dentalen Fissuren und Grübchen und im Bereich von freiliegendem Dentin erreichen.

In einer erfindungsgemäßen Zusammensetzung ist das organische Bindemittelsystem (b) so ausgestaltet, dass die Gesamtmenge des organischen Bindemittelsystems (b) umfasst

(b1) ein, zwei oder mehr Monomere ausgewählt aus der Gruppe bestehend aus Verbindungen (Monomere) der Struktur $Q(Y_xZ_e)_{b,}$ wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlen wasserstoffresten, wobei optional ein, zwei oder mehr der nicht durch Substituenten $Y_xZ_e$ substituierten Wasserstoffatome dieses polyalicyclischen Strukturelements Q durch Alkylgruppen, Alkoxygruppen, Halogenatome oder Trifluormethylgruppen substituiert sind,

- b ist eine natürliche Zahl ausgewählt aus der Gruppe der natürlichen Zahlen 1, 2, 3 und 4,

- jedes Z bedeutet ein Strukturelement, das unabhängig von etwaigen weiteren Strukturelementen Z ausgewählt ist aus der Gruppe bestehend aus

  $-O-(C{=}O)-CH{=}CH_2$, $-O-(C{=}O)-C(CH_3){=}CH_2$,

  $-(C{=}O)-CH{=}CH_2$, $-(C{=}O)-C(CH_3){=}CH_2$

  $-CH{=}CH_2$, $-C(CH_3){=}CH_2$ und $-O-CH{=}CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jeder Index x bedeutet unabhängig von etwaigen weiteren Indizes x 0 oder 1,

- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,
  und vorzugsweise

(b2) ein, zwei oder mehr polymerisierbare Monomere, wobei das oder die polymerisierbaren Monomere keine Verbindungen (Monomere) der vorstehend definierten Struktur $Q(Y_xZ_e)_b$ sind.

Eine solche Verbindung der Struktur $Q(Y_xZ_e)_b$ umfasst ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten $Y_xZ_e$ ersetzt sind (wie oben beschrieben), und optional ein, zwei oder mehr der nicht durch Substituenten $Y_xZ_e$ substituierten Wasserstoffatome durch Alkylgruppen, Alkoxygruppen, Halogenatome oder Trifluormethylgruppen substituiert sind. Das polyalicyclische Strukturelement Q wird konstituiert durch Kohlenstoff-Ring-Atome. Kohlenstoffatome außerhalb der Ringe sind Bestandteil von Substituenten.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", "tricyclisch", tetracyclisch", "pentacyclisch" und "hexacyclisch" entsprechen dabei der IUPAC-Nomenklatur.

Q bedeutet mit anderen Worten ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $Y_xZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert ist bzw. sind.

Vorzugsweise bedeutet jedes Y ein Strukturelement, welches in der Struktur $Q(Y_xZ_e)_b$ mit x = 1 das polyalicyclische Strukturelement Q mit e Strukturelementen Z verbindet, wobei jedes Y unabhängig von etwaigen weiteren Strukturelementen Y gewählt ist.

Die Summe der Zahlenwerte des Index b und des Index e beträgt vorzugsweise 3, 4, 5, 6, 7 oder 8.

Es wurde gefunden, dass die erfindungsgemäße Zusammensetzungen für die genannten Zwecke sehr gute dentale Versiegelungsmaterialien sind, die im Vergleich zu einem Versiegelungsmaterial aus dem Stand der Technik am getrockneten Zahnschmelz ein sehr viel besseres Anfließverhalten zeigen (insbesondere in Bezug auf den Kontaktwinkel am trockenen Zahnschmelz), sehr viel weniger Wasser aufnehmen und gute mechanische Werte aufweisen (insbesondere Biegefestigkeit).

Vorzugsweise ist in einer erfindungsgemäßen Zusammensetzung das organische Bindemittelsystem so ausgestaltet, dass das eine Monomer bzw. eines, mehrere oder sämtliche der mehreren polymerisierbaren Monomere radikalisch oder nicht-radikalisch polymerisierbar ist.

Das erfindungsgemäß einzusetzende bzw. anzuwendende Kompositmaterial ist vorzugsweise lichthärtbar.

Innerhalb einer erfindungsgemäßen Zusammensetzung besteht die Funktion des organisches Bindemittelsystems (b) darin, eine Matrix zu bilden, in welchem das oben genannte Füllstoffsystem eingebunden ist.

Komponente (b1): ein, zwei oder mehr Monomere der Struktur $Q(Y_xZ_e)_b$ mit mindestens einem polyalicyclischen Strukturelement

Komponente (b1) bilden ein, zwei oder mehr Monomere der oben definierten Struktur $Q(Y_xZ_e)_b$, wobei Z vorzugsweise ein Strukturelement bedeutet, das unabhängig von etwaigen weiteren Strukturelementen Z ausgewählt ist aus der Gruppe bestehend aus - $O-(C=O)-CH=CH_2$, $-O-(C=O)-C(CH_3)=CH_2$, $-(C=O)-CH=CH_2$ oder $-(C=O)-C(CH_3)=CH_2$. Bevorzugt sind Verbindungen der Struktur $Q(Y_xZ_e)_b$, wobei Z ausgewählt ist aus der Gruppe bestehend aus $-O-(C=O)-CH=CH_2$, $-O-(C=O)-C(CH_3)=CH_2$, d.h. solche Verbindungen der Struktur $Q(Y_xZ_e)_b$, die ein, zwei oder mehr Acrylat- und/oder Methacrylatgruppen aufweisen, vorzugsweise zwei oder mehr Acrylat- und/oder Methacrylatgruppen.

Die mit den erfindungsgemäß vorzugsweise zu verwendenden Monomeren der Komponente (b1) erhältlichen Polymere und Zusammensetzungen weisen eine ausgeprägte Hydrophobie auf, die sich u.a. in einer sehr geringen Wasseraufnahme der Polymere und Zusammensetzungen zeigt. Daneben zeichnen sich die unter Verwendung der Monomere der Komponente (b1) erhältlichen Polymere durch eine hohe mechanische Stabilität aus, die sich u.a. in einer hohen Biegefestigkeit der Polymere zeigt. Die erfindungsgemäß vorzugsweise zu verwendenden Monomere der Komponente (b1), insbesondere gemäß den besonders bevorzugten Ausgestaltungen und Ausführungsformen, lassen sich zu Polymeren verarbeiten, die sowohl eine geringe Wasseraufnahme als auch eine hohe Biegefestigkeit aufweisen.

Die Monomere der Komponente (b1) sind mit den weiteren Monomeren der Komponente (b2) copolymerisierbar,

wobei die ausgehärteten Polymere bzw. Formstoffe einen geringen Schrumpf, eine gute Haftung auf verschiedenen Untergründen, eine hohe Hydrolysebeständigkeit, eine geringe Wasseraufnahme und eine hohe mechanische Festigkeit aufweisen. Die genannten Eigenschaften sind insbesondere im Bereich der Dentaltechnik wichtig.

Insbesondere die bevorzugten und besonders bevorzugten zu verwendenden Verbindungen der Komponente (b1) ermöglichen einen hohen Vernetzungsgrad und sind ferner vorzugsweise radikalisch vernetzbar. Wegen ihrer hochfunktionalisierten Struktur weisen sie eine hohe Vernetzungs- und Polymerisationswahrscheinlichkeit auf.

Bevorzugt einzusetzende Verbindungen der Komponente (b1) sind solche, wobei Q ein polyalicyclisches Strukturelement bedeutet, vorzugsweise ein gesättigtes polyalicyclisches Strukturelement, das ausgewählt ist aus der Gruppe bestehend aus bicyclischen oder tricyclischen Kohlenwasserstoffresten, wobei vorzugsweise keines der nicht durch Substituenten $Y_xZ_e$ substituierten Wasserstoffatome dieses polyalicyclischen Strukturelements Q substituiert ist.

Besonders bevorzugt sind Monomere $Q(Y_xZ_e)_b$, deren polyalicyclisches Strukturelement Q sich von einem der folgenden tricyclischen Kohlenwasserstoffe ableitet: Tricyclo[5.2.1.0$^{2,6}$]decan (TCD), Tricyclo[5.2.1.0$^{2,6}$]dec-3-en oder Tricyclo[3.3.1.1$^{3,7}$]decan (Adamantan), d.h. bevorzugt sind Verbindungen, die ein TCD - Gerüst, ein Tricyclo[5.2.1.0$^{2,6}$]dec-3-en - Gerüst oder ein Adamantan-Gerüst aufweisen.

Bei den genannten besonders bevorzugt einzusetzenden Verbindungen, in denen das Strukturelement Q einen Tricyclo[5.2.1.0$^{2,6}$]decan-Rest, einen Tricyclo[5.2.1.0$^{2,6}$]dec-3-en-Rest, einen Tricyclo[3.3.1.1$^{3,7}$]decan-Rest oder einen Bicyclo[2.2.1]heptan-Rest bedeutet, handelt es sich vorzugsweise um solche mit einem Tricyclo[5.2.1.0$^{2,6}$]decan-Gerüst, einem Tricyclo[5.2.1.0$^{2,6}$]dec-3-en-Gerüst, einem Tricyclo[3.3.1.1$^{3,7}$]decan-Gerüst bzw. einem Bicyclo[2.2.1]heptan-Gerüst, bei dem jeweils keines der nicht durch Substituenten $Y_xZ_e$ substituierten Wasserstoffatome dieses polyalicyclischen Strukturelements Q substituiert ist.

Besonders bevorzugt einzusetzende Verbindungen $Q(Y_xZ_e)_b$ der Komponente (b1) sind solche, wobei das Strukturelement Q einen Tricyclo[5.2.1.0$^{2,6}$]decan-Rest, einen Tricyclo[5.2.1.0$^{2,6}$]dec-3-en-Rest, einen Tricyclo[3.3.1.1$^{3,7}$]decan-Rest oder einen Bicyclo[2.2.1]heptan-Rest bedeutet, ganz besonders bevorzugt bedeutet das Strukturelement Q einen Tricyclo[5.2.1.0$^{2,6}$]decan-Rest, einen Tricyclo[5.2.1.0$^{2,6}$]dec-3-en-Rest. Besonders bevorzugt einzusetzende Verbindungen $Q(Y_xZ_e)_b$ der Komponente (b1) sind solche, in denen das Strukturelement Q ein Tricyclo[5.2.1.0$^{2,6}$]-decan- oder Tricyclo[5.2.1.0$^{2,6}$]-decen-Strukturelement ist und Z vorzugsweise ausgewählt ist aus der Gruppe bestehend aus $-O-(C=O)-CH=CH_2$ und $-O-(C=O)-C(CH_3)=CH_2$, wobei wiederum die Gruppe Z besonders bevorzugt $-O-(C=O)-C(CH_3)=CH_2$ ist.

Besonders bevorzugte Zusammensetzungen enthalten als Komponente (b1) ein, zwei oder mehr Verbindungen der Struktur $Q(Y_xZ_e)_b$, die jeweils ein Tricyclo[5.2.1.0$^{2,6}$]-decan- oder Tricyclo[5.2.1.0$^{2,6}$]-decen-Strukturelement aufweisen und Z vorzugsweise ausgewählt ist aus der Gruppe bestehend aus $-O-(C=O)-CH=CH_2$ und $-O-(C=O)-C(CH_3)=CH_2$, wobei wiederum die Gruppe Z besonders bevorzugt $-O-(C=O)-C(CH_3)=CH_2$ ist.

Bevorzugt eingesetzt werden Methacrylsäure- oder Acrylsäureester mit einem Tricyclo[5.2.1.0$^{2,6}$]-decan- oder Tricyclo[5.2.1.0$^{2,6}$]-decen-Strukturelement, ausgewählt aus der Gruppe bestehend aus

- 8,9-Bis(acryloxymethyl)tricyclo[5.2.1.0$^{2,6}$]decan
- 8,9-Bis(methacryloyloxymethyl)tricyclo[5.2.1.0$^{2,6}$]decan
- 8,9-[Bis(2-vinyloxyethyl)oxymethyl]tricyclo[5.2.1.0$^{2,6}$]dec-3-en
- 8,9-[Bis(2-vinyloxyethyl)oxymethyl]tricyclo[5.2.1.0$^{2,6}$]decan
- 8-Hydroxymethyl-9-(2-vinyloxyethyl)oxymethyl]tricyclo[5.2.1.0$^{2,6}$]dec-3-en
- 9-Hydroxymethyl-8-(2-vinyloxyethyl)oxymethyl]tricyclo[5.2.1.0$^{2,6}$]dec-3-en
- 8,9-Bis(acryloyloxymethyl)tricyclo[5.2.1.0$^{2,6}$]dec-3-en
- 8,9-Bis(methacryloyloxymethyl)tricyclo[5.2.1.0$^{2,6}$]dec-3-en
- Diacrylsäure- oder Dimethacrylsäureester von Verbindungen ausgewählt aus der Gruppe bestehend aus:
  - 3,8-Dihydroxymethyl- tricyclo[5.2.1.0$^{2,6}$]decan
  - 3,9-Dihydroxymethyltricyclo- [5.2.1.0$^{2,6}$]decan
  - 4,8-Dihydroxymethyltricyclo[5.2.1.0$^{2,6}$]decan

- 3,8-Dihydroxytricyclo[5.2.1.$0^{2,6}$]decan
- 3,9-Dihydroxytricyclo-[5.2.1.$0^{2,6}$]decan
- 4,8-Dihydroxytricyclo[5.2.1.$0^{2,6}$]decan

- Methacrylsäure- oder Acrylsäureester von Verbindungen aus der Gruppe bestehend aus:
  - Poly(hydroxymethyl-tricyclo[5.2.1.$0^{2,6}$]decanyl-siloxanen
  - oxyalkyliertes Bishydroxymethyltricyclo[5.2.1.$0^{2,6}$]decan
  - oxyalkyliertes Bishydroxytricyclo[5.2.1.$0^{2,6}$]decan

- Urethan- oder Harnstoffgruppen enthaltende Methacrylsäure- oder Acrylsäureester von Verbindungen ausgewählt aus der Gruppe bestehend aus:
  - 3,8-Dihydroxymethyl- tricyclo[5.2.1.$0^{2,6}$]decan
  - 4,8-Dihydroxymethyl- tricyclo[5.2.1.$0^{2,6}$]decan
  - 3,9-Dihydroxymethyl- tricyclo[5.2.1.$0^{2,6}$]decan
  - 4,9-Dihydroxymethyl- tricyclo[5.2.1.$0^{2,6}$]decan

Hierbei kann in den genannten Verbindungen Wasserstoff am Tricyclo[5.2.1.$0^{2,6}$]-decan- oder Tricyclo[5.2.1.$0^{2,6}$]-decen-Rest durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sein.

Viele der vorstehend aufgelisteten radikalisch polymerisierbaren Methacrylsäure- oder Acrylsäureester mit einem TCD-Strukturelement sind aus dem Stand der Technik bekannt.

Y ist vorzugsweise ein Strukturelement, welches in der Struktur $Q(Y_xZ_e)_b$ das polyalicyclische Strukturelement Q mit e Strukturelementen Z verknüpft und ein Strukturelement enthält oder aus diesem besteht, das ausgewählt ist aus der Gruppe bestehend aus

$-CH_2-$ , 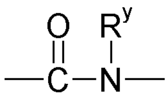 und 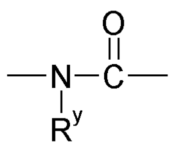

wobei $R^y$ einen sonstigen Rest der Verbindung bedeutet und

wobei die jeweils im Formelbild links angeordnete Bindung dem Strukturelement Q und die rechts angeordnete Bindung dem Strukturelement Z näher ist.

Der sonstige Rest $R^y$ einer bevorzugt einzusetzenden Verbindung der Struktur $Q(Y_xZ_e)_b$ ist vorzugsweise ausgewählt aus der Gruppe bestehend aus Wasserstoff, linearen, verzweigten oder Ringe umfassenden Strukturelementen mit 1 bis 50 C-Atomen und 0 bis 12 Heteroatomen, wobei die gegebenenfalls vorhandenen Heteroatome vorzugsweise gewählt sind aus der Gruppe bestehend aus N und O.

Der sonstige Rest $R^y$ ist dabei bevorzugt ausgewählt aus der Gruppe bestehend aus Wasserstoff, linearen, verzweigten oder Ringe umfassenden Strukturelementen mit 1 bis 40 C-Atomen und 0 bis 10 Heteroatomen, wobei die gegebenenfalls vorhandenen Heteroatome vorzugsweise gewählt sind aus der Gruppe bestehend aus N und O.

Der sonstige Rest $R^y$ ist dabei besonders bevorzugt ausgewählt aus der Gruppe bestehend aus Wasserstoff, linearen, verzweigten oder Ringe umfassenden Strukturelementen mit 1 bis 35 C-Atomen und 1 bis 10 Heteroatomen, wobei die gegebenenfalls vorhandenen Heteroatome vorzugsweise gewählt sind aus der Gruppe bestehend aus N und O.

Y ist dabei bevorzugt ein Strukturelement, welches ein Strukturelement enthält oder aus diesem besteht, ausgewählt aus der Gruppe bestehend aus

$-CH_2-$

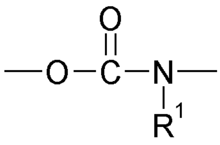
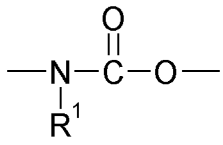
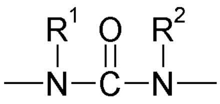
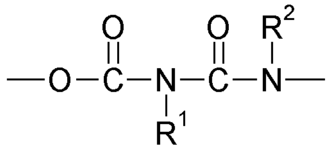
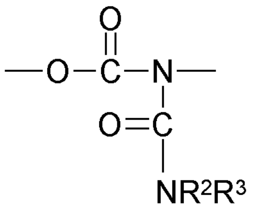
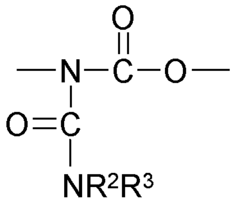
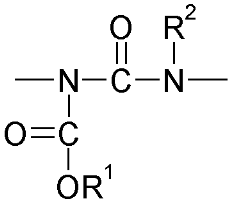
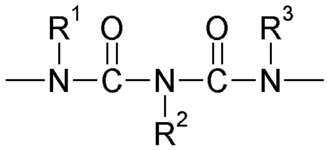
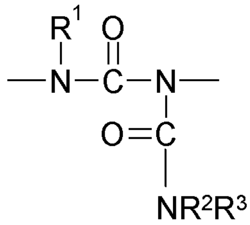
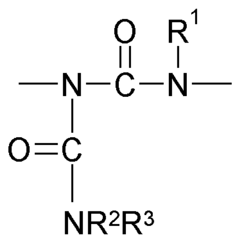
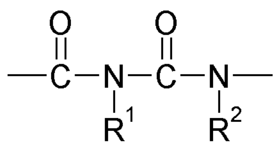
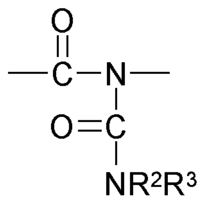
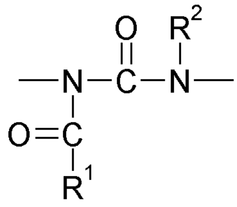
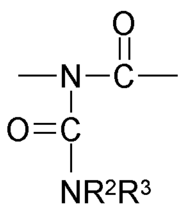
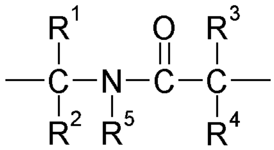
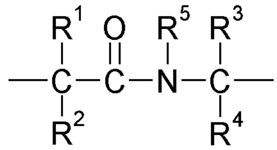

wobei $R^1$, $R^2$, $R^3$, $R^4$ und $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Die oben genannten sonstigen Reste $R^1$, $R^2$, $R^3$, $R^4$ bzw. $R^5$ einer bevorzugt einzusetzenden Verbindung der Struktur $Q(Y_xZ_e)_b$ sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$, $R^3$, $R^4$ bzw. $R^5$ sind dabei, jeweils unabhängig voneinander, bevorzugt ausgewählt

aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$, $R^3$, $R^4$ bzw. $R^5$ sind dabei, jeweils unabhängig voneinander, besonders bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

In mit vergleichsweise geringem Aufwand synthetisierbaren bevorzugt einzusetzenden Verbindungen der Struktur $Q(Y_xZ_e)_b$ ist Y ein Strukturelement, welches ein Strukturelement enthält oder aus diesem besteht, das ausgewählt ist aus der Gruppe bestehend aus

$-CH_2-$

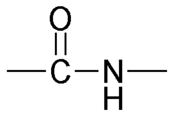
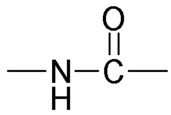

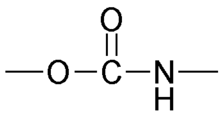
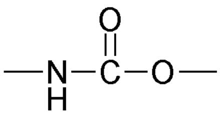
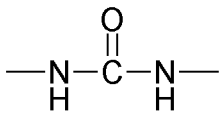

wobei die jeweils im Formelbild links angeordnete Bindung dem Strukturelement Q und die rechts angeordnete Bindung dem Strukturelement Z näher ist.

Die bevorzugt einzusetzenden Verbindungen der Struktur $Q(Y_xZ_e)_b$ können gemäß dem Fachmann bekannten Herstellungsverfahren erhalten werden.

Bevorzugt einzusetzende Verbindungen der Struktur $Q(Y_xZ_e)_b$ mit einem Amid-Strukturelement können beispielsweise durch Umsetzung von (i) einer Edukt-Verbindung mit einer Isocyanatgruppe und (ii) einer Edukt-Verbindung mit einer Carbonsäuregruppe erhalten werden.

Bevorzugt einzusetzende Verbindungen der Struktur $Q(Y_xZ_e)_b$ mit einem Urethan-Strukturelement können beispielsweise durch Umsetzung von (i) einer Edukt-Verbindung mit einer Isocyanatgruppe und einer Edukt-Verbindung mit einer Alkoholgruppe erhalten werden.

Bevorzugt einzusetzende Verbindungen der Struktur $Q(Y_xZ_e)_b$ mit einem Harnstoff-Strukturelement können beispielsweise durch Umsetzung von (i) einer Edukt-Verbindung mit einer Isocyanatgruppe und (ii) einer Edukt-Verbindung mit einer Aminogruppe erhalten werden.

Bevorzugt einzusetzende Verbindungen der Struktur $Q(Y_xZ_e)_b$ mit einem Allophanat-Strukturelement können beispielsweise durch Umsetzung von (i) einer Edukt-Verbindung mit einer Urethangruppe und (ii) einer Edukt-Verbindung mit einer Isocyanatgruppe erhalten werden.

Bevorzugt einzusetzende Verbindungen der Struktur $Q(Y_xZ_e)_b$ mit einem Biuret-Strukturelement können beispielsweise durch Umsetzung von (i) einer Edukt-Verbindung mit einer Harnstoffgruppe und (ii) einer Edukt-Verbindung mit einer Isocyanatgruppe erhalten werden.

Bevorzugt einzusetzende Verbindungen der Struktur $Q(Y_xZ_e)_b$ mit einem *N*-Acylharnstoff-Strukturelement können beispielsweise durch Umsetzung von (i) einer Edukt- Verbindung mit einer Amidgruppe und (ii) einer Edukt-Verbindung mit einer Isocyanatgruppe erhalten werden.

In einer besonders bevorzugten Ausgestaltung einer erfindungsgemäß anzuwendenden Zusammensetzung ist Komponente (b1) so gewählt, dass diese umfasst oder besteht aus Bis(methacryloyloxymethyl)tricyclo[5.2.1.$0^{2,6}$]decan und/oder Bis(acryloyloxymethyl)tricyclo [5.2.1.$0^{2,6}$]decan.

In erfindungsgemäß anzuwendenden Zusammensetzungen sind die Methacrylsäureester wegen ihrer höheren biologischen Verträglichkeit gegenüber den entsprechenden Acrylsäureestern bevorzugt, d.h. dass Z in Verbindungen der Struktur $Q(Y_xZ_e)_b$ bevorzugt $-O-(C=O)-C(CH_3)=CH_2$ bedeutet.

Unter (Meth)acryl ist im Rahmen des vorliegenden Textes sowohl Acryl als auch Methacryl zu verstehen.

Besonders geeignet zum Einsatz als Monomere in erfindungsgemäß anzuwendenden Zusammensetzungen sind Verbindungen der Struktur $Q(Y_xZ_e)_b$ mit x = 1 mit ein, zwei, drei, vier oder mehr funktionellen Gruppen, welche ausgewählt sind aus der Gruppe bestehend aus *N*-Acylharnstoff, Allophanat und Biuret,
wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $YZ_e$ substituierten Wasserstoffatome dieses polyalicyclischen Strukturelements Q durch Alkylgruppen, Alkoxygruppen, Halogenatome oder Trifluormethylgruppen substituiert ist bzw. sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,

  $-(C=O)-CH=CH_2$, $-(C=O)-C(CH_3)=CH_2$,

  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(Y_xZ_e)_b$ mit x = 1 das polyalicyclische Strukturelement Q mit e Strukturelementen Z verknüpft und ein Strukturelement enthält oder aus diesem besteht, ausgewählt aus der Gruppe bestehend aus

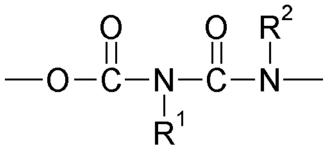
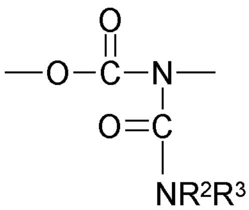
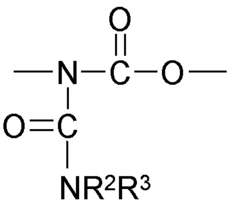

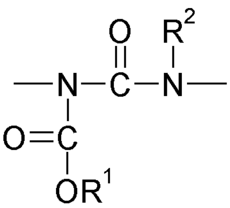
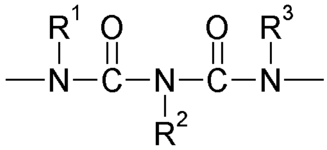
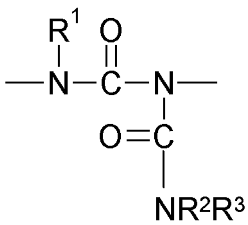

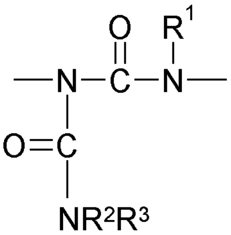
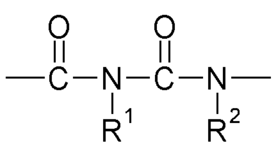
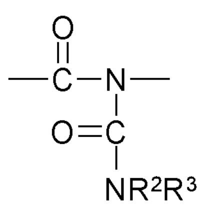

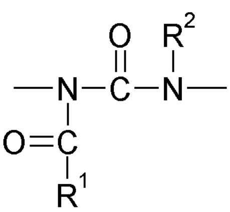
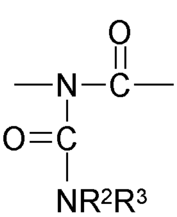

wobei $R^1$, $R^2$ und $R^3$ sonstige Reste der Verbindung bedeuten, wobei die jeweils im Formelbild links angeordnete Bindung dem Strukturelement Q und die rechts angeordnete Bindung dem Strukturelement Z näher ist.

Vorzugsweise umfasst eine solche Verbindung der Struktur $Q(Y_xZ_e)_b$ mit x = 1 zwei, drei, vier oder mehr funktionelle Gruppen ausgewählt aus der Gruppe bestehend aus N-Acylharnstoff, Allophanat und Biuret.

In einer bevorzugten Ausgestaltung bedeutet jeder Index e eine natürliche Zahl, die unabhängig von etwaigen weiteren Indizes e ausgewählt ist aus der Gruppe der natürlichen Zahlen 2, 3 und 4.

Die oben genannten sonstigen Reste $R^1$, $R^2$ bzw. $R^3$ einer solchen Verbindung sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$ bzw. $R^3$ einer solchen bevorzugt einzusetzenden Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O. Die sonstigen Reste $R^1$, $R^2$ bzw. $R^3$ einer solchen bevorzugt einzusetzenden Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

Eine in einer erfindungsgemäß anzuwendenden Zusammensetzung einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, ist vorzugsweise herstellbar durch Umsetzung eines ersten Umsetzungsproduktes, welches das Umsetzungsprodukt ist aus einer ersten Reaktion von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,
wobei gilt:

- R bedeutet jeweils unabhängig von etwaigen weiteren R ein Wasserstoffatom oder einen Alkylrest;
- m ist eine natürliche Zahl ausgewählt aus der Gruppe der natürlichen Zahlen von 0 bis 10,
- jeder Index n ist eine natürliche Zahl, die unabhängig von etwaigen weiteren Indizes n ausgewählt ist aus der Gruppe bestehend aus 0 und 1,

wobei die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Eine bevorzugt einzusetzende Verbindung gemäß einer bevorzugten Ausgestaltung ist ein zweites Umsetzungsprodukt aus einer Reaktion von dem obigen ersten Umsetzungsprodukt mit

C) einer weiteren Verbindung gemäß A) oder B), wobei jede weitere Verbindung gemäß A) bzw. B) dieselbe Bedeutung hat wie in der ersten Reaktion,
und/oder

wobei die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

In einer bevorzugten Ausführungsform ist m = 0.

In bevorzugt einzusetzenden Verbindungen erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

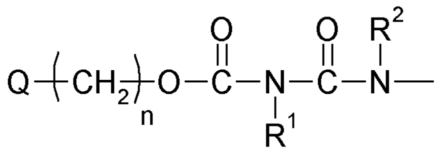

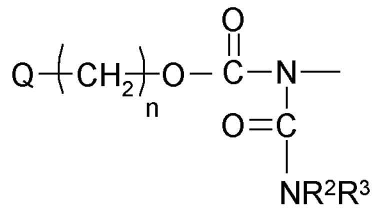

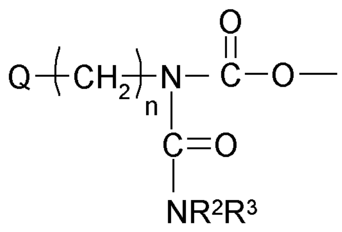

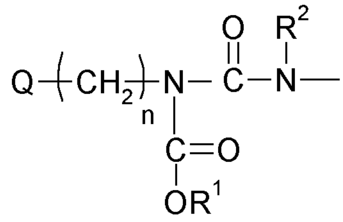

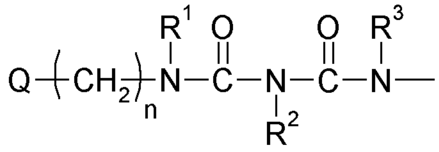

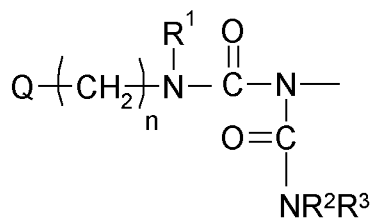

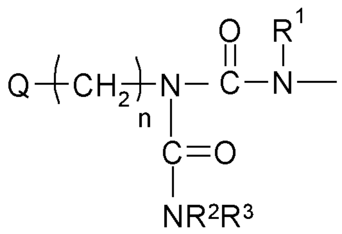

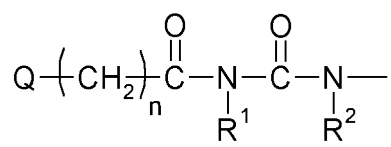

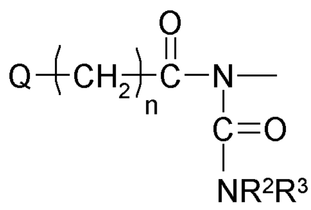

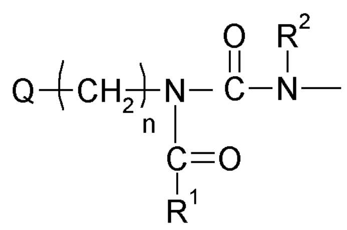

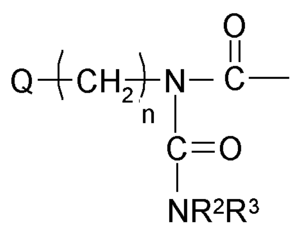

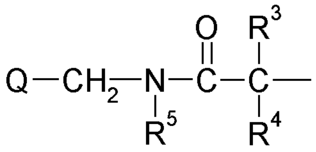

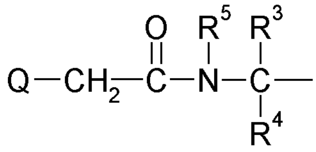

wobei $R^1$, $R^2$, $R^3$ $R^4$, $R^5$ sonstige Reste der Verbindung bedeuten und Q sowie der Index n die oben angegebene Bedeutung haben.

Die jeweils im Formelbild rechts angeordnete Bindung ist dem Strukturelement Z näher.

In einer bevorzugten Ausgestaltung umfasst eine bevorzugt einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, ein oder mehrere Strukturelemente ausgewählt aus der Gruppe bestehend aus

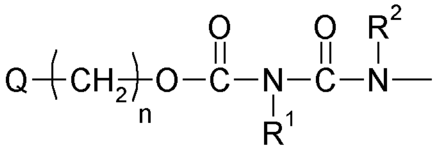

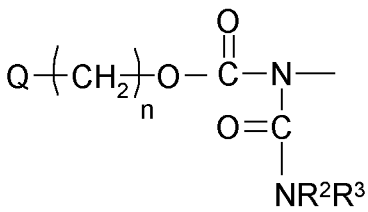

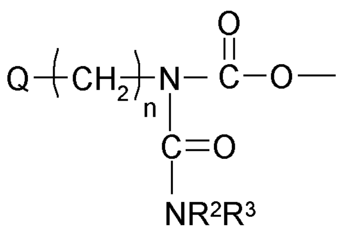

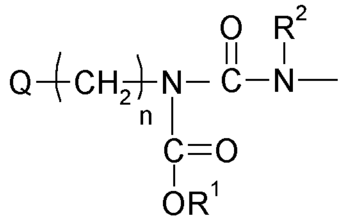

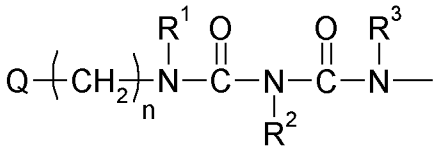

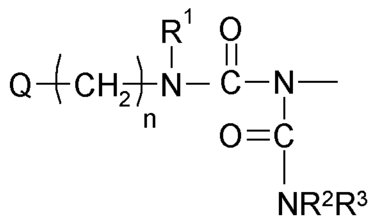

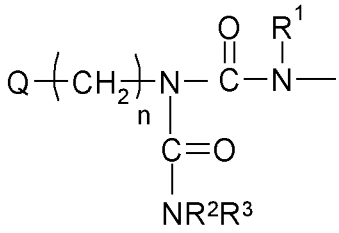

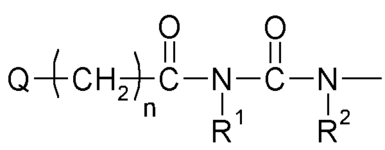

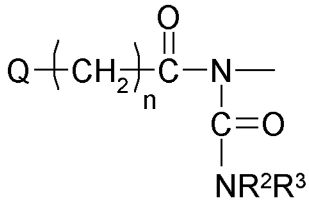

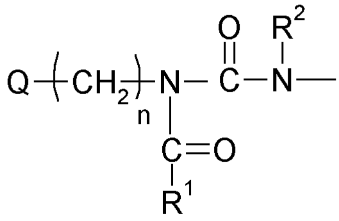

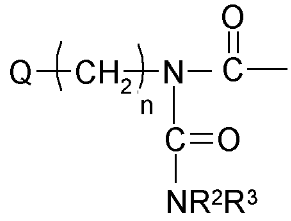

wobei $R^1$, $R^2$ und $R^3$ sonstige Reste der Verbindung bedeuten (und vorzugsweise die oben genannte bevorzugte Bedeutung haben) und Q die oben genannte Bedeutung hat und der Index n ausgewählt ist aus der Gruppe bestehend

aus 0 und 1.

Wie oben bereits erwähnt sind bevorzugt einzusetzendeVerbindungen solche, wobei Q ein gesättigtes polyalicyclisches Strukturelement bedeutet, das ausgewählt ist aus der Gruppe bestehend aus bicyclischen und tricyclischen Kohlenwasserstoffresten, wobei vorzugsweise keines der nicht durch Substituenten $Y_xZ_e$ substituierten Wasserstoffatome dieses polyalicyclischen Strukturelements Q substituiert ist.

Besonders bevorzugt einzusetzende Verbindungen sind solche, wobei das Strukturelement Q der Verbindungen der Struktur $Q(Y_xZ_e)_b$ der Komponente (b1) einen Tricyclo[5.2.1.0$^{2,6}$]decan-Rest, einen Tricyclo[5.2.1.0$^{2,6}$]dec-3-en-Rest, einen Tricyclo[3.3.1.1$^{3,7}$]decan-Rest oder einen Bicyclo[2.2.1]heptan-Rest bedeutet.

Bevorzugt einzusetzende Verbindungen der Komponente (b1) sind solche, wobei Q einen tricyclischen Kohlenwasserstoffrest bedeutet, wobei vorzugsweise keines der nicht durch Substituenten $Y_xZ_e$ (wobei jedes Y unabhängig von etwaigen weiteren Strukturelementen Y gewählt ist oder entfällt) substituierten Wasserstoffatome dieses tricyclischen Kohlenwasserstoffrestes substituiert ist.

Besonders bevorzugt einzusetzende Verbindungen sind solche, wobei das Strukturelement Q einen Tricyclo[5.2.1.0$^{2,6}$]decan-Rest, einen Tricyclo[5.2.1.0$^{2,6}$]dec-3-en-Rest oder einen Tricyclo[3.3.1.1$^{3,7}$]decan-Rest bedeutet, weiter bevorzugt einen Tricyclo[5.2.1.0$^{2,6}$]decan-Rest oder einen Tricyclo[3.3.1.1$^{3,7}$]decan-Rest.

Bevorzugte erfindungsgemäß einzusetzende Verbindungen sind solche, in denen

(i) das Strukturelement Z = $-O-(C=O)-C(CH_3)=CH_2$ bedeutet, wobei die funktionellen Gruppen Allophanat-, Biuret- oder Acylharnstoff-Gruppen sind, da mit diesen Verbindungen besonders gute Ergebnisse erzielt wurden, und/oder

(ii) das Strukturelement Q einen Tricyclo[5.2.1.0$^{2,6}$]decan-Rest bedeutet.

Weiter bevorzugt sind solcheVerbindungen, in denen das Strukturelement Z = $-O-(C=O)-C(CH_3)=CH_2$ bedeutet, wobei die funktionellen Gruppen Allophanat-, Biuret-oder Acylharnstoff-Gruppen sind und das Strukturelement Q einen Tricyclo[5.2.1.0$^{2,6}$]decan-Rest bedeutet.

Bevorzugt sind solcheVerbindungen, in denen sämtliche vorhandenen lichthärtbaren Gruppen dem Strukturelement Z entsprechen.

Bevorzugt sind solcheVerbindungen, in denen sämtliche vorhandenen endständigen polymerisierbaren Gruppen dem Strukturelement Z entsprechen.Eine solche Verbindung kann neben lichthärtbaren Gruppen des Strukturelements Z auch weitere polymerisierbare, vorzugsweise endständige polymerisierbare, Gruppen umfassen, die nicht lichthärtbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.

Weiter bevorzugte Verbindungen sind solche, in denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

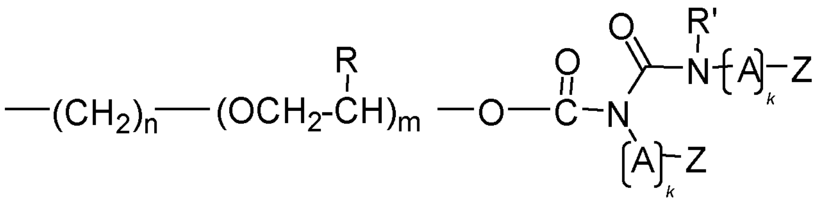

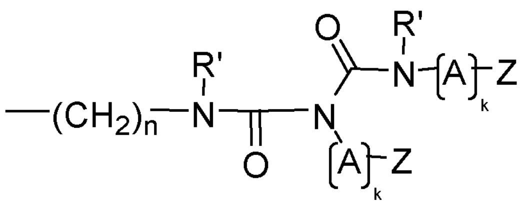

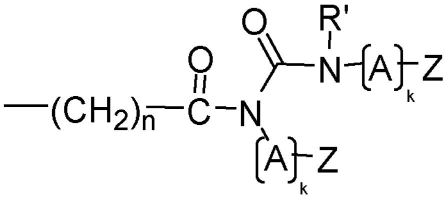

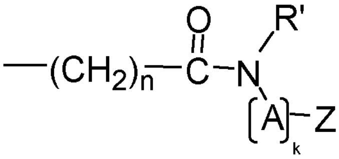

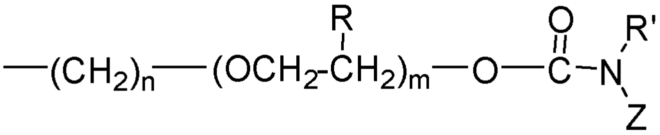

wobei Z, R, m sowie n die obige Bedeutung haben und worin ferner gilt:

- jedes A bedeutet ein zweibindiges organisches Brückenglied,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

In einer bevorzugten Ausführungsform ist m = 0.

Ebenfalls bevorzugte Verbindungen sind solche, bei denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

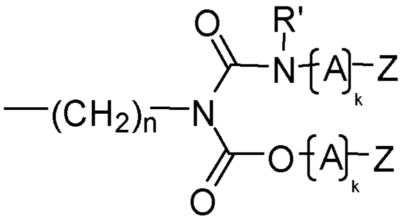

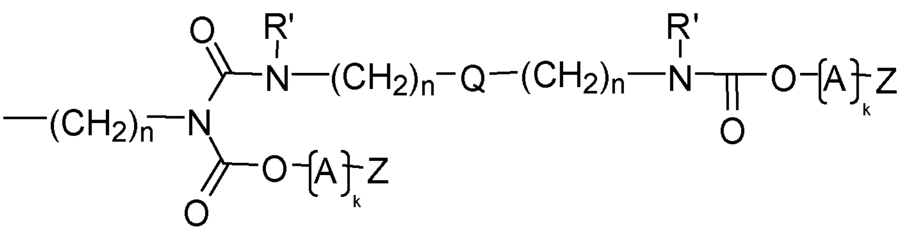

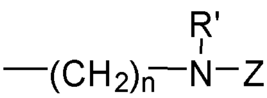

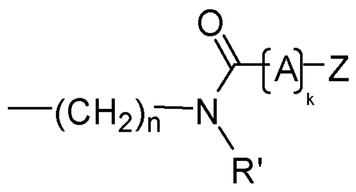

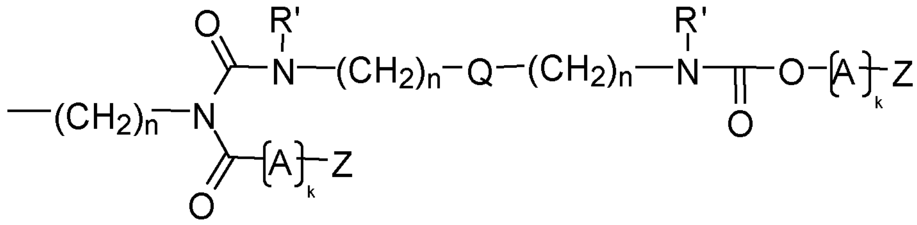

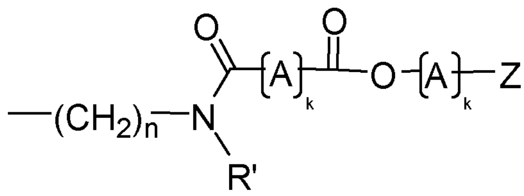

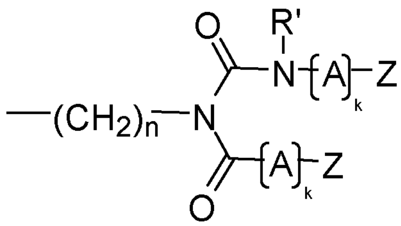

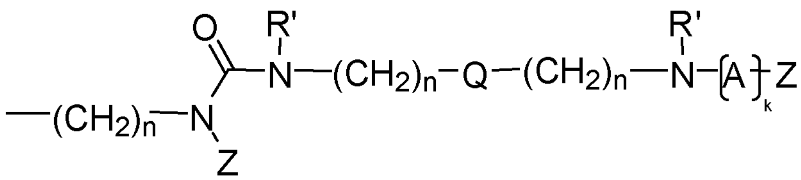

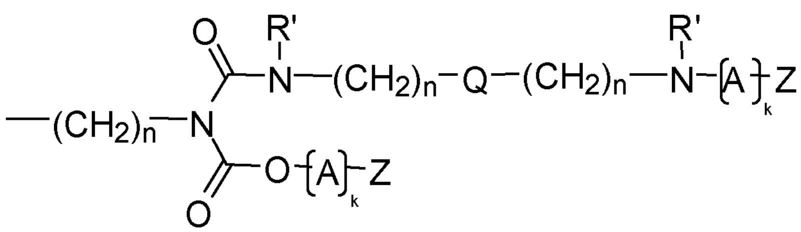

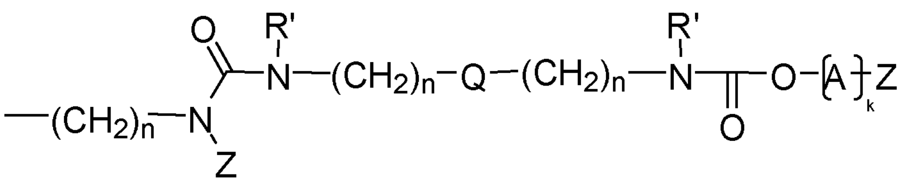

wobei jedes Q unabhängig von etwaigen weiteren Strukturelementen Q die obige Bedeutung hat und wobei Z, A, k und R' sowie n die oben genannte Bedeutung haben.

Besonders geeignet zum Einsatz als Monomere in erfindungsgemäß anzuwendenden Zusammensetzungen ist eine Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, wobei
mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind,
aus der Gruppe bestehend aus

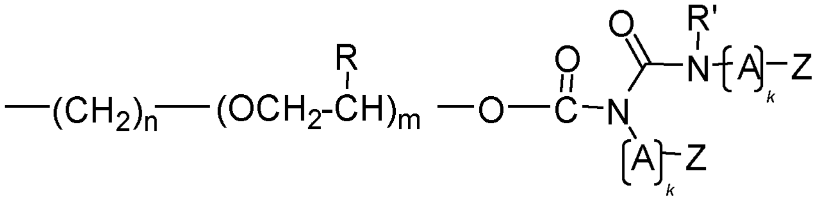

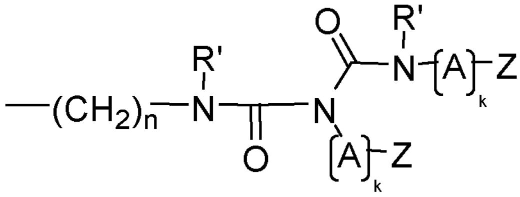

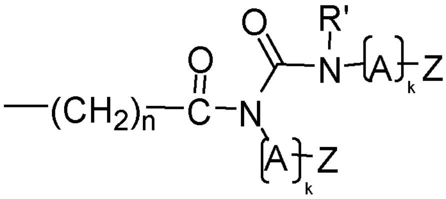

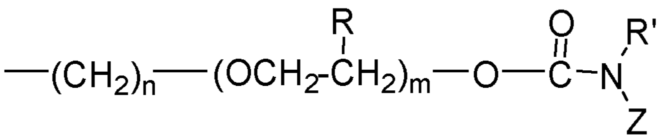

wobei Z, R, m sowie n die obige Bedeutung haben und worin ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R’ bedeutet ein Strukturelement, das unabhängig von etwaigen weiteren Strukturelementen R’ ausgewählt ist aus der Gruppe bestehend aus H und einem Strukturelement (C=O)-NH-$(A)_k$-Z, wobei A, Z und k wiederum die obigen Bedeutungen haben.

In einer bevorzugten Ausgestaltung wird eine Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, wobei
mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind,
aus der Gruppe bestehend aus

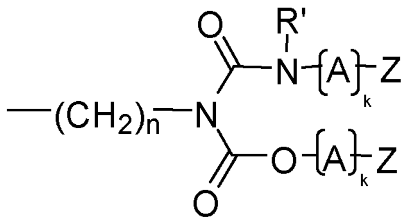

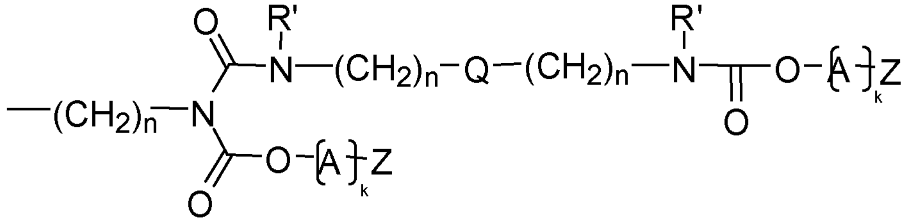

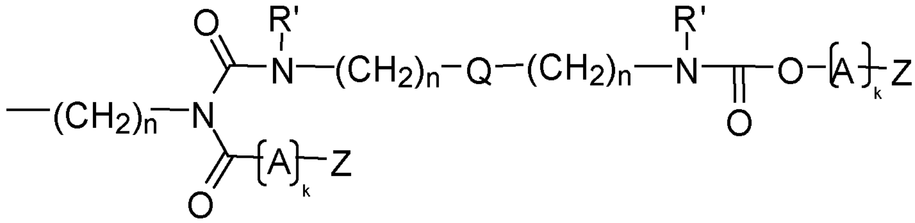

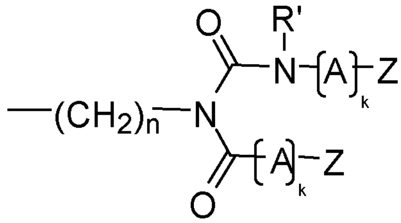

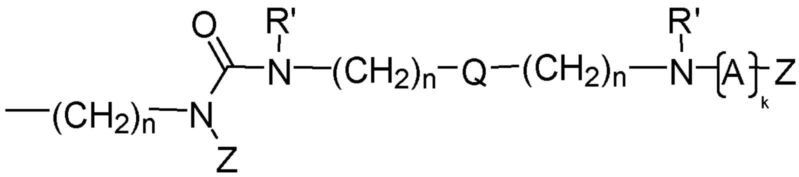

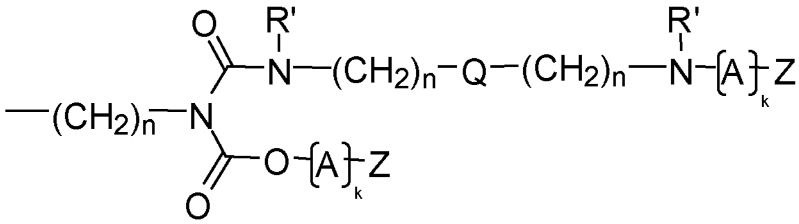

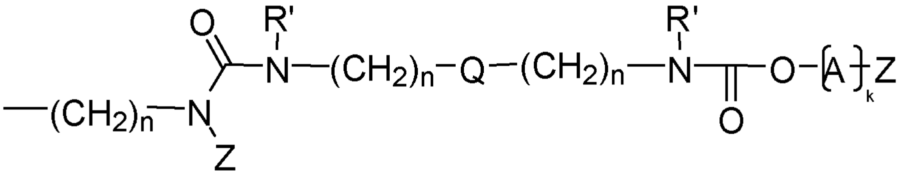

wobei jedes Q unabhängig von etwaigen weiteren Strukturelementen Q die obige Bedeutung hat, und
wobei Z und n die oben genannte Bedeutung haben und wobei ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

Dabei wiederum bevorzugt sind Verbindungen, in denen jedes Strukturelement A unabhängig von etwaigen weiteren Strukturelementen A ausgewählt ist aus der Gruppe bestehend aus linearen, verzweigten oder Ringe umfassenden zweibindigen organischen Brückengliedern mit 1 bis 25 C-Atomen und gegebenenfalls 1 bis 10, vorzugsweise 1 bis 5 Heteroatomen, wobei die gegebenenfalls vorhandenen Heteroatome vorzugsweise gewählt sind aus der Gruppe bestehend aus N und O.

Dabei wiederum bevorzugt ist eine Verbindung, in der jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, vorzugsweise mit 1 bis 5 Heteroatomen, wobei die gegebenenfalls vorhandenen Heteroatome vorzugsweise gewählt sind aus der Gruppe bestehend aus N und O.

Weiter bevorzugt sind Verbindungen, in denen jedes Strukturelement A unabhängig von etwaigen weiteren Strukturelementen A ausgewählt ist aus der Gruppe bestehend aus $C_1$-$C_{20}$-Alkylen, $C_1$-$C_{20}$-Heteroalkylen, $C_3$-$C_{20}$-Cycloalkylen, $C_4$-$C_{20}$-Cycloalkylalkylen, $C_2$-$C_{20}$-Alkenylen, $C_3$-$C_{20}$-Cycloalkenylen, $C_4$-$C_{20}$-Cycloalkenylalkylen, $C_4$-$C_{20}$-Cycloalkenylenalkylen, $C_3$-$C_{25}$-Arylen, $C_2$-$C_{25}$-Heteroarylen, $C_4$-$C_{25}$-Arylalkylen, $C_4$-$C_{25}$-Arylenalkylen, $C_4$-$C_{25}$-Arylheteroalkylen, $C_4$-$C_{25}$-Arylenheteroalkylen.

In bevorzugten Ausgestaltungen umfasst Strukturelement A eine oder mehrere der folgenden Atome oder Atomgruppen:

$$-O-,\ -O-Ar^1-CR^6R^7-Ar^2-O-,\ -NR^8-,\ -N-(C=O)-,\ -NH-(C=O)-O-,\ -NH-C(=O)-NH-$$

wobei gilt:

$Ar^1$ und $Ar^2$ bedeuten unabhängig voneinander einen gegebenenfalls substituierten, dabei vorzugsweise einen ein oder mehrfach mit C1-C4-Alkylresten substituierten, aromatischen Ring, dabei wiederum bevorzugt einen Phenylring,

$R^6$, $R^7$ und $R^8$ bedeuten unabhängig voneinander Wasserstoff oder einen C1-C8-Rest, dabei vorzugsweise einen C1-C4-Alkylrest, dabei wiederum bevorzugt Methyl oder Ethyl.

Zur Herstellung der genannten Verbindungen der Struktur $Q(Y_xZ_e)_b$ können vorzugsweise Hydroxylverbindungen von (Meth)acrylaten eingesetzt werden, wobei auch Gemische von Acrylaten und Methacrylaten verwendet werden können. Bevorzugt sind zum Einsatz als Reaktionspartner gemäß Komponente B), C) oder D):

Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritol-tri(meth)acrylat, wie Dipentaerythrit, wie beispielsweise Dipentaerythritol-penta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc.

Zur Herstellung der Verbindungen können als Komponente B) auch Isocyanate eingesetzt werden. Bevorzugt sind dabei Mono- und Diisocyanate.

Bevorzugte Diisocyanate sind gewählt aus der Gruppe bestehend aus Cyclohexandiisocyanat, Methylcyclohexandiisocyanat, Ethylcyclohexandiisocyanat, Propylcyclohexandiisocyanat, Methyldiethylcyclohexandiisocyanat, Phenylendiisocyanat, Toluylendiisocyanat, Bis(isocyanatophenyl)methan, Propandiisocyanat, Butandiisocyanat, Pentandiisocyanat, Hexandiisocyanat, wie Hexamethylendiisocyanat oder 1,5-Diisocyanato-2-methylpentan, Heptandiisocyanat, Octandiisocyanat, Nonandiisocyanat, wie 1,6-Diisocyanato-2,4,4-trimethylhexan oder 1,6-Diisocyanato-2,2,4-trimethylhexan, Nonantriisocyanat, wie 4-Isocyanatomethyl-1,8-octandiisocyanat, Decandi- und triisocyanat, Undekandi- und -triisocyanat, Dodecandi- und -triisocyanat, Isophorondiisocyanat, Dicyclohexylmethan-4,4'-diisocyanat, Isocyanatomethylmethylcyclohexylisocyanat, 1,3-Bis(isocyanatomethyl)cyclohexan oder 1,4-Bis(isocyanatomethyl)cyclohexan.

Bevorzugte Monoisocyanate sind (Meth)acryloylisocyanat und (Meth)acryl-C2-C8-alkylisocyanate (d.h. (Meth)acrylalkylisocyanate mit Alkylspacern, die über 2 bis 8, besonders bevorzugt 2 bis 6 Kohlenstoffatome verfügen), dabei wiederum bevorzugt ist (Meth)acrylethylisocyanat (2-Isocyanatoethyl(meth)acrylat).

Außerdem haben sich als Komponente B) Monoisocyanate vorteilhaft erwiesen, die Umsetzungsprodukte sind aus Amino- bzw. Hydroxyalkyl(meth)acrylaten, deren Alkylspacer über 1 bis 12, bevorzugt 2 bis 8, besonders bevorzugt 2 bis 6 Kohlenstoffatome verfügen, und Diisocyanaten.

Vorzugsweise wird dazu ein vorstehend genanntes Diisocyanat äquimolar mit einer (oben als bevorzugt gekennzeichneten) Amino- oder Hydroxylalkylverbindung eines (Meth)acrylats umgesetzt, wobei die Hydroxylalkylverbindungen wiederum vorzugsweise gewählt sind aus der Gruppe bestehend aus Hydroxyethyl(meth)acrylat, Hydroxypropyl(meth)acrylat, Hydroxybutyl(meth)acrylat, Hydroxypentyl(meth)acrylat, Hydroxyhexyl(meth)acrylat.

Als Beispiele seien die Umsetzungsprodukte im molaren Verhältnis von 1 : 1 von Hydroxyethylmethacrylat mit Isophorondiisocyanat, Dicyclohexylmethan-4,4'-diisocyanat oder Hexamethylendiisocyanat genannt.

Die vorliegende Erfindung betrifft ferner die Verwendung bzw. den Einsatz einer vorstehend genannten Verbindung, vorzugsweise in einer der als bevorzugt bzw. besonders bevorzugt gekennzeichneten Ausgestaltungen, in einem erfindungsgemäßen bzw. erfindungsgemäß für die genannten Zwecke anzuwendende Zusammensetzung, vorzugsweise in einer dentalen Zusammensetzung.

Die vorliegende Erfindung betrifft auch die Verwendung einer vorstehend genannten Verbindung der Struktur $Q(Y_xZ_e)_b$, vorzugsweise in einer der als bevorzugt bzw. besonders bevorzugt gekennzeichneten Ausgestaltungen, zur Herstellung einer Zusammensetzung, vorzugsweise einer dentalen Zusammensetzung, insbesondere eines dentalen Versiegelungsmaterials zur Versiegelung von Fissuren, zur Versiegelung von Grübchen und zur Versiegelung kariöser Läsionen.

Besonders bevorzugte Verbindung $Q(Y_xZ_e)_b$ mit x = 1, die als Bestandteil (b1) einer erfindungsgemäßen Zusammensetzung eingesetzt werden können, lassen sich mit dem nachfolgenden Verfahren herstellen. Das Verfahren umfasst die folgenden Schritte:

In einer ersten Reaktion Umsetzen von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$, vorzugsweise einer Verbindung der Struktur $QG_b$, worin jedes G eine reaktive Gruppe bedeutet, die unabhängig von weiteren Gruppen G ausgewählt ist aus der Gruppe bestehend aus $-NH_2$, $-CH_2NH_2$, -OH, $-CH_2OH$, -NCO, $-CH_2NCO$, und - COOH,
mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH
zu einem ersten Umsetzungsprodukt,
gegebenenfalls in einer zweiten Reaktion Umsetzen des ersten Umsetzungsproduktes mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,

zu einem zweiten Umsetzungsprodukt
und gegebenenfalls in einer dritten Reaktion Umsetzen des zweiten Umsetzungsproduktes mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
wobei Q, b, Y, Z, und e jeweils die oben genannte Bedeutung haben, und wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;

- m ist eine natürliche Zahl ausgewählt aus der Gruppe der natürlichen Zahlen von 0 bis 10,

- jeder Index n ist eine natürliche Zahl, die unabhängig von etwaigen weiteren Indizes n ausgewählt ist aus der Gruppe bestehend aus 0 und 1,

wobei das Verhältnis der Gesamtzahl von NCO-Gruppen zu der Gesamtzahl von - $NH_2$, -OH und -COOH in der Gesamtzahl von Verbindungen gemäß A) und B) in der ersten, gegebenenfalls zweiten und gegebenenfalls dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

Dabei liegt das Verhältnis der Gesamtzahl von umgesetzten NCO-Gruppen zu der Gesamtzahl von umgesetzten -$NH_2$, -OH und -COOH in der Gesamtzahl von Verbindungen gemäß A) und B) in der ersten, gegebenenfalls zweiten und gegebenenfalls dritten Reaktion bevorzugt im Bereich von 1,1 : 1 bis 5 : 1, weiter bevorzugt im Bereich von 1,25 : 1 bis 4 : 1, besonders bevorzugt im Bereich von 1,5 : 1 bis 3 : 1, und am meisten bevorzugt im Bereich von 2 : 1 bis 2,5 : 1.
Vorzugsweise erfolgt die Reaktion zu dem ersten Umsetzungsprodukt, zu dem zweiten Umsetzungsprodukt und/oder zu dem dritten Umsetzungsprodukt in Gegenwart eines Katalysators.
Bevorzugte Katalysatoren sind dabei tertiäre Amine oder Lewis Säuren, dabei wiederum bevorzugt Metallsalze höherer Fettsäuren, insbesondere Dibutylzinndilaurat oder Zinn(II)octoat.
Die Menge des Katalysators liegt dabei vorzugsweise im Bereich von 0,01 bis 2 Gew.-%, bevorzugt von 0,08 bis 1 Gew.-%, bezogen auf die Gesamtmenge der Reaktanden gemäß A) und B) sowie gegebenenfalls C) und gegebenenfalls D).
Die Reaktion zu dem ersten Umsetzungsprodukt, zu dem zweiten Umsetzungsprodukt und/oder zu dem dritten Umsetzungsprodukt erfolgt vorzugsweise in einem Temperaturbereich von 0 bis 160°C, bevorzugt im Bereich von 30 bis 140°C und besonders bevorzugt im Bereich von 60 bis 120°C. Die Umsetzung wird vorzugsweise bei Normaldruck (1013 mbar) ausgeführt.
Besonders geeignet zum Einsatz als Monomerkomponente in erfindungsgemäß anzuwendenden Zusammensetzungen ist eine Mischung umfassend ein, zwei oder mehr unterschiedliche Verbindungen, herstellbar nach einem erfindungsgemäßen Verfahren.
Im Folgenden wird die Erfindung zunächst für Monomere umfassend tricyclische Strukturelemente Q am Beispiel von Tricyclo[5.2.1.$0^{2,6}$]decan (TCD) - Derivaten im Detail erläutert.

1.) Ausgehend vom Bis(hydroxymethyl)tricyclo[5.2.1.$0^{2,6}$]decan (TCD-Diol)

Bis(hydroxymethyl)tricyclo[5.2.1.$0^{2,6}$]decan ist kommerziell erhältlich, beispielsweise als Dicidolgemisch der isomeren Verbindungen 3,8-Bis(hydroxymethyl)tricyclo[5.2.1.$0^{2,6}$]decan und 4,8-Bis(hydroxymethyl)tricyclo[5.2.1.$0^{2,6}$]decan sowie 3,9- Bis(hydroxymethyl)tricyclo[5.2.1.$0^{2,6}$]decan und 4,9- Bis(hydroxymethyl)tricyclo[5.2.1.$0^{2,6}$]decan.
Die Bis(hydroxymethyl)tricyclo[5.2.1.$0^{2,6}$]decane können, ausgehend von Dicyclopentadien (Tricyclo[5.2.1.$0^{2,6}$]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.$0^{2,6}$]decan. Je nach Synthesepfad können gezielt an unterschiedlichen Positionen substituierte Bis(hydroxymethyl)tricyclo[5.2.1.$0^{2,6}$]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.$0^{2,6}$]decan herstellbar ist. Die DE 103 52 260 B3 beschreibt dagegen Verfahren zur Herstellung von 3(4), 8(9)-Bis(hydroxymethyl)tricyclo[5.2.1.$0^{2,6}$]decan. Die Notation der Positionen der Hydroxymethylgruppen 3(4), 8(9) bedeutet 3 oder 4, 8 oder 9.

Das im Handel erhältliche und als Ausgangsverbindung zur Herstellung von Monomeren einsetzbare Bis(hydroxymethyl)tricyclo[5.2.1.0$^{2,6}$]decan enthält somit Hydroxymethylgruppen sowohl an den Positionen 3 oder 4 als auch in den Stellungen 8 oder 9. Es ist nun möglich, durch Anlagerung von Alkylenoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die Umsetzung der 3(4), 8(9)-Bis(hydroxymethyl)tricyclo[5.2.1.0$^{2,6}$]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.0$^{2,6}$]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

Das erhaltene Reaktionsprodukt (Formel (1)) von 2-Isocyanatoethylmethacrylat mit 3(4), 8(9)-Bis(hydroxymethyl)tricyclo[5.2.1.0$^{2,6}$]decan weist in einer Formulierung nach Aushärtung einen niedrigen Reaktionsschrumpf und eine hohe mechanische Festigkeit auf.

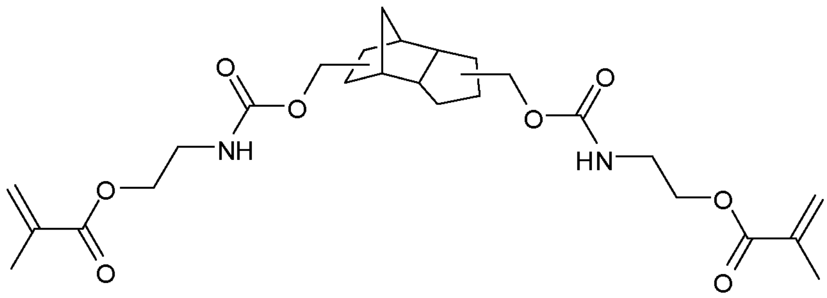

Formel (1)

Das Urethan der Formel (1) besitzt noch zwei reaktionsfähige Wasserstoffatome am Stickstoff, die nun in einer zweiten Umsetzungsstufe mit überschüssigem Isocyanat weiter abreagiert werden unter Bildung einer bevorzugten Verbindung. Es bildet sich hierbei zunächst das Allophanat der Formel (2) als tetrafunktionalisierte, radikalisch vernetzbare Verbindung. Auch dieses Monomer weist wiederum noch umsetzungsfähige Wasserstoffatome am Stickstoff auf, die bei Reaktion mit weiterem Isocyanat das hexafunktionalisierte, radikalisch härtbare Allophanat der Formel (3) bilden.

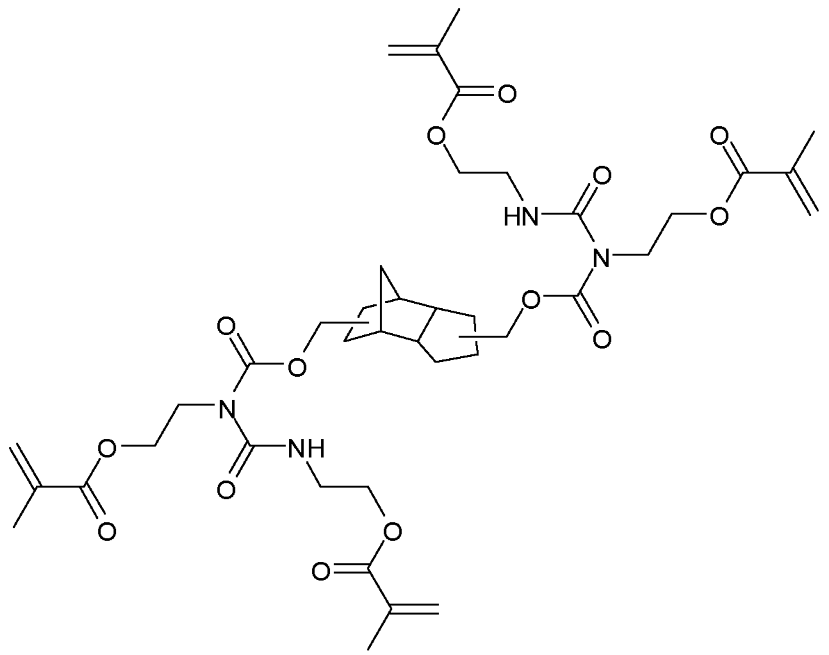

Formel (2)

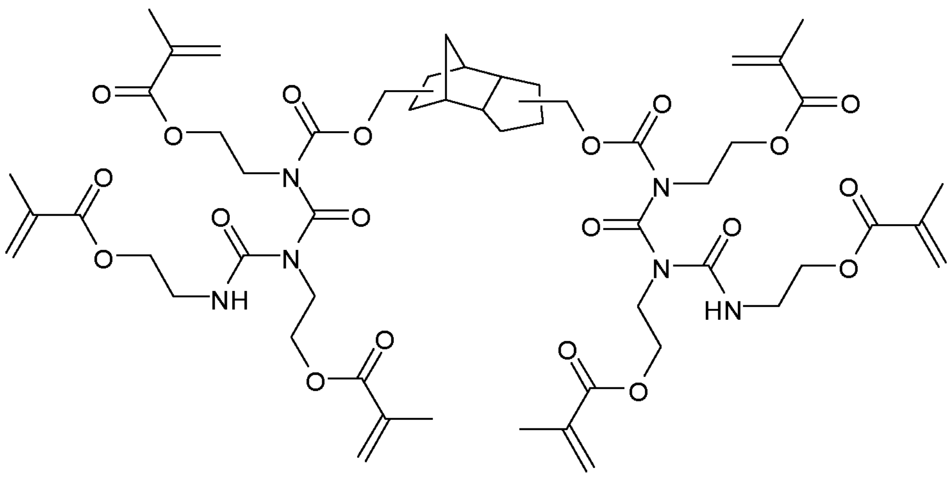

Formel (3)

Alternativ kann das 3(4), 8(9)-Bis(hydroxymethyl)tricyclo[5.2.1.$0^{2,6}$]decan auch mit Methacryloylisocyanat zur Reaktion gebracht werden. Methacryloylisocyanat ist kommerziell oder durch Umsetzung von Methacrylamid mit Oxalylchlorid erhältlich, wie in der EP 0 143 613 B1 beschrieben. Durch Umsetzung von 3(4), 8(9)-Bis(hydroxymethyl)tricyclo[5.2.1.$0^{2,6}$]decan mit Methacryloylisocyanat wird eine Verbindung der Formel (4) erhalten:

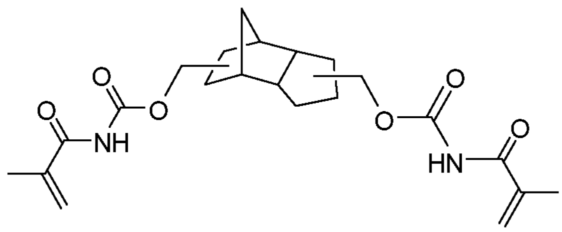

Formel (4)

Die verbleibenden reaktiven Wasserstoffatome am Stickstoff der Verbindung der Formel (4) können anschließend wiederum in Isocyanatreaktionen zu Allophanaten umgesetzt werden. Beispielhaft sei hier das Reaktionsprodukt mit 2-Isocyanatoethylmethacrylat (Formel (5)) gezeigt.

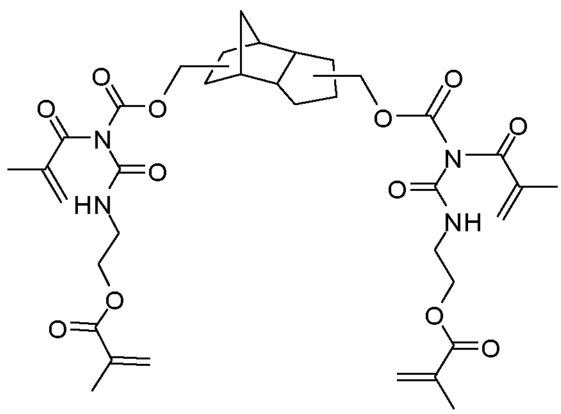

Formel (5)

2.) Ausgehend von 3(4), 8(9)-Bis(Carbonsäure)tricyclo[5.2.1.$0^{2,6}$]decan

Das 3(4), 8(9)-Bis(Carbonsäure)tricyclo[5.2.1.$0^{2,6}$]decan ist durch einfache Oxidation des kommerziell erhältlichen 3(4), 8(9)-Bis(formyl)tricyclo[5.2.1.$0^{2,6}$]decans herstellbar. Umsetzung der Dicarbonsäure mit 2-Isocyanatoethylmethacrylat ergibt das Amid der Formel (8):

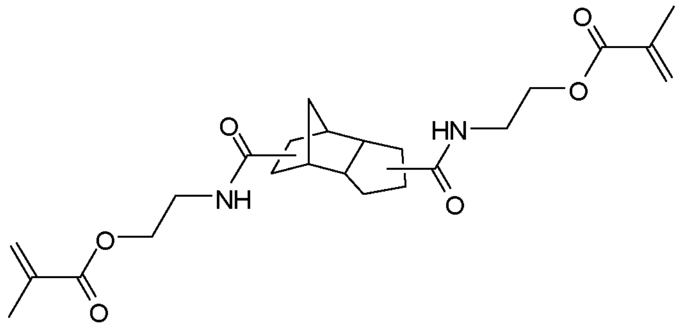

Formel (8)

Weitere Umsetzung der beiden reaktionsfähigen Amid-Wasserstoffatome des Amids der Formel (8) mit 2-Isocyanatoethylmethacrylat ergibt den Acylharnstoff der Formel (9).

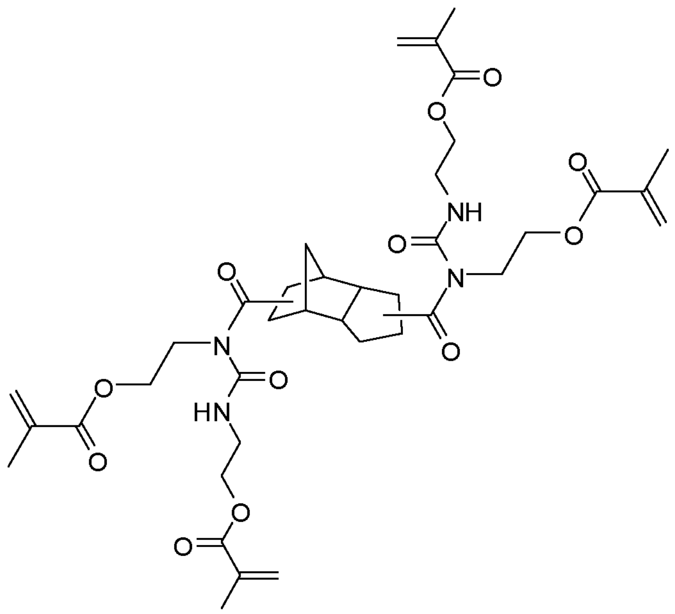

Formel (9)

Wird 3(4), 8(9)-Bis(Carbonsäure)tricyclo[5.2.1.$0^{2,6}$]decan mit Methacryloylisocyanat umgesetzt, ergibt sich das Imid der Formel (10). Die reaktiven Wasserstoffatome am Stickstoff können auch hier weiter in Isocyanatreaktionen umgesetzt werden.

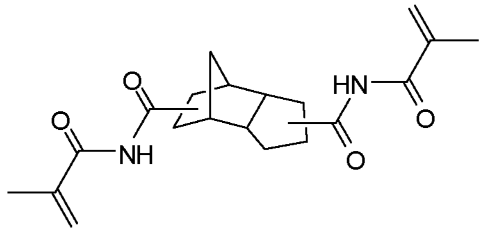

Formel (10)

3.) Ausgehend von 3(4), 8(9)-Bis(isocyanatomethyl)tricyclo[5.2.1.0$^{2,6}$]decan

Das 3(4), 8(9)-Bis(isocyanatomethyl)tricyclo[5.2.1.0$^{2,6}$]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Die Durchführung der zweiten Umsetzungsstufe der Isocyanat-Alkoholreaktion kann nicht nur ausgehend vom Tricyclodecandiol mit dem Isocyanatoethylmethacrylat, sondern auch ausgehend vom Tricyclodecandiisocyanat und Hydroxyethylmethacrylat eingeleitet werden. Durch stöchiometrische Umsetzung der beiden Reaktanden erhält man das Urethan der Formel (11).

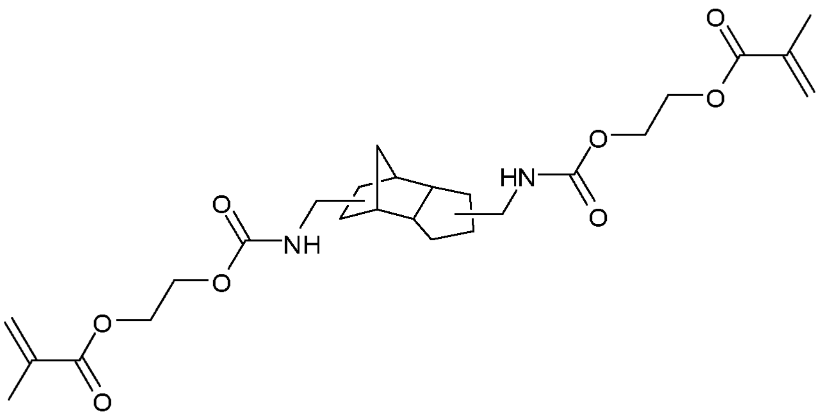

Formel (11)

Auch dieses Carbamat (Formel (11)) weist zwei reaktionsfähige Wasserstoffatome am Stickstoff auf, die mit einem Überschuss an Bis(isocyanatomethyl)tricyclo[5.2.1.0$^{2,6}$]decan zu dem Diisocyanat der Formel (12) weiter umgesetzt werden kann.

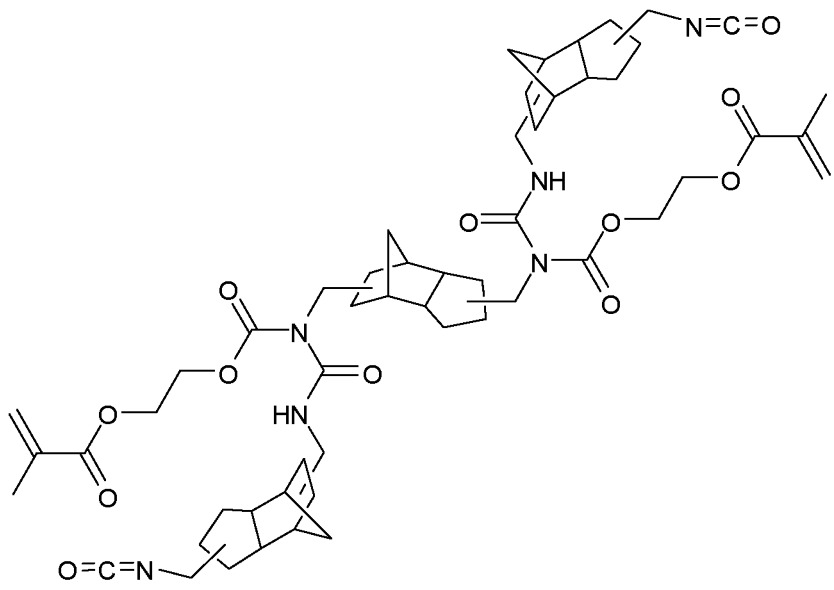

Formel (12)

Umsetzung des Allophanat-Diisocyanats (Formel (12)) mit Methacrylsäure liefert die Verbindung der Formel (13).

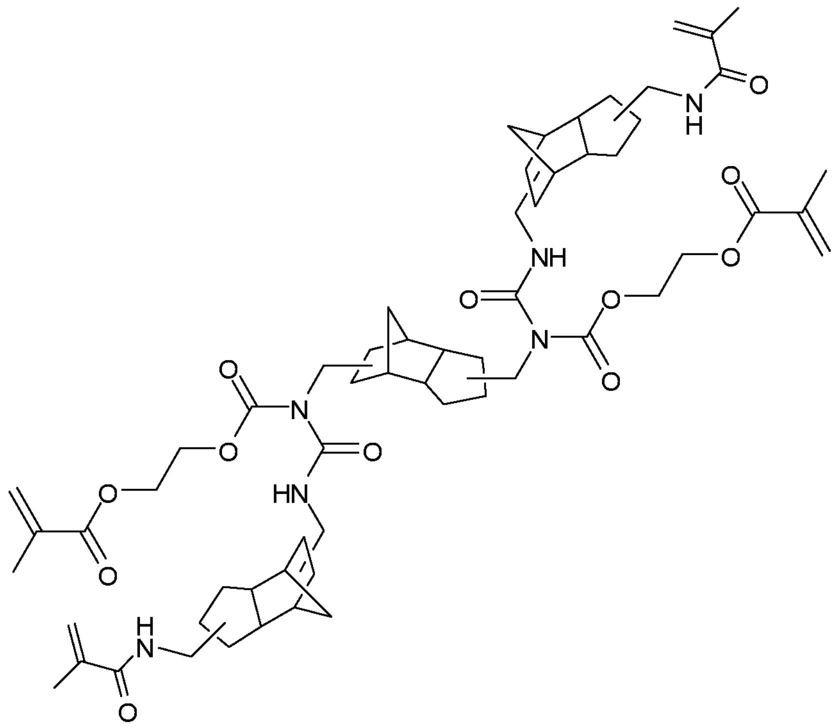

Formel (13)

Anstelle von Hydroxyethylmethacrylat können in den oben exemplarisch dargelegten Umsetzungen auch andere Hydroxylverbindungen von (Meth)acrylaten eingesetzt werden, wobei auch Gemische von Acrylaten und Methacrylaten verwendet werden können. So kann - analog dem obigen Beispiel - 3(4), 8(9)-Bis(isocyanatomethyl)-tricyclo[5.2.1.0$^{2,6}$]decan umgesetzt werden. Dabei bevorzugte Hydroxylverbindungen von (Meth)acrylaten sind

Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritol-tri(meth)acrylat, wie Dipentaerythrit, wie beispielsweise Dipentaerythritol-penta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Alle diese Verbindungen weisen sowohl (Meth)acrylatgruppen als auch Hydroxygruppen auf. Letztere können mit Isocyanatgruppen in der oben für die Reaktion zwischen Hydroxyethylmethacrylat und 3(4), 8(9)-Bis(isocyanatomethyl)-tricyclo[5.2.1.0$^{2,6}$]decan beschriebenen Weise reagieren. So lässt sich in einem einzigen Reaktionsschritt ein hoher Funktionalisierungsgrad erreichen.

3(4), 8(9)-Bis(isocyanatomethyl)tricyclo[5.2.1.0$^{2,6}$]decan kann mit 2-Carbonsäureethylmethacrylat zum entsprechenden Amid der Formel (16) umgesetzt werden.

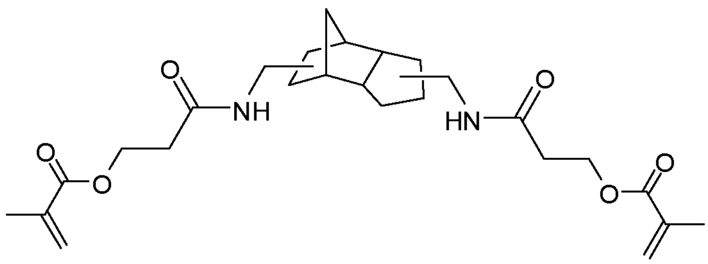

Formel (16)

Umsetzung des Amids der Formel (16) mit 2-Isocyanatoethylmethacrylat liefert den Acylharnstoff der Formel (17).

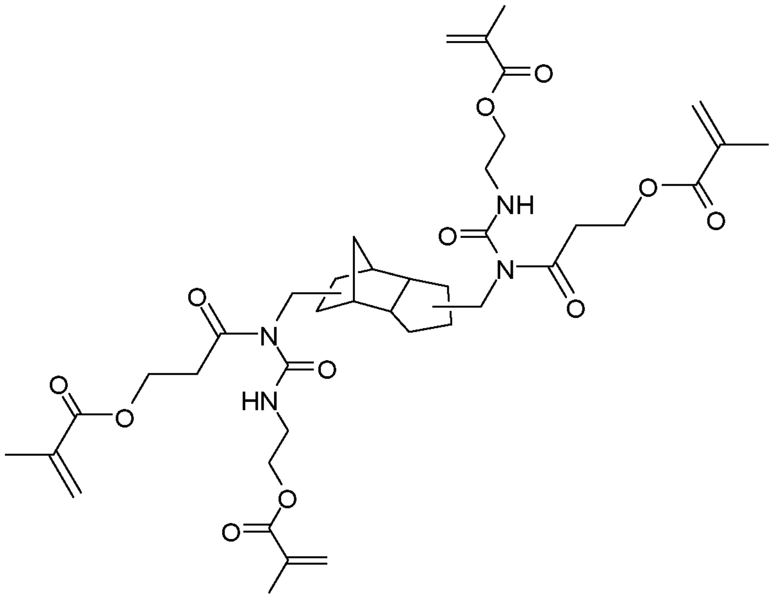

Formel (17)

Das Amid der Formel (16) kann auch mit einem Überschuss an 3(4), 8(9)-Bis(isocyanatomethyl)tricyclo[5.2.1.0$^{2,6}$]decan zum entsprechenden Isocyanat umgesetzt werden, wobei das gebildete Isocyanat mit Hydroxyethylmethacrylat weiter zum vernetzbaren Monomer der Formel (18) weiter umgesetzt wird.

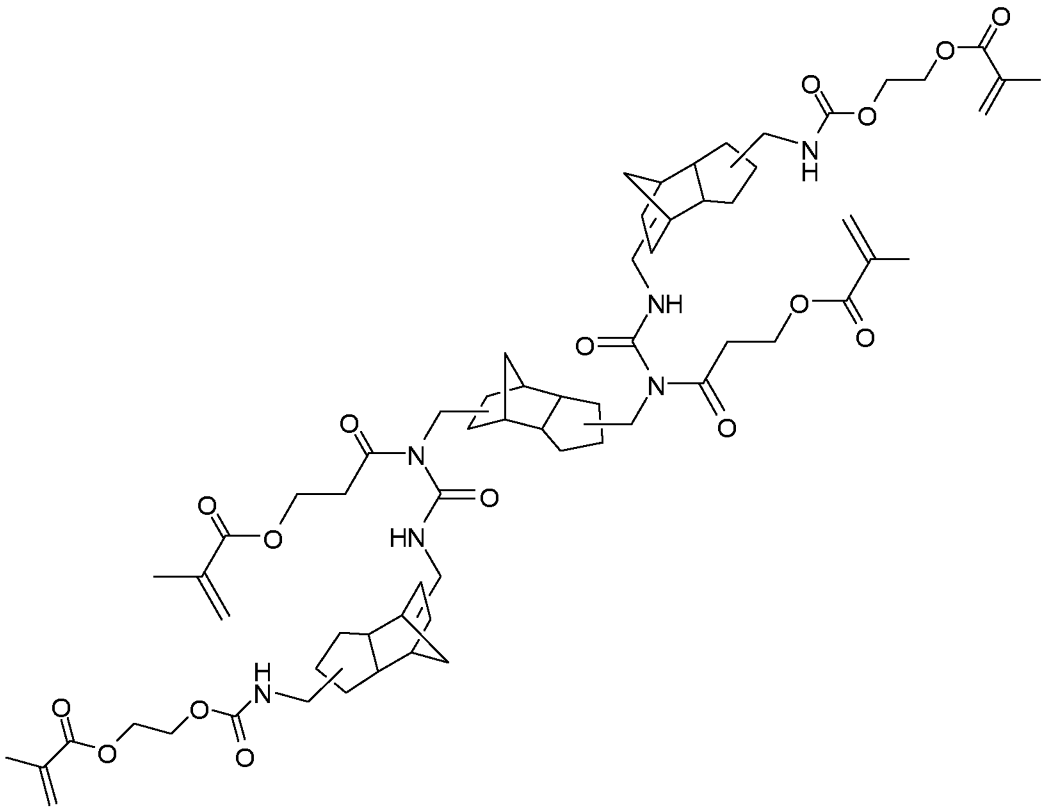

Formel (18)

Wird 3(4), 8(9)-Bis(isocyanatomethyl)tricyclo[5.2.1.0$^{2,6}$]decan mit 2-Methacryloyloxyethylhydrogensuccinat umgesetzt, so erhält man das Amid der Formel (19), das weiter mit 2-Isocyanatoethylmethacrylat zum Acylharnstoff der Formel (20) abreagiert wird.

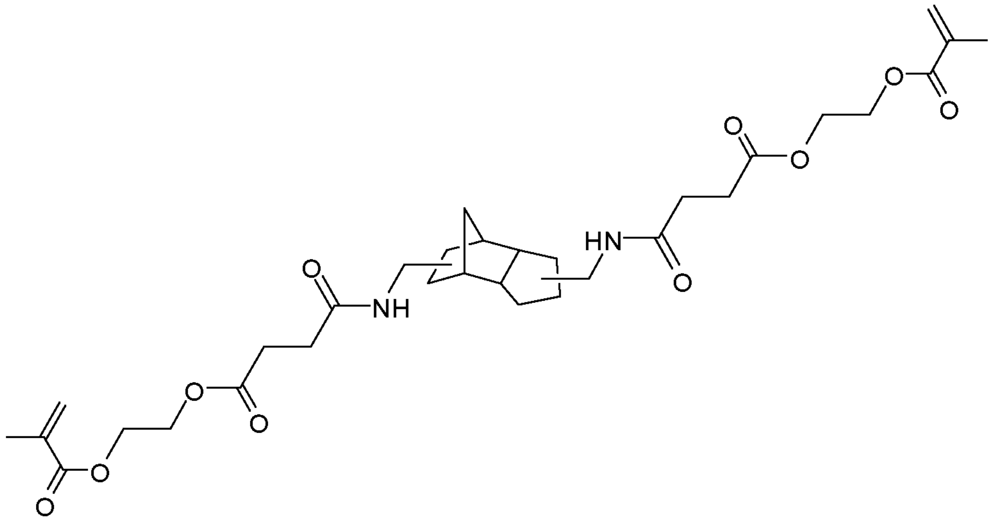

Formel (19)

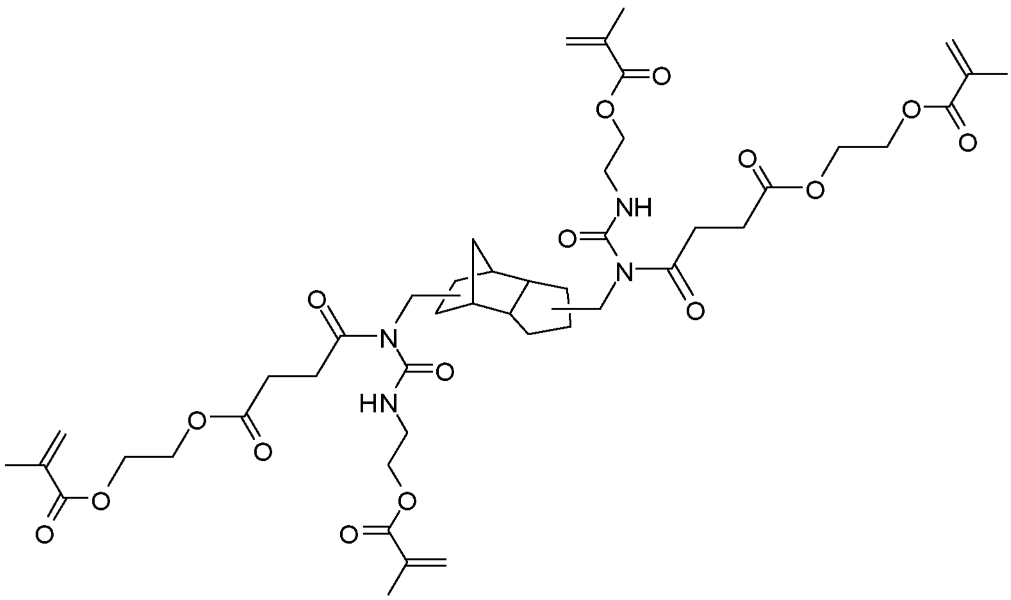

Formel (20)

Weitere geeignete Carbonsäuremethacrylate können durch Reaktionen von Di- oder Tetracarbonsäuremono- oder dianhydrid mit geeigneten OH-funktionalisierten, härtbaren Verbindungen wie beispielsweise 2-Hydroxyethylmethacrylat erhalten werden.

4.) Ausgehend von 3(4), 8(9)-Bis(Aminomethyl)tricyclo[5.2.1.0$^{2,6}$]decan

Das 3(4), 8(9)-Bis(Aminomethyl)tricyclo[5.2.1.0$^{2,6}$]decan ist an sich bekannt oder kann beispielsweise durch Umsetzung der entsprechenden Tosylate mit Ammoniak hergestellt werden. Reaktion des 3(4), 8(9)-Bis(Aminomethyl)tricyclo[5.2.1.0$^{2,6}$]decans mit 2-Isocyanatoethylmethacrylat ergibt die aus der EP 0 209 700 A2 bekannte Harnstoff-

verbindung der Formel (26).

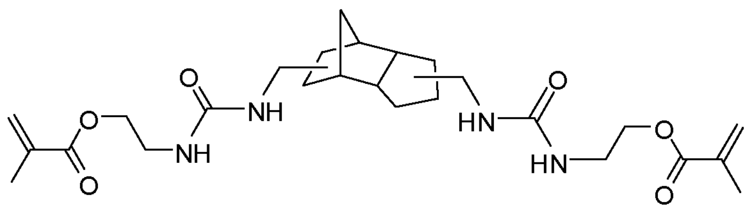

Formel (26)

Auch hier befinden sich noch aktive, reaktionsfähige Wasserstoffatome am Stickstoff, die mit einem Überschuss an Isocyanat beispielsweise zum Biuret der Formel (27) reagieren.

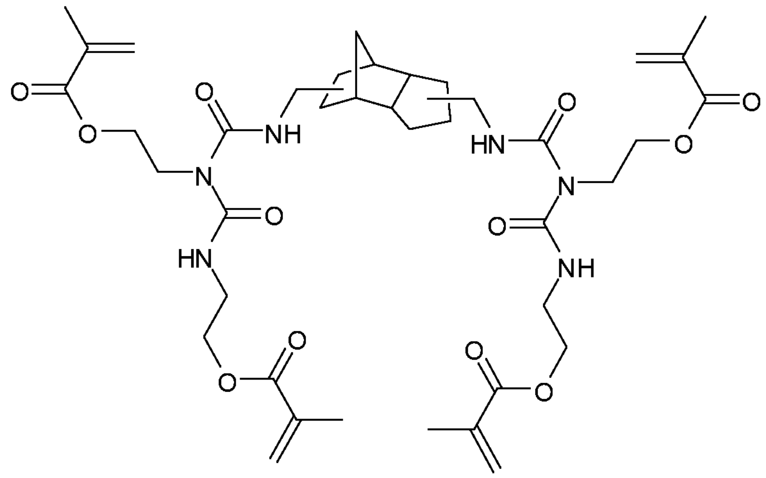

Formel (27)

Das 3(4), 8(9)-Bis(Aminomethyl)tricyclo[5.2.1.0$^{2,6}$]decan kann auch mit Methacryloylisocyanat zum entsprechenden Acylharnstoff umgesetzt werden. Die weitere Reaktion der verbleibenden reaktiven am Stickstoff befindlichen Wasserstoffatome mit Methacryloylisocyanat liefert das Biuret der Formel (28).

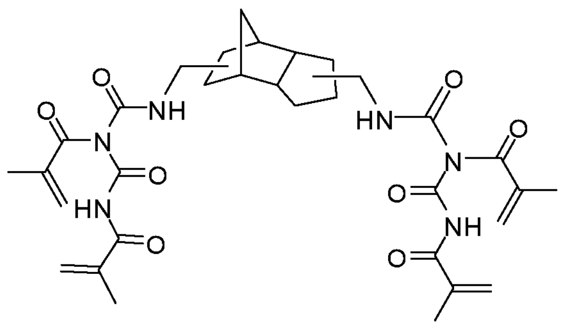

Formel (28)

Analog zu den vorstehend beschriebenen Monomeren, welche ein vom Tricyclo[5.2.1.0$^{2,6}$]decan abgeleitetes polyalicyclisches Strukturelement Q umfassen, können auch Monomere hergestellt werden, welche ein vom Tricyclo[3.3.1.1$^{3,7}$]decan (Adamantan) abgeleitetes polyalicyclisches Strukturelement Q umfassen. Als Beispiele seien fol-

gende Reaktionsprodukte gezeigt:

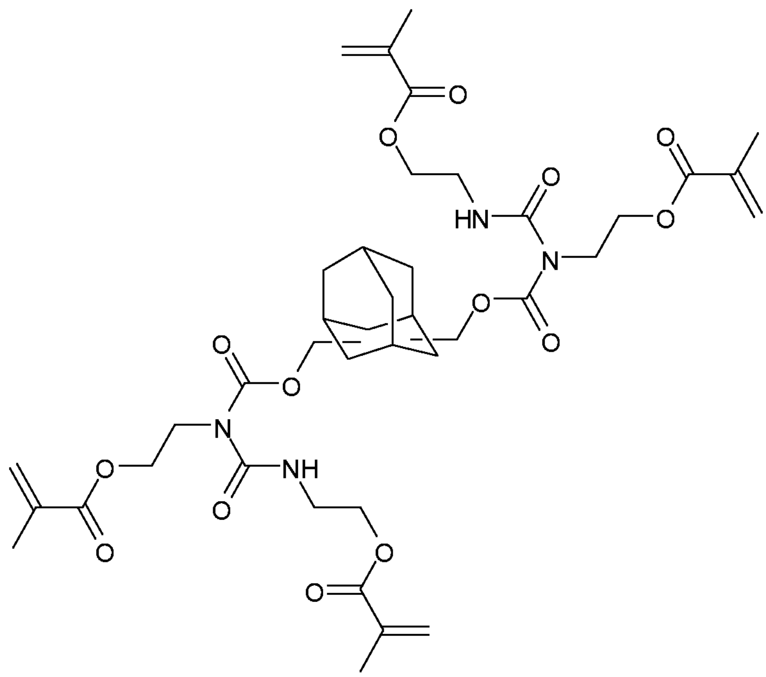

Formel (29)

Die Umsetzung der Verbindung der Formel (11) mit Diisocyanatoadamantan [(Bis(isocyanatomethyl)tricyclo[3.3.1.1$^{3,7}$]decan] liefert ein einzusetzendes Monomer, dessen Molekül zwei voneinander verschiedene polyalicyclische Strukturelemente Q umfasst, wie die nachfolgende Strukturformel der Verbindung der Formel (69) zeigt.

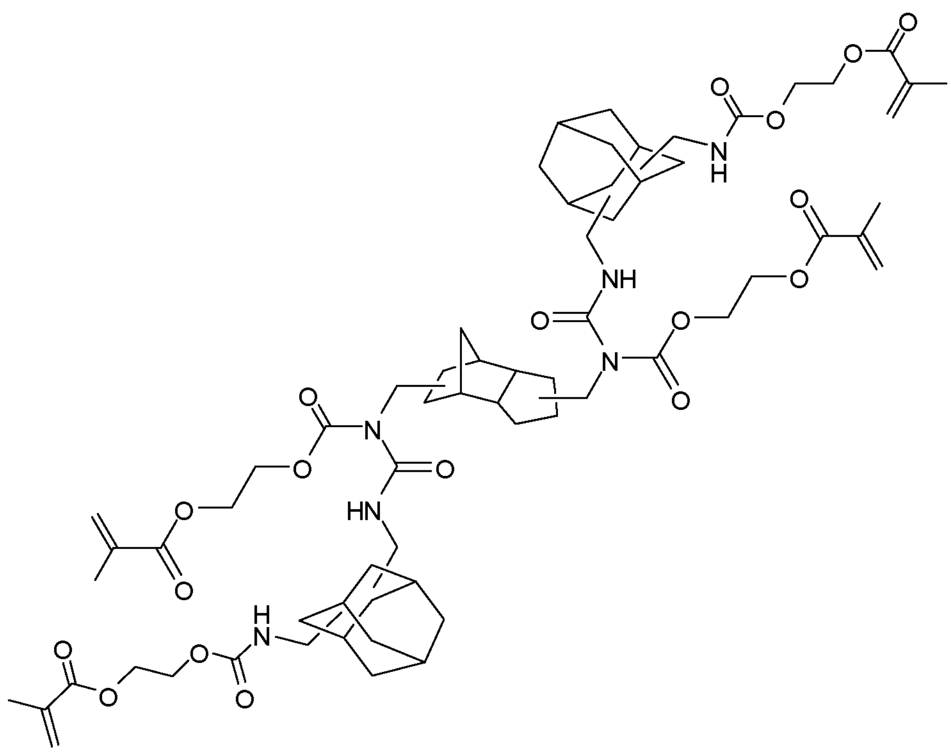

Formel (69)

Ein bevorzugtes erfindungsgemäß einzusetzendes organisches Bindemittelsystem umfasst als Komponente (b1) ein, zwei oder mehr Monomere ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $YZ_e$ substituierten Wasserstoffatome dieses polyalicyclischen Strukturelements Q durch Alkylgruppen, Alkoxygruppen, Halogenatome oder Trifluormethylgruppen substituiert ist bzw. sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,

  $-(C=O)-CH=CH_2$, $-(C=O)-C(CH_3)=CH_2$

  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jeder Index x bedeutet unabhängig von etwaigen weiteren Indizes x 0 oder 1,

- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist, wobei das, eines, zwei oder mehr der Monomere der Komponente (b1) ausgewählt ist bzw. sind aus der Gruppe bestehend aus Verbindungen (Monomere) der Struktur $Q(Y_xZ_e)_b$ mit x = 1 mit ein, zwei, drei, vier oder mehr funktionellen Gruppen, welche ausgewählt sind aus der Gruppe bestehend aus *N*-Acylharnstoff, Allophanat und Biuret, wobei gilt:

- jedes Y bedeutet ein Strukturelement, welches in der Struktur $Q(Y_xZ_e)_b$ mit x = 1 das polyalicyclische Strukturelement Q mit e Strukturelementen Z verknüpft und ein Strukturelement enthält oder aus diesem besteht, ausgewählt aus der Gruppe bestehend aus

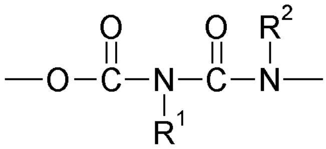
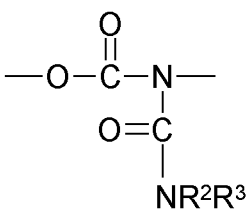
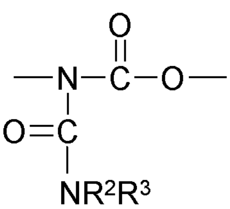

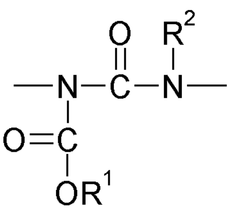
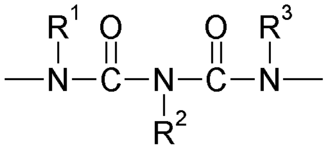
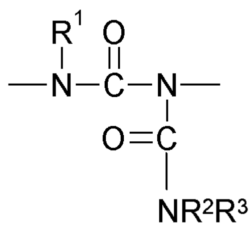

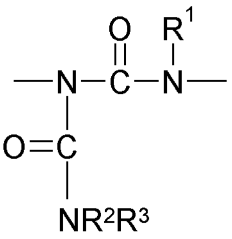

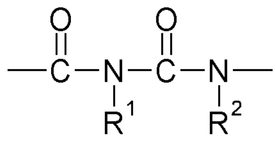

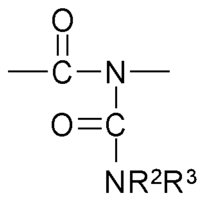

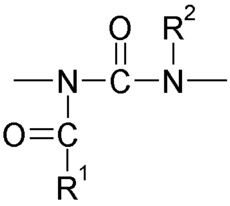

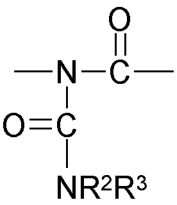

wobei $R^1$, $R^2$ und $R^3$ sonstige Reste der Verbindung bedeuten, wobei die jeweils im Formelbild links angeordnete Bindung dem Strukturelement Q und die rechts angeordnete Bindung dem Strukturelement Z näher ist.

Komponente (b2): ein, zwei oder mehr polymerisierbare Monomere, wobei das oder die polymerisierbaren Monomere keine Verbindungen (Monomere) der vorstehend definierten Struktur $Q(Y_xZ_e)_b$ sind

Die radikalisch polymerisierbaren Monomere der Komponente (b2) sind vorzugsweise radikalisch lichtpolymerisierbare Monomere, die bevorzugt eine, zwei oder mehr ethylenische Gruppen aufweisen. Vorzugsweise werden in einer erfindungsgemäßen Zusammensetzung die in der Dentalchemie üblichen (Meth)acrylatmonomere eingesetzt.

In der Patentliteratur sind eine Vielzahl von (Meth)acrylatmonomeren genannt (beispielsweise auch in der DE 39 41 629 A1), die sich zum Einsatz in einer erfindungsgemäßen Zusammensetzung eignen.

Ein bevorzugtes erfindungsgemäß einzusetzendes organisches Bindemittelsystem ist so ausgestaltet, dass das eine Monomer bzw. eines, mehrere oder sämtliche der mehreren polymerisierbaren Monomere der Monomerenkomponente (b2) ausgewählt ist bzw. sind aus der Gruppe bestehend aus Acrylaten und Methacrylaten, vorzugsweise aus der Gruppe der Methacrylate.

Ein bevorzugtes erfindungsgemäß einzusetzendes organisches Bindemittelsystem umfasst ein oder mehrere Dimethacrylat-Monomere gewählt aus der Gruppe bestehend aus Ethylenglykoldimethacrylat, 1,6-Hexandioldimethacrylat (HEDMA), Triethylenglykoldimethacrylat (TEDMA), 1,12-Dodecandioldimethacrylat, ethoxyliertes Bisphenol-A-dimethacrylat, Polyethylenglykoldimethacrylat, 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat (GlyDMA), Bisphenol-A-Glycidyl-Methacrylat (Bis-GMA) und Dimethacrylaten des Dihydroxymethyltricyclo[5.2.1.$0^{2,6}$]decans.

Die radikalisch lichtpolymerisierbaren Monomere können auch mindestens eine ethylenische Doppelbindung aufweisende Hydroxylverbindungen sein. Dabei können vorzugsweise die in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Zudem können auch lichthärtbare Monomere mit ethylenischen Doppelbindungen auf Basis von Polysiloxanen, wie sie beispielsweise in der DE 199 03 177 oder in der DE 44 16 857 beschrieben sind, verwendet werden.

Die radikalisch polymerisierbaren Monomere der Komponente (b2) können auch als sogenannte Haftmonomere ausgestaltet sein. Als eine Haftung vermittelnde Verbindung aus der Gruppe der Mono- oder Diphosphatester der Hydroxyalkylmethacrylate erwies sich 10-(Meth)acryloyloxydecyldihydrogenphosphat (10-MDP) (EP 0 074 708, EP 1 084 131). Die Phosphorsäurefunktion bildet mit dem Hydroxylapatit stabile, wasserunlösliche Salze, wobei das Calcium mit Hilfe der Phosphorsäuregruppe komplexiert wird. Der Methylenspacer scheint eine genau abgestimmte Länge aufzuweisen, um gegenseitige Störungen durch sterische Effekte bei der Bindungsbildung an beiden Enden des Haftvermittlers zu vermeiden. Dies wiederum scheint eine Voraussetzung zu sein, um die Substratoberfläche optimal und gleichmäßig benetzen zu können.

Präparativ kann 10-MDP durch Reaktion von 10-Hydroxydecyl(meth)acrylat mit Phosphoroxychlorid erhalten

werden.

Weitere Verbindungen dieses Typs sind beispielsweise 2-(Meth)acryloyloxyethyldihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogenphosphat, 2-(Meth)acryloyloxynonyldihydrogenphosphat, 11-(Meth)acryloyloxyundecyldihydrogenphosphat, 20-(Meth)acryloyloxyeicosyldihydrogenphosphat, 1,3-Di(meth)acyloyloxypropyl-2-dihydrogenphosphat oder 2-(Meth)acryloyloxyethylphenyldihydrogenphosphat.

Anstelle eines Phosphorsäurerestes können die polymerisierbaren Monomeren auch einen Diphosphorsäurerest aufweisen, wie beispielsweise das Di(2-(meth)acryloyloxyethyl)pyrophosphat, das Di(2-(meth)acryloyloxypropyl)pyrophosphat, das Di(2-(meth)acryloyloxybutyl)pyrophosphat, das Di(2-(meth)acryloyloxypentyl)pyrophosphat, das Di(2-(meth)acryloyloxyhexyl)pyrophosphat, das Di(2-(meth)acryloyloxydecyl)pyrophosphat, etc.. Es können auch die entsprechenden Säurehalogenide eingesetzt werden.

Neben den polymerisierbaren Monomeren mit einem Phosphorsäure- oder Pyrophosphorsäurerest können entsprechende Verbindungen eingesetzt werden, die einen Phosphonsäure-, einen Thiophosphorsäure- oder einen Sulfonsäurerest aufweisen.

Analog können Haftung vermittelnde Monomere beispielsweise aus Hydroxyalkylmethacrylat oder Glyceryldimethacrylat synthetisiert werden. So bilden sich beispielsweise bei den Umsetzungen von Hydroxyethylmethacrylat mit Phosphoroxychlorid Gemische, die Mono-, Di- und Triester aufweisen.

Ein weiterer Typ eines Haftmonomers ist der Phosphorsäureester von Pentaerythritoltriacrylat oder von Dipentaerythritolpentaacrylat (PENTA, US 4,514,342). Der Ester wird aus Dipentaerythritolmonohydroxypentaacrylat und Phosphoroxychlorid in Gegenwart von Triethylamin hergestellt.

Andere Haftung vermittelnde Verbindungen (Haftmonomere), die zum Einsatz in Komponente (b2) geeignet sind, sind Methacryloyloxyalkylderivate aromatischer Carbonsäuren. Es zeigte sich, dass insbesondere Trimellitsäure-4-methacryloyloxyethylester (4-MET) oder Trimellitsäureanhydrid-4-methacryloyloxyethylester (4-META) als haftungsfördernde Monomere eingesetzt werden können (DE 28 28 381, US 4,148,988, EP 0 684 033, EP 0 684 034). 4-META kann durch Dehydrochlorierungsreaktion zwischen Hydroxyethylmethacrylat und wasserfreiem Trimellitsäurechlorid oder durch Dehydrationsreaktion zwischen 2-Hydroxyethylmethacrylat und Trimellitsäureanhydrid hergestellt werden.

Ananlog sollen Pyromellitsäuredimethacrylat sowie Pyromellitsäureglyceroldimethacrylat ebenfalls als Haftmonomer geeignet sein.

Andere Methacryloyloxyethylderivate aromatischer Carbonsäuren, die als Haftmonomere geeignet sein sollen, sind entsprechende Verbindungen der Phthalsäure.

Des Weiteren sollen Methacryloyloxyethylderivate der Bernsteinsäure und der Maleinsäure als Haftmonomer einsetzbar sein.

Weitere reaktive Haftkomponenten, die zum Einsatz in Komponente (b2) geeignet sind, werden in EP 1 148 060, EP 0 909 761 und EP 1 148 071 offenbart, wo polymerisierbare und hydrolysestabile Acrylphosphonsäuren beschrieben sind. Der aufwendige Syntheseweg beginnt mit der Umsetzung von Formaldehyd und einem geeigneten Acrylsäureester in Gegenwart eines Katalysators unter Bildung einer Methylolgruppe in $\alpha$-Position des Esters und anschließender Halogenierung des Alkohols mit einem anorganischen Säurehalogenid. Der so hergestellte $\alpha$-Halogenmethacrylsäureester wird dann mit geeigneten und zuvor geschützten mono- oder difunktionellen Phosphonsäureestern umgesetzt. Nach Abspaltung der Schutzgruppe wird dann die polymerisierbare und hydrolysestabile Acrylphosphonsäure erhalten, deren Merkmal die endständige Oxo-ethylacrylatfunktion darstellt.

In der EP 1 346 717 wird Tetramethacryloxyethylpyrophosphat als Haftung vermittelnde Substanz beschrieben; auch diese Verbindung ist zum Einsatz in Komponente (b2) geeignet. Das Pyrophosphat soll unter den wässrigen Bedingungen aufbrechen und zu Phosphorsäureresten hydrolysieren, die anfänglich für einen sehr niedrigen pH-Wert sorgen sollen und helfen sollen, das Hydroxylapatit anzuätzen. Die Phosphorsäurereste sollen dann durch Calciumionen, die aus dem Dentin herauswandern sollen, neutralisiert werden und so einen zementartigen Haftverbund am Zahn bilden, während die Methacrylatgruppen durch Lichtpolymerisation mit dem Zahnfüllungsmaterial abreagieren können.

In der EP 1 721 949 A1 werden polymerisierbare Derivate der Ethylendiamintetraessigsäure als Haftvermittler in dentalen Adhäsivmaterialien vorgeschlagen; diese sind zum Einsatz in Komponente (b2) geeignet. Der haftvermittelnde Effekt wurde in der EP 1 721 949 A1 am Beispiel des Di-Oxyethoxymethacrylsäureethylendiamintetraessigsäureesters gezeigt.

Vorzugsweise ist das eine Monomer bzw. eines, mehrere oder sämtliche der mehreren polymerisierbaren Monomere der Komponente (b2) ausgewählt aus der Gruppe bestehend aus Acrylaten und Methacrylaten.

In eigenen Untersuchungen haben sich Zusammensetzung besonderes bewährt, in denen das organische Bindemittelsystem so ausgestaltet ist, dass das eine Monomer bzw. eines, mehrere oder sämtliche der mehreren polymerisierbaren Monomere ausgewählt ist bzw. sind aus der Gruppe bestehend aus Triethylenglykoldimethacrylat (TEDMA), Urethandimethacrylat (7,7,9-Trimethyl-4,13-dioxo-3,14-dioxa-5,12-diazahexadecan-1,16-dioxydimethacrylat (UDMA)), Glycerindimethacrylat (GlyDMA) und Bisphenol-A-Glycidyl-Methacrylat (Bis-GMA).

Zum organischen Bindemittelsystem (b), vorzugsweise als Mischung der Komponenten (b1) und (b2):

Vorzugsweise ist die Monomerenkomponente (b) eine Monomerenmischung, die sowohl Komponente (b1) als auch Komponente (b2) umfasst oder aus dieser besteht.

Besonders gute Ergebnisse im Sinne der vorliegenden Erfindung werden erzielt, wenn das Gewichtsverhältnis der Gesamtmenge an Monomerkomponente (b1) zu der Gesamtmenge an Monomerkomponente (b2) im Bereich 4 : 1 bis 1 : 3 liegt, vorzugsweise im Bereich 3 : 1 bis 1 : 2, bevorzugt im Bereich 2 : 1 bis 2 : 3, besonders bevorzugt im Bereich 3 : 2 bis 2 : 3 und ganz besonders bevorzugt im Bereich 4 : 3 bis 3 : 4.

Sofern eine erfindungsgemäße Zusammensetzung sowohl Komponente (b1) als auch Komponente (b2) umfasst, hat es sich als vorteilhaft herausgestellt, dass Bisphenol-A-Glycidyl-Methacrylat (Bis-GMA) bzw. sämtlichen Verbindungen mit einem Bisphenol-A-Strukturelement nicht Bestandteil der Zusammensetzung sind. Bisphenol-A-Glycidyl-Methacrylat (Bis-GMA) kann zwar eingesetzt werden, vorzugsweise enthält eine erfindungsgemäße Zusammensetzung, jedoch nicht die Verbindung Bis-GMA. Vorzugsweise ist ein erfindungsgemäß einzusetzende Zusammensetzung, frei von sämtlichen Verbindungen mit einem Bisphenol-A-Strukturelement.

Sofern eine erfindungsgemäße Zusammensetzung sowohl Komponente (b1) als auch Komponente (b2) umfasst, werden sehr gute Ergebnisse erzielt, wenn als Monomerkomponente (b2) eine Mischung von TEDMA und UDMA eingesetzt wird, wobei das Gewichtsverhältnis von TEDMA zu UDMA im Bereich 4 : 1 bis 1 : 4 liegt, vorzugsweise im Bereich 3 : 1 bis 1 : 3, bevorzugt im Bereich 2 : 1 bis 1 : 2, besonders bevorzugt im Bereich 3 : 2 bis 2 : 3 und ganz besonders bevorzugt im Bereich 4 : 3 bis 3 : 4.

Besonders gute Ergebnisse werden erzielt, wenn das Gewichtsverhältnis der Gesamtmenge an (Meth)acrylsäureestern des Bis(hydroxymethyl)tricyclo[5.2.1.0$^{2,6}$]decans (Monomerkomponente (b1)) zu der Gesamtmenge der Mischung von TEDMA und UDMA (Monomerkomponente (b2)) im Bereich 4 : 1 bis 1 : 3 liegt, vorzugsweise im Bereich 3 : 1 bis 1 : 2, bevorzugt im Bereich 2 : 1 bis 2 : 3, besonders bevorzugt im Bereich 3 : 2 bis 2 : 3 und ganz besonders bevorzugt im Bereich 4 : 3 bis 3 : 4, wobei in der Monomerkomponente (b2) wiederum vorzugsweise die oben genannten Gewichtsverhältnisse von TEDMA zu UDMA eingestellt sind.

Zum Füllstoffsystem (a):

In einer erfindungsgemäßen Zusammensetzung ist das Füllstoffsystem so ausgestaltet, dass es aus mittels organischer Strukturelemente oberflächenmodifizierten anorganischen Füllstoffpartikeln besteht oder solche Füllstoffpartikel umfasst, wobei die organischen Strukturelemente mit den Monomeren unter Bildung kovalenter Bindungen radikalisch oder nicht-radikalisch umsetzbar sind, vorzugsweise mit einem oder mehreren Monomeren der Komponenten (b1) und/oder (b2) umsetzbar sind, wobei die Füllstoffpartikel des Füllstoffsystems eine mittlere Teilchengröße im Bereich zwischen 1 nm und 150 nm besitzen. Vorzugsweise sind die besagten mittels organischer Strukturelemente oberflächenmodifizierten anorganischen Füllstoffpartikel ausgewählt aus der Gruppe der mittels organischer Strukturelemente oberflächenmodifizierten hergestellten Partikel von Siliziumoxid, Zirkoniumoxid, Aluminiumoxid und deren Mischoxiden. Besonders bevorzugte Partikel der genannten Art sind mittels Nassverfahren (nasschemisch) hergestellte (und anschließend oberflächenmodifizierte) Partikel. Zu den Nassverfahren (nasschemischen Verfahren) gehören insbesondere Sol-Gel-Verfahren sowie die Verfahrensgestaltungen, bei denen von einem Wasserglas ausgegangen wird, welches mittels eines Ionenaustauschers entionisiert wird. Wir verweisen insoweit auf die Ausführungen weiter oben.

Die besagten nicht flammhydrolytisch hergestellten Partikel von Siliziumoxid, Zirkoniumoxid, Aluminiumoxid und deren Mischoxiden können die üblichen Verunreinigungen enthalten. Besonders relevante Mischoxide sind die Mischoxide von Siliziumoxid, und Zirkoniumoxid.

Vorzugsweise umfasst eine erfindungsgemäße Zusammensetzung neben den bevorzugten, mittels organischer Strukturelemente oberflächenmodifizierten, nicht flammhydrolytisch hergestellten (vorzugsweise nasschemisch hergestellten) anorganischen Füllstoffpartikeln (wie oben definiert) keine weiteren anorganischen, oberflächenmodifizierten oder nicht oberflächenmodifizierten Füllstoffpartikel. Vorzugsweise umfasst die Zusammensetzung überhaupt keine weiteren Füllstoffpartikel.

Besonders bevorzugt sind erfindungsgemäße Zusammensetzungen, die mehr als 40 Gew.-%, bevorzugt mehr als 55 Gew.-%, besonders bevorzugt mehr als 60 Gew.-% an Füllstoffpartikeln umfassen, bezogen auf die Gesamtmasse der Zusammensetzung. Dabei gilt weiterhin, dass die erfindungsgemäß anzuwendenden Zusammensetzungen vorzugsweise mittels organischer Strukturelemente oberflächenmodifizierte, nicht flammhydrolytisch hergestellte, anorganische Füllstoffpartikel in einem Anteil von 50 bis 100 Gew.-% umfassen, bezogen auf die eingesetzte Gesamtmasse an Füllstoffpartikeln in der erfindungsgemäßen Zusammensetzung. Zudem gilt auch für eine solche erfindungsgemäße Zusammensetzung mit einem Anteil an Füllstoffpartikeln in den vorstehend genannten Bereichen, dass sie vorzugsweise gleichzeitig zu einem Versiegelungsmaterial aushärtbar ist, das den weiter oben als bevorzugt bezeichneten Transmissionsgrad besitzt und weiter bevorzugt auch (i) die genannte Biegefestigkeit und/oder (ii) die genannte Abrasion besitzt. Dabei ist in der insbesondere bevorzugten Zusammensetzung in dem organischen Bindemittelsystem ein Füllstoffsystem

dispergiert, bestehend aus oder umfassend nicht-aggregierte, nicht-agglomerierte, mittels organischer Strukturelemente oberflächenmodifizierte, nasschemisch hergestellte Füllstoffpartikel, die ausgewählt sind aus der Gruppe der mittels organischer Strukturelemente oberflächenmodifizierten nasschemisch hergestellten Partikel von Siliziumoxid, Zirkoniumoxid, Aluminiumoxid und deren Mischoxiden. Die insbesondere bevorzugte Zusammensetzung umfasst neben den besagten Partikeln keine weiteren Füllstoffpartikel; sie umfasst vorzugsweise mehr als 40 Gew.-%, bevorzugt mehr als 55 Gew.-%, besonders bevorzugt mehr als 60 Gew.-% der besagten Partikel, bezogen auf die Gesamtmasse der Zusammensetzung. Die mittlere Teilchengröße der Füllstoffpartikel der Zusammensetzung beträgt zwischen 1 nm und 150 nm, bevorzugt zwischen 20 nm und 100 nm, besonders bevorzugt zwischen 30 und 70 nm. Die mittlere Teilchengröße wird dabei bestimmt für den Gesamtanteil an Füllstoffpartikeln in der Zusammensetzung. Sofern verschiedene Typen von Füllstoffpartikeln in der Zusammensetzung vorliegen, neben oberflächenmodifizierten, anorganischen beispielsweise organische oder nicht oberflächenmodifizierte Füllstoffpartikel, oder neben Partikeln auf Basis von Siliziumoxid auch solche auf Basis von Zirkoniumoxid, werden also sämtliche dieser Füllstoffpartikel bei der Bestimmung der mittleren Teilchengröße berücksichtigt. Zur Methode der Bestimmung der mittleren Teilchengröße siehe unten.

Wie oben bereits ausgeführt, ist auch die insbesondere bevorzugte erfindungsgemäße Zusammensetzung transparent für Licht, welches zur Anregung einer Fluoreszenz sowohl von gesunder als auch kariöser Zahnhartsubstanz geeignet ist, und die insbesondere bevorzugte Zusammensetzung ist zudem transparent für die entsprechende von der Zahnhartsubstanz emittierte Fluoreszenzstrahlung.

Additive in der Zusammensetzung:

Vorzugsweise umfasst eine erfindungsgemäße Zusammensetzung im organischen Bindemittelsystem ein oder mehrere Additive, welche (i) eine Lichthärtung und/oder (ii) chemische Härtung des einen oder der mehreren polymerisierbaren Monomere ermöglichen oder unterstützen.

Beispiele für Additive, welche eine Lichthärtung des einen oder der mehreren polymerisierbaren Monomere ermöglichen oder unterstützen sind Katalysatoren, die nur photosensibilisierend wirken (Photosensibilisatoren, Photoinitiatoren), sowie Beschleuniger (Akzeleratoren, Co-Initiatoren), die vorzugsweise in Kombination mit Photosensibilisatoren eingesetzt werden.

Beispiele für geeignete Photosensibilisatoren sind alpha-Diketone (z.B. Campherchinon), Benzoinalkylether, Thioxanthone, Benzophenone, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2. Beispiele für geeignete Beschleuniger, die zusammen mit den Photosensibilisatoren eingesetzt werden, sind tertiäre Amine (z.B. Ethyl-*p*-*N*,*N*-dimethylaminobenzoat (DABE)), sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Additive (Initiatoren sowie Initiatorkombinationen) sind in der DE 601 16 142 beschrieben.

Die im Rahmen der vorliegenden Erfindung verwendbaren Photoinitiatoren sind vorzugsweise dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Co-initiatoren, die Aushärtung einer erfindungsgemäßen Zusammensetzung bewirken können.

Das Absorptionsmaximum von Campherchinon (CC) liegt bei ca. 470 nm und somit im Bereich des blauen Lichts. Campherchinon (CC) zählt zu den $PI_2$-Initiatoren und wird regelmäßig zusammen mit einem Coinitiator eingesetzt.

Vorzugsweise enthält eine erfindungsgemäße Zusammensetzung die Kombination eines alpha-Diketons und eines aromatischen tertiären Amins, bevorzugt ist die Kombination von Campherchinon (CC) und Ethyl-*p-N,N*-dimethylaminobenzoat (DABE).

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere im Rahmen der vorliegenden Erfindung geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt, die auch im Rahmen der vorliegenden Erfindung eingesetzt werden können. Es sei insoweit exemplarisch auf die EP 1 720 506 verwiesen.

Bevorzugte Initiatoren zur chemischen Härtung einer erfindungsgemäßen Zusammensetzung sind Benzoylperoxid, Lauroylperoxid und Dibenzoylperoxid. Die besagten chemischen Initiatoren, insbesondere Dibenzoylperoxid, werden vorzugsweise in Kombination mit Aminen eingesetzt, wie z.B. N,N-Dimethyl-p-toluidin, N,N-Dihydroxyethyl-p-toluidin sowie strukturverwandte Amine.

Die Peroxide und die Amine werden dabei üblicherweise auf zwei unterschiedliche Komponenten eines Dentalmaterials aufgeteilt, wobei vorzugsweise beide Komponenten sowie die nach Mischung der Komponenten vorliegende Mischung erfindungsgemäße Zusammensetzungen sind. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme sind sowohl chemisch härtbare als auch lichthärtbare Systeme, die üblicherweise zwei Komponenten umfassen, wobei vorzugsweise beide Komponenten sowie die nach Mischung der Komponenten vorliegende Mischung erfindungsgemäße Zusammensetzungen sind. Dualhärtende Systeme umfassen eine Kombination aus Photoinitiatoren und Initiatoren zur chemischen Aushärtung.

Beispielsweise kann die Basispaste eines dualhärtenden Systems eine erfindungsgemäße Zusammensetzung sein und neben einer aminhaltigen Komponente zusätzlich einen Photoinitiator enthalten, so dass die Basispaste entweder allein als lichthärtender oder zusammen mit der Initiatorpaste als licht- und chemisch härtender Dentalwerkstoff eingesetzt werden kann.

Alternativ oder zusätzlich zu den oxidativ wirksamen organischen Peroxidverbindungen können im Rahmen der vorliegenden Erfindung als Redoxsysteme in chemisch härtenden Systemen auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonylsulfamide sind in der EP 0 059 451. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

In der Regel weisen Initiatoren, die eine Lichthärtung und/oder chemische Härtung des einen oder der mehreren polymerisierbaren Monomere ermöglichen, nach der Härtung keine weiteren positiven Eigenschaften auf. Vielmehr besteht die Möglichkeit, dass nach der Härtung verbleibende Initiatoren oder deren Folgeprodukte nach abgeschlossener Lichthärtung und/oder chemischer Härtung einen negativen Einfluss auf die mechanischen Eigenschaften der zu einem Versiegelungsmaterial ausgehärteten Zusammensetzung aufweisen. Es ist daher vorteilhaft, eine solche Menge an Initiatoren einzusetzen, dass die Härtung unter Praxisbedingungen vollständig verläuft und gleichzeitig die mechanischen Eigenschaften (z.B. Biegefestigkeit und Abrasionsresistenz) nach der Härtung nicht negativ beeinflusst werden.

Besonders bevorzugt sind erfindungsgemäße Zusammensetzungen, die insgesamt weniger als 1,00 Gew.-% , vorzugsweise weniger als 0,50 Gew.-% , besonders bevorzugt weniger als 0,25 Gew.-% bezogen auf die Gesamtmasse der Zusammensetzung, an Initiatoren umfassen, welche (insbesondere in Kombination mit Beschleunigern) (i) eine Lichthärtung und/oder (ii) chemische Härtung des einen oder der mehreren polymerisierbaren Monomere ermöglichen.

In eigenen Untersuchungen hat sich gezeigt, dass Initiatoren, die eine Phosphinoxid-Gruppe besitzen, wie z.B. Phenyl-bis(2,4,6-trimethylbenzoyl)phosphinoxid oder 2,4,6-Trimethylbenzoyldiphenylphosphinoxid, einen besonders negativen Einfluss auf die mechanischen Eigenschaften der ausgehärteten Zusammensetzung aufweisen.

Besonders bevorzugt sind daher erfindungsgemäße Zusammensetzungen, welche keine Initiatoren oder sonstige Verbindungen enthalten, die Phosphinoxid-Gruppen besitzen.

Die erfindungsgemäßen Zusammensetzungen enthalten im organischen Bindemittelsystem vorzugsweise einen oder mehrere Inhibitoren, auch Stabilisatoren genannt. Diese sind einer härtbaren erfindungsgemäßen Zusammensetzung zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität des härtbaren, insbesondere lichthärtbaren Zusammensetzungen. Gängige Inhibitoren, die auch in erfindungsgemäßen Zusammensetzungen bevorzugt eingesetzt werden, sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben. Alternative Inhibitoren sind in der DE 101 19 831 A1 oder in der EP 1 563 821 A1 angegeben. Der Fachmann ist in der Lage, den Transmissionsgrad einer erfindungsgemäßen Zusammensetzung durch Einstellung ihrer Rezeptur zu variieren. Wesentliche Rezepturparameter sind hierbei der Gewichtsanteil des Füllstoffsystems, die Größe der eingesetzten Füllstoffpartikel und die An- oder Abwesenheit von Additiven, welche die Zusammensetzung färben.

Herstellung von erfindungsgemäßen Zusammensetzungen:

Eine erfindungsgemäße Zusammensetzung umfasst ein Füllstoffsystem, welches in ein organisches Bindemittelsystem dispergiert ist. Vorzugsweise ist eine erfindungsgemäße

Zusammensetzung der vorgenannten Art herstellbar durch ein Verfahren mit den folgenden Schritten:

(a) Bereitstellen oder Herstellen einer kolloidalen Lösung (kolloidalen Suspension) anorganischer Partikel in Wasser,

(b) Austauschen des Wassers gegen ein mit Wasser vollständig mischbares organisches Lösungsmittel (Suspensionsmittel),

(c) vor oder nach Schritt (b) Modifizieren der Oberfläche der anorganischen Partikel, so dass Partikel resultieren, die mittels organischer Strukturelemente oberflächenmodifiziert sind, wobei die organischen Strukturelemente mit den Monomeren unter Bildung kovalenter Bindungen umsetzbar sind,

(d) teilweises oder vollständiges Austauschen des mit Wasser vollständig mischbaren organischen Lösungsmittels gegen das organische Bindemittelsystem umfassend ein oder mehrere polymerisierbare Monomere, so dass die erfindungsgemäß anzuwendende Zusammensetzung resultiert.

Zu bevorzugten Verfahren zum Modifizieren der Oberfläche der anorganischen Partikel verweisen wir auf die Ausführungen weiter oben.

Die vorliegende Erfindung betrifft auch ein Verfahren zum Herstellen einer erfindungsgemäß anzuwendenden Zusammensetzung, mit den folgenden Schritten:

(a) nasschemisches Herstellen einer kolloidalen Lösung (kolloidalen Suspension) anorganischer Partikel nach dem Sol-Gel-Verfahren (siehe oben) oder ausgehend von Wasserglas (siehe oben) in Wasser oder einem anderen Lösungsmittel (Suspensionsmittel),

(b) Überführen der gemäß Schritt (a) hergestellten kolloidalen Lösung in eine bzw. Bereitstellen der gemäß Schritt (a) hergestellten kolloidalen Lösung als kolloidale Lösung (kolloidale Suspension) anorganischer Partikel in einem mit Wasser vollständig mischbaren organischen Lösungsmittel (Suspensionsmittel), beispielweise durch Austauschen des in Schritt (a) eingesetzten Wassers gegen ein mit Wasser vollständig mischbares organisches Lösungsmittel (Suspensionsmittel),

(c) vor oder nach Schritt (b) Modifizieren der Oberfläche der anorganischen Partikel, so dass Partikel resultieren, die mittels organischer Strukturelemente oberflächenmodifiziert sind, wobei die organischen Strukturelemente mit den Monomeren unter Bildung kovalenter Bindungen umsetzbar sind,

(d) teilweises oder vollständiges Austauschen des mit Wasser vollständig mischbaren organischen Lösungsmittels gegen das organische Bindemittelsystem umfassend ein oder mehrere polymerisierbare Monomere, so dass die erfindungsgemäß anzuwendende Zusammensetzung resultiert.

Die erfindungsgemäße Zusammensetzung in der Anwendung:

Besonders bevorzugt wird eine erfindungsgemäße Zusammensetzung angewendet in einem Verfahren zum Infiltrieren und/oder Versiegeln von Zahnhartsubstanz und zur Detektion von Karies im infiltrierten Bereich bzw. unter der Versiegelung mittels Fluoreszenz sowie zur Bestimmung von Veränderungen des Infiltrationszustands bzw. der Versiegelung mittels Fluoreszenz. Nach dem Infiltrieren und/oder dem Versiegeln von Zahnhartsubstanz (gesund oder kariös) mittels der erfindungsgemäßen Zusammensetzung und nach dem Aushärten der Zusammensetzung in Kontakt mit der Zahnhartsubstanz unterliegt der infiltrierte Bereich bzw. die Versiegelung den üblichen Beanspruchungen einer Zahnfläche. Da die erfindungsgemäße Zusammensetzung und somit auch die ausgehärtete Versiegelung auf einer Kunststoff-Matrix basiert, ist trotz des erfindungsgemäß bevorzugten hohen Füllgrades damit zu rechnen, dass beispielsweise die Schichtdicke einer Versiegelung in einer tiefen Fissur im Laufe der abnimmt. Der behandelnde Zahnarzt kann die im Laufe der Zeit auftretenden Veränderungen einer solchen Versiegelung (bzw. die entsprechenden Veränderungen des Infiltrationszustandes), insbesondere die Reduktion ihrer Dicke, auch unter Zuhilfenahme der üblichen optischen Hilfsmittel (insbesondere einer Lupe) nur unzureichend verfolgen und unterliegt somit beispielsweise der Gefahr, dass er von der Entfernung einer fast vollständig abgetragenen Versieglung noch absieht obwohl diese bereits derart dünn und brüchig geworden ist, dass sie ihren eigentlichen Zweck, nämlich der Entstehung von Karies vorzubeugen, nicht mehr hinreichend genügen kann. Zwar kann der behandelnde Zahnarzt bei Anwendung der erfindungsgemäßen Zusammensetzung erkennen, ob sich bereits Karies unter der Versiegelung gebildet hat, doch folgt in einem solchen Falle die Entfernung der Versiegelung nicht mehr im prophylaktischen Sinne rechtzeitig. Vorteilhaft ist es deshalb, die erfindungsgemäße Zusammensetzung in einem kombinierten Verfahren zur Anwendung zu bringen, welches das Infiltrieren und/oder Versiegeln von Zahnhartsubstanz und die Detektion von Karies im infiltrierten Bereich bzw. unter der Versieglung mittels Fluoreszenz sowie die Bestimmung von Veränderungen der Versiegelung bzw. des Infiltrationszustands mittels Fluoreszenz umfasst. Es ist eine überraschende Erkenntnis, die im Rahmen der vorliegenden Erfindung gemacht wurde, dass die üblichen Veränderungen einer applizierten Versiegelung bzw. einer Infiltration auf Basis einer erfindungsgemäßen Zusammensetzung hervorragend überwacht werden können, indem am zu untersuchenden Zahn Daten einer Fluoreszenzmessung erfasst werden. Die erfindungsgemäß anzuwendenden Zusammensetzungen sind besonders abrasionsbeständig; es ist deshalb von besonderem Interesse, die Langzeitperformance der aus ihnen hergestellten Versiegelungen bzw. der mit ihnen infiltrierten Bereiche einfach und über lange Zeiträume überwachen zu können.

Die vorliegende Erfindung betrifft dementsprechend auch ein Verfahren zum Erfassen von Daten einer Fluoreszenzmessung an einem Zahn mit folgenden Schritten:

- Bestrahlen einer infiltrierten und/oder versiegelten Zahnfläche mit einer Anregungsstrahlung, welche die Emission von Fluoreszenzstrahlung bewirkt, wobei die Zahnfläche mit einer erfindungsgemäßen Zusammensetzung wie vorstehend stofflich definiert infiltriert und/oder versiegelt ist, wobei der Infiltrant in der infiltrierten Zahnfläche bzw. die Versiegelung auf der Zahnfläche so ausgestaltet ist, dass er bzw. sie sowohl für die die Anregungsstrahlung als auch für die emittierte Fluoreszenzstrahlung durchlässig ist,

- Erfassen von entsprechenden Fluoreszenzstrahlungs-Daten.

Die Anregungsstrahlung ist dabei vorzugsweise eine Strahlung mit Licht im Wellenlängenbereich von 600 nm bis 670nm; die emittierte Fluoreszenzstrahlung umfasst dann vorteilhafterweise die bereits oben genannte Wellenlänge von 700 nm, welche für kariöse und gesunde Zahnhartsubstanz typisch ist.

Es versteht sich, dass vorzugsweise Fluoreszenzstrahlungs-Daten für diejenigen Wellenlängenbereiche erfasst werden, für die die Versiegelung (ausreichend) durchlässig ist. Zur Detektion von Karies im infiltrierten Bereich bzw. unter der Versiegelung mittels Fluoreszenz ist es somit beispielsweise bevorzugt, bei Einsatz einer Anregungsstrahlung im Wellenlängenbereich von 600 nm bis 670 nm die Fluoreszenzstrahlung einer Wellenlänge von 700 nm zu erfassen. Bei gleicher Anregungsstrahlung wird vorzugsweise zur Bestimmung von Veränderungen der Versiegelung mittels Fluoreszenz eine Wellenlänge im Bereich von 680 nm bis 900 nm erfasst.

Der Vergleich entsprechender Daten, welche in größerem zeitlichen Abstand voneinander erfasst wurden, ermöglicht Aussagen einerseits hinsichtlich der Bildung von Karies im Infiltrierten Bereich bzw. unter der Versiegelung und andererseits hinsichtlich Veränderungen der Versiegelung.

Der Einsatz einer erfindungsgemäßen Zusammensetzung in einem Verfahren zum Infiltrieren und/oder Versiegeln von Zahnhartsubstanz ermöglicht zusammenfassend einerseits zur Detektion von Karies im Infiltrierten Bereich bzw. unter der Versiegelung mittels Fluoreszenz, andererseits aber auch die routinemäßige Überwachung von Veränderungen der Versiegelung selbst. Der behandelnde Zahnarzt ist somit nicht alleine auf die üblichen optischen Verfahren zur Bewertung einer dentalen Versiegelungssituation angewiesen, sondern kann durch Verwendung von Fluoreszenztechniken in sehr vorteilhafter Weise den rechtzeitigen Zeitpunkt für den Austausch einer Versiegelung abschätzen und bestimmen.

In Einzelfällen hat es sich in eigenen Untersuchungen als sinnvoll erwiesen, erfindungsgemäße Zusammensetzungen anzuwenden, die zusätzlich einen Fluoreszenzfarbstoff umfassen. Hierbei sollte jedoch der zusätzliche Fluoreszenzfarbstoff so ausgewählt sein, dass er bei Anregung durch eine gegebene Anregungsstrahlung nicht vollständig die Fluoreszenzstrahlung von gesundem bzw. kariöser Zahnhartsubstanz überdeckt. Bei Einsatz eines definierten Fluoreszenzfarbstoffes, der bei Anregung durch eine gegebene Anregungsstrahlung Licht einer Wellenlänge emittiert, die nicht in relevantem Ausmaß mit derjenigen Wellenlänge überlappt, die von kariöser oder gesunder Zahnhartsubstanz bei Anregung mit der gleichen Anregungsstrahlung emittiert wird, ist es in besonders günstiger Weise möglich, über einen längeren Zeitraum hinweg Veränderungen an einer Versiegelung zu beobachten. Der behandelnde Zahnarzt erhält auf diese Weise weitere Informationen zur dentalen Versiegelungssituation, die ihn noch besser in die Lage versetzt zu entscheiden, ob ein Austausch einer applizierten Versiegelung vorteilhaft wäre oder nicht.

In eigenen Untersuchungen hat sich gezeigt, dass erfindungsgemäße Zusammensetzungen zu Versiegelungen mit hervorragender prophylaktischer und therapeutischer Wirkung führen und über lange Zeit ihre mechanischen Eigenschaften auf dem Zahn bewahren, wenn sie in üblicher Weise nach entsprechender Vorbehandlung des Zahnes auf diesen appliziert und dort ausgehärtet werden. Aufgrund des Umstands, dass mit den erfindungsgemäßen Zusammensetzungen ein Langzeitschutz möglich ist, erscheint es besonders vorteilhaft, dass mit der vorliegenden Erfindung nun

Verfahren angegeben werden, welche ein einfaches Monitoring der Versiegelungsqualität ebenso ermöglichen wie die erforderliche Detektion auf die Bildung von Karies im Infiltrierten Bereich bzw. unter der Versiegelung.

Im erfindungsgemäßen Verfahren ist die versiegelte Zahnfläche vorzugsweise eine Zahnfläche eines komplett versiegelten Zahnes oder eines im Bereich von Fissuren und Grübchen versiegelten Zahnes. Insbesondere handelt es sich bei der versiegelten Zahnfläche vorzugsweise um eine okklusale, linguale, bukkale, distale oder mesiale Zahnfläche.

In einem besonders bevorzugten erfindungsgemäßen Verfahren ist die Zahnfläche eine Zahnfläche eines im Bereich von Fissuren und Grübchen versiegelten Zahnes; die Zahnfläche ist dabei so mit der (ausgehärteten) Zusammensetzung versiegelt, dass das Versiegelungsmaterial zumindest abschnittweise eine Dicke von 0,5 mm oder mehr besitzt, vorzugsweise von 1 mm oder mehr. Es versteht sich, dass in diesem Fall die Zusammensetzung vorzugsweise so ausgestaltet ist, dass sie (wie weiter oben für eine bevorzugte Zusammensetzung ausgeführt) zu einem Versiegelungsmaterial aushärtbar ist, das bei einer Schichtdicke von 1 mm einen Transmissionsgrad von 75 % oder mehr, bevorzugt von 70 % oder mehr, besonders bevorzugt von 80 % oder mehr für eine senkrecht einfallende Strahlung der Wellenlängen von 655 nm und/oder 700 nm besitzt, vgl. dazu die Ausführungen weiter oben.

Generell gilt, dass in erfindungsgemäßen Verfahren die vorstehend als bevorzugt bezeichneten erfindungsgemäßen Zusammensetzungen bevorzugt eingesetzt werden.

Die vorliegende Erfindung betrifft auch ein Verfahren zur Vorbereitung einer Kariesdiagnostik und/oder zur Bestimmung von Veränderungen des Infiltrationszustands eines Zahnes und/oder zur Bestimmung von Veränderungen der Versiegelung an einem Zahn eines Patienten, mit folgenden Schritten:

- Erfassen von Daten einer Fluoreszenzmessung gemäß einem entsprechenden erfindungsgemäßen Verfahren wie vorstehend beschrieben (insbesondere wie vorstehend als besonders bevorzugt bezeichnet), wobei eine infiltrierte und/oder versiegelte Zahnfläche eines Patienten bestrahlt wird, und
- Vergleich der erfassten Fluoreszenzstrahlungs-Daten mit Normdaten, ohne Wechselwirkung mit dem Patienten.

Dies entspricht natürlich einem erfindungsgemäßen Verfahren zum Erfassen von Daten einer Fluoreszenzmessung an einem Zahn mit folgendem zusätzlichen Schritt zur Vorbereitung einer Kariesdiagnostik und/oder zur Bestimmung von Veränderungen des Infiltrationszustands eines Zahnes und/oder zur Bestimmung von Veränderungen der Versiegelung an einem Zahn eines Patienten:

- Vergleich der erfassten Fluoreszenzstrahlungs-Daten mit Normdaten, ohne Wechselwirkung mit dem Patienten.

In solchen erfindungsgemäßen Verfahren zur Vorbereitung einer Kariesdiagnostik erfolgt der Vergleich vorzugsweise automatisiert mittels elektronischer Datenverarbeitung.

Vorzugsweise werden die besagten Schritte eines erfindungsgemäßen Verfahrens zum Erfassen von Daten einer Fluoreszenzmessung bzw. zur Vorbereitung einer Kariesdiagnostik und/oder zur Bestimmung von Veränderungen des Infiltrationszustands eines Zahnes und/oder zur Bestimmung von Veränderungen der Versiegelung mit einem zeitlichen Abstand von einem Monat oder mehr zwischen den Bestrahlungsschritten wiederholt.

Die jeweiligen Resultate des Vergleichs der erfassten Fluoreszenzstrahlungs-Daten mit den Normdaten können für die Zwecke einer anschließenden separaten Diagnose und/oder separaten Beurteilung der festgestellten Veränderungen weitergegeben bzw. weitergeleitet werden.

Ein entsprechendes Verfahren zur Kariesdiagnostik an einem Zahn eines Patienten umfasst die folgenden Schritte:

- Bestrahlen einer versiegelten Zahnfläche mit einer Anregungsstrahlung, welche die Emission von Fluoreszenzstrahlung bewirkt, wobei die Zahnfläche mit einer erfindungsgemäßen Zusammensetzung wie vorstehend stofflich definiert infiltriert und/oder versiegelt ist, wobei der Infiltrant in der infiltrierten Zahnfläche bzw. die Versiegelung so ausgestaltet ist, dass er bzw. sie sowohl für die die Anregungsstrahlung als auch für die emittierte Fluoreszenzstrahlung durchlässig ist,
- Erfassen von entsprechenden Fluoreszenzstrahlungs-Daten,
- Vergleich der erfassten Fluoreszenzstrahlungs-Daten mit Normdaten,
- Feststellen von signifikanten Abweichungen der Fluoreszenzstrahlungs-Daten mit den Normdaten, und
- Erstellen einer Kariesdiagnose.

Es versteht sich, dass auch insoweit die bevorzugten erfindungsgemäßen Zusammensetzungen vorzugsweise

eingesetzt werden.

Die vorliegende Erfindung betrifft auch ein Kit (a) zum Infiltrieren und/oder Versiegeln von Zahnhartsubstanz und (b) (i) zur Detektion von Karies im infiltrierten Bereich bzw. unter der Versiegelung mittels Fluoreszenz und/oder (ii) zur Bestimmung von Veränderungen des Infiltrationszustands eines Zahnes und/oder zur Bestimmung von Veränderungen der Versiegelung an einem Zahn eines Patienten, umfassend:

- eine erfindungsgemäße Zusammensetzung,

- eine Vorrichtung zur Detektion von aus der Zahnhartsubstanz und/oder der Versiegelung und/oder dem Infiltranten im infiltrierten Bereich emittierter Fluoreszenzstrahlung.

Hinsichtlich bevorzugter erfindungsgemäßer Zusammensetzungen sei auf die Ausführungen weiter oben verwiesen, die hier entsprechend gelten. Vorrichtungen zur Detektion von Fluoreszenzstrahlung aus der Zahnhartsubstanz sind weiter oben unter Verweis auf entsprechende Veröffentlichungen beschrieben; derartige Vorrichtungen sind auch zur Detektion von Fluoreszenzstrahlung aus der Versiegelung bzw. dem Infiltranten im infiltrierten Bereich geeignet, wobei eine solche Fluoreszenzstrahlung selbstverständlich das Vorhandensein fluoreszierender Stoffe in der Versiegelung bzw. dem Infiltranten voraussetzt.

Die vorliegende Erfindung und besonders bevorzugte Ausgestaltungen werden anhand von Bestimmungsmethoden und Beispielen nachfolgend näher erläutert.

<u>Methode 1: Bestimmung der mittleren Teilchengröße und der Polydispersität von Füllstoffpartikeln:</u>

Die mittlere (hydrodynamische) Teilchengröße (average hydrodynamic particle size) von Füllstoffpartikeln und ihre Polydispersität wird für die Zwecke der vorliegenden Erfindung in üblicher Weise mittels dynamischer Lichtstreuung (DLS) bestimmt.

Die Bestimmung der mittleren Teilchengröße wird dabei bevorzugt mit dem Gerät "Partikelsizer ALV-HPPS" der Firma ALV-Laser Vertriebsgesellschaft mbH, Langen, durchgeführt. Die Messung erfolgt mit einem Laser mit einer Wellenlänge von 632,8 nm. Die Proben werden bei 25°C in Quarzglasküvetten, mit einer Schichtdicke von 10 mm, vermessen. Als Lösungsmittel wird über Natrium getrocknetes und frisch destilliertes Isopropanol verwendet. Dabei wird die Intensität des gestreuten Lichts in Relation zur Intensität des Primärstrahls bei 13 Winkeln in 10° Schritten in einem Bereich von 30° bis 150° erfasst. Zur Auswertung der dynamischen Lichtstreuung kommt die Software "ALV HPPS 5" zum Einsatz. Die Auswertung der Korrelationsfunktion erfolgt mittels der Auswertungsmethoden *Simple Fit* (für monodisperse Proben (Polydispersität < 0.1 )) und *Regularized Fit* (für polydisperse Verteilungen (Polydispersität > 0.1)).

<u>Methode 2: Bestimmung der Haftung der gehärteten erfindungsgemäßen Zusammensetzung an Zahnschmelz:</u>

Die Bestimmung der Haftung (siehe Beispiel 17 und die zugehörige Tabelle) erfolgt analog zur ISO TS 11405:2003 E. Hierzu wird Zahnschmelz von Rinderzähnen mit Schleifpapier (6000er Körnung) geschliffen. Anschließend wird der geschliffene Zahnschmelz für 30 Sekunden mit einem Phosphorsäureätzgel (35%ig) behandelt, für 20 Sekunden mit einem Wasserspray gespült und für weitere 10 Sekunden mit Druckluft trockengeblasen. Auf die trockene, angeätzte Zahnschmelzoberfläche wird dann ein Silikonring mit einem Innendurchmesser von 5 mm gelegt. Die Zusammensetzung, deren Haftung an Zahnschmelz getestet werden soll, wird in den Silikonring gefüllt. Bei lichthärtenden Zusammensetzungen lässt man das Material für 10 Sekunden einwirken. Anschließend wird das Material für 20 Sekunden bei geeigneter Wellenlänge ausgehärtet. Nach dem Härten der Zusammensetzung wird der Silikonring entfernt. Der so hergestellte Prüfkörper wird für 24 Stunden bei 37°C in einem Dampfbad gelagert, bevor er mit einer Scherhaftapparatur nach Reinhardt (Reinhardt KJ, Phillip Journal, 3-4, 1997, 101-104) mit einer Vorschubgeschwindigkeit von 1 mm/s vermessen wird. Die Haftung wird in MPa angegeben.

Für die Bestimmung der Haftung nach Temperaturzyklen ("Haftung TC") wird der Prüfkörper 3000 Temperaturzyklen zwischen 5 und 55°C ausgesetzt (hierzu wird er alternierend jeweils für 1 Minute in ein Bad der Temperatur 55 °C gelegt und anschließend für 1 Minute in ein Bad der Temperatur 5 °C) und anschließend vermessen.

<u>Methode 3: Bestimmung der Biegefestigkeit der gehärteten erfindungsgemäßen Zusammensetzung:</u>

Die Bestimmung der Biegefestigkeit (siehe Beispiel 17 und die zugehörige Tabelle) erfolgt analog zur ISO 4049. Dazu wird ein Prüfkörper mit einer Dimension von 2x2 mm vermessen. Bei lichthärtenden Zusammensetzungen wird für 20 Sekunden bei geeigneter Wellenlänge ausgehärtet.

Für die Bestimmung der Biegefestigkeit nach Temperaturzyklen ("Biegefestigkeit TC"), wird der Prüfkörper 3000 Temperaturzyklen zwischen 5 und 55°C ausgesetzt (hierzu wird er alternierend jeweils für 1 Minute in ein Bad der

Temperatur 55 °C gelegt und anschließend für 1 Minute in ein Bad der Temperatur 5 °C) und anschließend vermessen.

Methode 4: Bestimmung der Abrasion:

Zur Bestimmung der Abrasion (siehe Beispiel 17 und die zugehörige Tabelle) wird die 3-Medien-Abrasions-Methode nach ACTA (Academic Center of Dentistry Amsterdam) gemäß J. Dent. Suppl. 1, 1994, 22, 21-27 (200000 Zyklen) durchgeführt.

Methode 5: Bestimmung der spezifischen Oberfläche nach dem BET-Verfahren:

Die Bestimmung der spezifischen Oberfläche erfolgt durch Gasadsorption nach dem BET-Verfahren gemäß DIN ISO 9277. Es wird das Volumetrische Verfahren (Punkt 6.3.1 der DIN ISO 9277) angewendet. Als Messgas wird Helium mit einer Reinheit von > 99,99 % verwendet.

Methode 6: Bestimmung des Transmissionsgrades bei bestimmten Wellenlängen:

Die Bestimmung des Transmissionsgrades erfolgt an einem Lambda 650 UV-Vis-Spektrometer der Firma PerkinElmer (Rodgau). Dazu wird ein Prüfkörper mit einer Dimension von 10x10x1 mm hergestellt und so in eine Transmissionshalterung eingespannt, dass die einfallende Strahlung senkrecht auf die 10x10 mm Fläche des Prüfkörpers trifft und den Prüfkörper durchstrahlt (Probendicke 1 mm). Die Messung wird einmal ohne Prüfkörper und einmal mit Prüfkörper durchgeführt. Der Transmissionsgrad entspricht dem Quotienten zwischen der Wellenlängenintensität $I_o$ ohne Prüfkörper und der Wellenlängenintensität $I$ mit Prüfkörper, bei entsprechender Wellenlänge. Bevorzugt wird der Transmissionsgrad in Prozent [%] angegeben.

Methode 7: Bestimmung des Transmissionsgrades im visuellen Bereich:

Die Bestimmung des Transmissionsgrades im visuellen Bereich erfolgt an einem Color Flex d/8° - Farbmessgerät der Firma HunterLab. In einer Teflonform wird dazu ein Prüfkörper mit einer Dicke von 1 mm und einem Durchmesser von 20 mm hergestellt. Zum Aushärten wird die Zusammensetzung für 30 Minuten mit Blaulicht bestrahlt. Die Messung erfolgt bei Raumtemperatur; die für die Messung zulässige relative Luftfeuchtigkeit liegt zwischen 5 und 85 %. Die Auswertung der Messergebnisse erfolgt mit der Software "Easymatch QC" (Version 4.3) der Firma HunterLab. Vordem Vermessen der Probe wird das Gerät entsprechend der Gebrauchsanweisung kalibriert. Die Probe wird anschließend viermal gegen weißen Hintergrund und viermal gegen schwarzen Hintergrund gemessen. Die Transmission wird nach der Formel [Transmission = 100 - Opazität] berechnet. Die gemessenen Transmissionen werden nachfolgend als "Transmission [%] (Vis)" bezeichnet und ohne eine definierte Wellenlänge angegeben.

Methode 8: Bestimmung des Anfließverhaltens: Kontaktwinkel auf trockenem Zahnschmelz

Das Anfließverhalten wurde ermittelt durch Messung des Kontaktwinkels auf trockenem Zahnschmelz.

Für die Kontaktwinkelmessungen (KW, in Grad [°]) auf trockenem Zahnschmelz wurde ein extrahierter humaner Molar verwendet. Dieser wurde vor der Messung mit einem Zellstofftuch durch Abwischen getrocknet. Anschließend wurde ein Tropfen des zu untersuchenden Materials auf den Schmelzbereich des Zahns aufgetragen. Anschließend wurde der Kontaktwinkel über einen Zeitraum von 30 Sekunden mit einem Kontaktwinkelmessgerät (DSA 100, Firma Krüss) bestimmt.

Methode 9: Bestimmung der Wasseraufnahme

Die Wasseraufnahme wurde analog zu ISO 4049 bestimmt. Dazu wurden die Zusammensetzungen luftblasenfrei in entsprechende Teflonformen eingefüllt, mit Folien und Glasplatten bedeckt und mit einer Schraubzwinge wurden die Überschüsse herausgepresst. Die Probekörper mit einem Durchmesser von 15,0 $\pm$ 0,1 mm und einer Höhe von 1,0 $\pm$ 0,1 mm wurden segmentweise lichtgehärtet. Anschließend wurden die Probekörper in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_1$, erreicht war.

Nach dem vollständigen Trocknen wurde der Durchmesser zweimal rechtwinklig zueinander mit einer Messgenauigkeit von 0,01 mm gemessen und daraus der mittlere Durchmesser berechnet. Die Dicke des Probekörpers wurde in der Mitte und an vier in gleichem Abstand liegenden Stellen des Randes auf 0,01 mm gemessen. Aus dem mittleren Durchmesser und der mittleren Dicke wurde das Volumen, V, berechnet.

Anschließend wurden die Probekörper für 7 Tage in Wasser bei 37°C gelagert. Danach wurden die Probekörper herausgenommen, mit Wasser abgespült und abgetupft, bis auf der Oberfläche keine Feuchtigkeit mehr sichtbar war. Die Probekörper wurden 15 s in der Luft hin- und hergeschwenkt und 1 min nach dem Herausnehmen aus dem Wasser gewogen. Diese Masse wird als $m_2$ angegeben.
Anschließend wurden die Probekörper erneut in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_3$, erreicht war.
Die Wasseraufnahme, $W_{sp}$, wurde nach folgender Gleichung berechnet:

$$W_{\mathrm{sp}} = \frac{m_2 - m_3}{V}$$

Dabei ist

$m_2$ die Masse des Probekörpers nach Wasserlagerung für 7 Tage in $\mu$g;
$m_3$ die Masse des wieder getrockneten Probekörpers in $\mu$g;
$V$ das Volumen des Probekörpers in $mm^3$

Synthesebeispiel: Synthese von Allophanat der Formel (2) (Monomer gemäß Komponente (b1)):

0,95 g (4,84 mmol) 3(4), 8(9)-Bis(hydroxymethyl)tricyclo[5.2.1.$0^{2,6}$]decan wurden in 10 mL Toluol gelöst und mit 0,04 g BHT und 0,103 g der Katalysatorlösung versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 72 Stunden wurden weitere 0,102 g Katalysatorlösung zugesetzt und weiter erhitzt, bis keine Isocyanatbande mehr detektiert wurde. Das Lösungsmittel wurde am Rotationsverdampfer entfernt. Das Allophanat der Formel (2) wurde in einer Ausbeute von 3,83 g (4,69 mmol, 97%) als leicht gelbliches Öl erhalten.

Beispiel 1: Allgemeine Vorschrift zur Herstellung einer radikalisch polymerisierbaren erfindungsgemäßen Zusammensetzung:

1.1 Herstellung von mittels organischer Strukturelemente oberflächenmodifizierten Kieselsäurepartikel:

Es wird eine kolloidale Dispersion von nasschemisch hergestellten, anorganischen Füllstoffpartikeln geeigneter mittlerer Teilchengröße bereitgestellt, wobei als Dispersionsmittel eine organische, wasserlösliche Matrix, beispielsweise Isopropanol, eingesetzt wird.
Die Dispersion wird vermischt mit

(a) einer ausreichenden Menge eines Oberflächenmodifizierungsmittels, welches einerseits Alkoxysilan-Gruppen besitzt und andererseits organische Gruppen, welche mit radikalisch polymerisierbaren Monomeren unter Bildung kovalenter Bindungen umsetzbar sind (ein solches Oberflächenmodifizierungsmittel ist z.B. 3-Methacryloyloxypropyltrialkoxysilan),

(b) der zwei bis zehnfachen molaren Menge Wasser, bezogen auf das Oberflächenmodifizierungsmittel, sowie

(c) ca. 1 Gew.-%, bezogen auf das Oberflächenmodifizierungsmittel, Methacrylsäure.

Die resultierende Mischung wird bei 50-80°C mindestens 8h gerührt, sodass die Oberflächenmodifizierung der eingesetzten anorganischen Füllstoffpartikel vollständig ablaufen kann.

1.2 Herstellung der Zusammensetzung:

Zu der gemäß Beispiel 1.1 erhaltenen Zwischenproduktmischung, die oberflächenmodifizierte, nasschemisch hergestellte, anorganische Füllstoffpartikel in einem Dispersionsmittel umfasst, wird dann ein organisches Bindemittelsystem zugesetzt, welches ein oder mehrere radikalisch polymerisierbare Monomere umfasst. Flüchtige Bestandteile der resultierenden Mischung (insbesondere Wasser, das Dispersionsmittel der anfänglich eingesetzten anorganischen Füllstoffpartikel sowie der durch Umsetzung des Alkoxysilans mit Wasser entstandene Alkohol) werden im Vakuum

abgetrennt.

Beispiel 2: Vorschrift zur Herstellung einer erfindungsgemäßen Zusammensetzung:

Zum Herstellen einer bevorzugten erfindungsgemäßen Zusammensetzung werden eingesetzt:

2.1 Bestandteile des organischen Bindemittelsystems (Monomer gemäß Komponente (b2)):

60g Glycerindimethacrylat (GlyDMA),

50g Triethylenglykoldimethacrylat (TEDMA),

60g Bisphenol-A-Glycidyl-Methacrylat (Bis-GMA).

2.2 Materialien zur Herstellung von mittels organischer Strukturelemente oberflächenmodifizierten, nicht flammhydrolytisch hergestellten Kieselsäurepartikeln:

864g einer ethanolischen, 20%igen Suspension nicht flammhydrolytisch hergestellter Kieselsäurepartikel (mit einer mittleren Teilchengröße von 40 nm),

40g gamma-Methacryloyloxypropylsilan (23 % bezogen auf den Feststoffanteil, zur Oberflächenmodifizierung),

0,4g Methacrylsäure und

18g Wasser.

2.3 Additive:

0,25g 2,6-Di-tert.butyl-4-methylphenol (BHT),

2,5g 2-(2H-benzotriazol-2-yl)-p-cresol (Tinuvin P),

0,5g Campherchinon (CC) und

2,4g Ethyl-*p*-*N,N*-dimethylaminobenzoat (DABE).

Aus den unter 2.2 genannten Materialien werden gemäß Beispiel 1 (allgemeine Vorschrift), Punkt 1.1, mittels organischer Strukturelemente oberflächenmodifizierte, nicht flammhydrolytisch hergestellte Kieselsäurepartikel hergestellt.

Diese Kieselsäurepartikel (in Form der Zwischenproduktmischung, d.h. in Dispersionsittel), die Bestandteile gemäß 2.1 und die Additive gemäß 2.3 werden gründlich miteinander vermischt. Flüchtige Bestandteile der resultierenden Mischung (insbesondere Wasser, das Dispersionsmittel der anfänglich eingesetzten anorganischen Füllstoffpartikel sowie der durch Umsetzung des Alkoxysilans mit Wasser entstandene Alkohol) werden im Vakuum abgetrennt.

Es resultiert eine erfindungsgemäße Zusammensetzung in Pastenform, die durch Bestrahlung mit Licht einer Wellenlänge von 470 nm lichthärtbar ist.

Beispiele 3 bis 9: Vorschrift zur Herstellung von erfindungsgemäß anzuwendenden Zusammensetzungen mit unterschiedlichen Füllstoffgehalten:

Die Herstellung erfolgt analog zu Beispiel 2, jedoch werden unterschiedliche Mengen der oberflächenmodifizierten Kieselsäurepartikel (mit einer Teilchengröße von 40 nm) die gemäß Beispiel 1 oberflächenfunktionalisiert wurden, verwendet.

| | |
|---|---|
| Beispiel 3 | 19,5g Kieselsäurepartikel (entspricht 10 Gew.-%) |
| Beispiel 4 | 43,9g Kieselsäurepartikel (entspricht 20 Gew.-%) |
| Beispiel 5 | 75,3g Kieselsäurepartikel (entspricht 30 Gew.-%) |

(fortgesetzt)

| | |
|---|---|
| Beispiel 6 | 117,1g Kieselsäurepartikel (entspricht 40 Gew.-%) |
| Beispiel 7 (entspricht Beispiel 2) | 175,7g Kieselsäurepartikel (entspricht 50 Gew.-%) |
| Beispiel 8 | 263,5g Kieselsäurepartikel (entspricht 60 Gew.-%) |
| Beispiel 9 (Vergleich) | 0g Kieselsäurepartikel (entspricht 0 Gew.-%); ungefüllte Zusammensetzung |

Beispiele 10 bis 16: Vorschrift zur Herstellung von erfindungsgemäß anzuwendenden Zusammensetzungen mit unterschiedlichen Füllstoffgehalten:

Die Herstellung der erfindungsgemäß anzuwendenden Zusammensetzungen erfolgte analog zu Beispiel 2, jedoch wurden in allen Beispielen 10 bis 15 die Bestandteile des organischen Bindemittelsystems durch die folgenden Bestandteile ausgetauscht:

Bestandteile des organischen Bindemittelsystems (anstelle der Bestandteile gemäß 2.1; Kombination von Monomeren gemäß Komponenten (b1) und (b2)):

60g Glycerindimethacrylat (GlyDMA),
50g Triethylenglykoldimethacrylat (TEDMA),
60g Bis(methacryloyloxymethyl)-tricyclo[5.2.1.$0^{2,6}$]decan.

Die Mengen der oberflächenmodifizierten Kieselsäurepartikel (mit einer Teilchengröße von 40 nm), die gemäß Beispiel 1 oberflächenfunktionalisiert wurden, wurden ebenfalls entsprechend der nachfolgenden Tabelle variiert.

| | |
|---|---|
| Beispiel 10 | 19,5g Kieselsäurepartikel (entspricht 10 Gew.-%) |
| Beispiel 11 | 43,9g Kieselsäurepartikel (entspricht 20 Gew.-%) |
| Beispiel 12 | 75,3g Kieselsäurepartikel (entspricht 30 Gew.-%) |
| Beispiel 13 | 117,1g Kieselsäurepartikel (entspricht 40 Gew.-%) |
| Beispiel 14 | 175,7g Kieselsäurepartikel (entspricht 50 Gew.-%) |
| Beispiel 15 | 263,5g Kieselsäurepartikel (entspricht 60 Gew.-%) |
| Beispiel 16 (Vergleich) | 0g Kieselsäurepartikel (entspricht 0 Gew.-%); ungefüllte Zusammensetzung |

Vergleichsbeispiel 1 (vgl. WO 2007/028159 A2, Beispiel 1):

9,5 g Nanocryl D120 (50 Gew.-% $SiO_2$ 20 nm Partikel, dispergiert in alkoxyliertem Pentaerythritol-tetramethacrylat; Hanse Chemie) und 0,5 g Lucirin TPO (2,4,6-Trimethylbenzoyldiphenylphosphinoxid; Aldrich) wurden bei 50°C verblendet. Es resultiert eine transparente Zusammensetzung, die durch Bestrahlung mit Licht einer Wellenlänge von 470 nm lichthärtbar ist.

Vergleichsbeispiel 2 (vgl. WO 2007/028159 A2, Beispiel 2):

9,74 g Nanocryl D322 (50 Gew.-% $SiO_2$ 20 nm Partikel, dispergiert in Urethandimethacrylat (80 Gew.-%) und Triethylenglycoldimethacrylat; Hanse Chemie), 0,01g Campherchinon, 0,05g EDAB (Ethyl-(4-*N*,*N*-dimethylamino)benzoat; Aldrich) und 0,2 g Lucirin TPO (2,4,6- Trimethylbenzoyldiphenylphosphinoxid; Aldrich) wurden bei 50°C verblendet. Es resultiert eine transparente Zusammensetzung, die durch Bestrahlung mit Licht einer Wellenlänge von 470 nm lichthärtbar ist.

Vergleichsbeispiel 3 (vgl. WO 2007/028159 A2, Beispiel 3):

2,5 g PENTA (Dipentaerythritolpentacrylatphosphorsäureester), 1,8g Nanocryl XP21/0568 (50 Gew.-% $SiO_2$ 20 nm Partikel, dispergiert in Triethylenglycoldimethacrylat; Hanse Chemie), 1,5 g Nanocryl XP21/0746 (50 Gew.-% $SiO_2$ 20 nm Partikel, dispergiert in Hydroxyethylmethacrylat; Hanse Chemie), 2,5 g Nanocryl D322 (50 Gew.-% $SiO_2$ 20 nm Partikel, dispergiert in Urethandimethacrylat (80 Gew.-%) und Triethylenglycoldimethacrylat; Hanse Chemie), 0,5 g of Nanocryl XP21/1045 (50 Gew.-% $SiO_2$ 20 nm Partikel, dispergiert in Trimethylpropantriacrylat; Hanse Chemie),

1,0 g bis-HEMA-phosphat, 0,02 g Campherchinon, 0,08g EDAB (Ethyl-(4-*N,N*-dimethylamino)benzoat; Aldrich) und 0,1 g Lucirin TPO (2,4,6-Trimethylbenzoyldiphenylphosphinoxid; Aldrich) wurden bei 50°C verblendet. Es resultiert eine transparente Zusammensetzung, die durch Bestrahlung mit Licht einer Wellenlänge von 470 nm lichthärtbar ist.

Beispiel 17: Vergleichende Untersuchung physikalischer und mechanischer Eigenschaften:

Die in den Beispielen 3 bis 16 hergestellten und die in den Vergleichsbeispielen 1 bis 3 hergestellten Zusammensetzungen (soweit erforderlich nach Aushärtung und Herstellung einer geeigneten Prüfköpers) wurden entsprechend der oben genannten Methoden auf ihre Haftung, Haftung TC, Biegefestigkeit, Biegefestigkeit TC, Abrasion, Transmission, Wasseraufnahme und Anfließverhalten (Kontaktwinkel) untersucht. Die ermittelten Ergebnisse sind in der folgenden Tabelle 1 zusammengefasst.

Der Vergleich der Ergebnisse gemäß Tabelle 1 zeigt, dass die erfindungsgemäßen Zusammensetzungen gemäß Beispielen 3 bis 8 sowie 10 bis 15 bei ähnlichem Gehalt an Füllstoff den Vergleichszusammensetzungen hinsichtlich ihrer mechanischen Eigenschaften überlegen sind. Sie weisen eine ausgezeichnete Haftung auf Zahnsubstanz auf, gleichzeitig verfügen sie über ausgesprochen gute mechanische Eigenschaften sowie sehr hohe Transmissionsgrade. Dabei weisen die erfindungsgemäßen Zusammensetzungen eine geringe Wasseraufnahme in ausgehärteter Form und zudem ein ausgezeichnetes Anfließverhalten auf.

Ein Vergleich der Ergebnisse zu den Beispielen 3 bis 8 mit dem Ergebnis zu Beispiel 9 zeigt überdies ebenso wie ein Vergleich der Ergebnisse zu den Beispielen 10 bis 15 mit dem Ergebnis zu Beispiel 16, dass trotz zum Teil sehr hoher Füllstoffgehalte jeweils die Transmission der erfindungsgemäßen Beispiele nicht entscheidend von denen der Vergleichsbeispiele abweicht.

**Tabelle 1: Ergebnisse zu Beispiel 17**

| | Haftung [MPa] | Haftung TC [MPa] | Biegefestigkeit [MPa] | Biegefestigkeit TC [MPa] | Abrasi on [μm] | Transmission[%] (Vis) | Wasseraufnahme [μg/mm$^3$] | Kontaktwinkel [°] |
|---|---|---|---|---|---|---|---|---|
| Vergleichsbeispiel 1 | 12,5 | 10,8 | 71,3 | 65,3 | 92 | 85,0 | n.b. | n.b. |
| Vergleichsbeispiel 2 | 13,1 | 8,9 | 78,3 | 71,6 | 88,7 | 86,1 | n.b. | n.b. |
| Vergleichsbeispiel 3 | 17,9 | 13,2 | 61,5 | 53,2 | 111 | 85,9 | n.b. | n.b. |
| Beispiel 3 | 12,4 | 9,8 | 95,6 | 94,3 | 143 | 89,3 | 17,9 | 31,4 |
| Beispiel 4 | 13,9 | 8,4 | 98,4 | 95,4 | 111 | 88,7 | 16,4 | 33,1 |
| Beispiel 5 | 15,2 | 9,3 | 101,3 | 98,7 | 88 | 88,7 | 16,0 | 33,7 |
| Beispiel 6 | 17,8 | 13,5 | 105,8 | 97,6 | 65 | 88,4 | 14,2 | 34,9 |
| Beispiel 7 | 20,2 | 18,9 | 111,6 | 107,8 | 58 | 87,5 | 13,5 | 35,7 |
| Beispiel 8 | 19,5 | 19 | 125,3 | 118,9 | 53 | 87,3 | 12,2 | 37,3 |
| Beispiel 9 (Vergleich) | 9,5 | 6,3 | 82,9 | 76,1 | 145 | 90,0 | 41,3 | 31,3 |
| Beispiel 10 | 15,7 | 12,4 | 125,2 | 129,2 | 115,8 | 89,1 | 11,8 | 30,5 |
| Beispiel 11 | 17,7 | 10,7 | 128,9 | 130,7 | 89,9 | 88,5 | 11,8 | 33,2 |
| Beispiel 12 | 19,3 | 11,8 | 132,7 | 135,2 | 71,3 | 89,1 | 11,6 | 32,8 |
| Beispiel 13 | 22,0 | 17,1 | 138,6 | 133,7 | 52,7 | 88,7 | 9,9 | 33,9 |
| Beispiel 14 | 25,7 | 24,0 | 146,2 | 147,7 | 47,0 | 87,9 | 8,6 | 35,9 |
| Beispiel 15 | 24,8 | 24,1 | 151,2 | 162,9 | 42,9 | 87,6 | 7,6 | 36,2 |
| Beispiel 16 (Vergleich) | 12,1 | 8,0 | 108,6 | 104,3 | 117,5 | 90,1 | 13,5 | 29,3 |
| n.b. = nicht bestimmt | | | | | | | | |

Beispiel 18: Vergleich der Transmissionen von Prüfkörpern auf Basis unterschiedlicher Zusammensetzungen bei verschiedenen Wellenlängen:

Aus gemäß den Beispielen 3 bis 8 und 10 bis 15 hergestellten erfindungsgemäßen Zusammensetzungen und aus (Vergleichs-)Zusammensetzungen aus den Beispielen 9 und 16 wurden Prüfkörper hergestellt und entsprechend der oben genannten Methode zur Bestimmung des Transmissionsgrades bei bestimmten Wellenlängen auf ihre Transmission bei den Wellenlängen 300, 400 und 500 nm untersucht. Die ermittelten Ergebnisse sind in der nachfolgenden Tabelle 2 zusammengefasst.

Der Vergleich der Ergebnisse gemäß Tabelle 2 zeigt, dass Prüfkörper auf Basis erfindungsgemäßer Zusammensetzungen aus den Beispielen 3 bis 8 bereits ausgezeichnete Transmissionseigenschaften aufweisen, die Transmissionsgrade liegen weit über 80%. Durch Variation der Bestandteile des organischen Bindemittelsystems in den erfindungsgemäßen Beispielen 10 bis 15 konnten die Transmissionswerte vor allem bei den kurzen Wellenlängen von 300, 400 und 500 nm aber noch weiter verbessert werden. Beispiele 9 (Vergleich) und 16 (Vergleich) betreffen ungefüllte Systeme und zeigen daher selbstverständlich noch bessere Transmissionswerte.

**Tabelle 2: Ergebnisse zu Beispiel 18**

| | Transmission 300nm [%, 1mm Schichtdicke] | Transmission 400nm [%, 1mm Schichtdicke] | Transmission 500nm [%, 1mm Schichtdicke] | | Transmission 300nm [%, 1mm Schichtdicke] | Transmission 400nm [%, 1mm Schichtdicke] | Transmission 500nm [%, 1mm Schicht dicke] |
|---|---|---|---|---|---|---|---|
| Beispiel 3 | 88,2 | 90,1 | 90,2 | Beispiel 10 | 92,4 | 94,1 | 94,7 |
| Beispiel 4 | 87,5 | 88,6 | 89,4 | Beispiel 11 | 91,7 | 93,0 | 94,1 |
| Beispiel 5 | 86,4 | 87,9 | 88,3 | Beispiel 12 | 91,1 | 92,5 | 91,8 |
| Beispiel 6 | 85,8 | 86,7 | 87,1 | Beispiel 13 | 89,8 | 91,0 | 91,7 |
| Beispiel 7 | 84,6 | 85,8 | 86,4 | Beispiel 14 | 87,9 | 90,0 | 90,9 |
| Beispiel 8 | 84,1 | 84,6 | 85,4 | Beispiel 15 | 87,9 | 88,9 | 89,7 |
| | | | | | | | |
| Beispiel 9 (Vergleich) | 90,1 | 90,1 | 90,1 | Beispiel 16 (Vergleich) | 94,6 | 94,6 | 94,6 |

Beispiel 19: Vergleich der Transmissionen von trockenen und wassergelagerten Prüfkörper auf Basis erfindungsgemäßer Zusammensetzungen:

Die im Beispiel 18 untersuchten Prüfkörper, die aus den Produkten gemäß den Beispielen 7, 8, 14 und 15 hergestellt wurden, wurden für eine Woche in Wasser gelegt, anschließend trockengetupft und erneut auf ihren Transmissionsgrad vermessen. Die ermittelten Ergebnisse sind in der nachfolgenden Tabelle 3 zusammengefasst.

Der Vergleich der Transmissionsgrade der nicht in Wasser gelagerten Prüfkörper (vgl. Beispiel 18, Tabelle 2) mit den Transmissionsgraden von in Wasser gelagerten Materialien zeigt, dass die Lagerung in Wasser eine lediglich minimale Auswirkung auf den Transmissionsgrad aufweist. Prüfkörper auf Basis erfindungsgemäßer Zusammensetzungen, die sowohl die Monomerkomponente (b1) als auch die Monomerkomponente (b2) aufweisen (Beispiele 14 und 15), zeigen eine besonders geringe Änderung des Transmissionsgrades nach Wasserlagerung.

**Tabelle 3: Ergebnisse zu Beispiel 19 - Werte nach Wasserlagerung**

| | Transmission 300nm [%, 1mm Schichtdicke] | Transmission 400nm [%, 1mm Schichtdicke] | Transmission 500nm [%, 1mm Schichtdicke] | | Transmission 300nm [%, 1mm Schichtdicke] | Transmission 400nm [%, 1mm Schichtdicke] | Transmission 500nm [%, 1mm Schichtdicke] |
|---|---|---|---|---|---|---|---|
| Beispiel 7 | 81,0 | 82,3 | 82,9 | Beispiel 14 | 85,8 | 88,2 | 89,0 |
| Beispiel 8 | 80,8 | 81,4 | 82,0 | Beispiel 15 | 86,1 | 86,9 | 87,9 |

## Patentansprüche

**1.** Zusammensetzung umfassend:

(a) ein Füllstoffsystem bestehend aus oder umfassend mittels organischer Strukturelemente oberflächenmodifizierte, anorganische Füllstoffpartikel, wobei die organischen Strukturelemente mit einem organischen Bindemittelsystem unter Bildung kovalenter Bindungen umsetzbar sind,
wobei die Füllstoffpartikel der Zusammensetzung eine mittlere Teilchengröße im Bereich zwischen 1 nm und 150 nm besitzen,
(b) ein organisches Bindemittelsystem umfassend ein oder mehrere polymerisierbare Monomere,

wobei das Füllstoffsystem in dem organischen Bindemittelsystem (b) dispergiert ist,
zur Anwendung in einem Verfahren zum Infiltrieren und/oder Versiegeln von Zahnhartsubstanz und zur Detektion von Karies im infiltrierten Bereich bzw. unter der Versiegelung mittels Fluoreszenz,
wobei die Gesamtmenge des organischen Bindemittelsystems (b) umfasst (b1) ein, zwei oder mehr Monomere ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen, Alkoxygruppen, Halogenatome oder Trifluormethylgruppen substituiert sind,
- b ist eine natürliche Zahl ausgewählt aus der Gruppe der natürlichen Zahlen 1, 2, 3 und 4,
- jedes Z bedeutet ein Strukturelement, das unabhängig von etwaigen weiteren Strukturelementen Z ausgewählt ist aus der Gruppe bestehend aus

$-O-(C{=}O)-CH{=}CH_2$, $-O-(C{=}O)-C(CH_3){=}CH_2$,

$-(C{=}O)-CH{=}CH_2$, $-(C{=}O)-C(CH_3){=}CH_2$

$-CH{=}CH_2$, $-C(CH_3){=}CH_2$ und $-O-CH{=}CH_2$,

- jeder Index e ist eine natürliche Zahl, die unabhängig von etwaigen weiteren Indizes e ausgewählt ist aus der Gruppe der natürlichen Zahlen 1, 2, 3 und 4,
- jeder Index x bedeutet unabhängig von etwaigen weiteren Indizes x 0 oder 1,
- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist.

**2.** Zusammensetzung zur Anwendung nach Anspruch 1, wobei die Gesamtmenge des organischen Bindemittelsystems (b) umfasst

(b2) ein, zwei oder mehr polymerisierbare Monomere, wobei das oder die polymerisierbaren Monomere keine Verbindungen (Monomere) der vorstehend definierten Struktur $Q(Y_xZ_e)_b$ sind.

**3.** Zusammensetzung zur Anwendung nach einem der vorangehenden Ansprüche, **dadurch gekennzeichnet, dass** ein, zwei oder mehr Verbindungen der Struktur $Q(Y_xZ_e)_b$ der Monomerenkomponente (b1) ein Tricyclo[5.2.1.$0^{2,6}$]-decan- oder Tricyclo[5.2.1.$0^{2,6}$]-decen-Strukturelement aufweisen und Z vorzugsweise ausgewählt ist aus der Gruppe bestehend aus $-O-(C{=}O)-CH{=}CH_2$ und $-O-(C{=}O)-C(CH_3){=}CH_2$, bevorzugt bedeutet Z die Gruppe $-O-(C{=}O)-C(CH_3){=}CH_2$.

**4.** Zusammensetzung zur Anwendung nach einem der vorangehenden Ansprüche, **dadurch gekennzeichnet, dass** Komponente (b1) umfasst oder besteht aus Bis(methacryloyloxymethyl)tricyclo[5.2.1.$0^{2,6}$]decan und/oder Bis(acryloyloxymethyl)tricyclo [5.2.1.$0^{2,6}$]decan.

**5.** Zusammensetzung zur Anwendung nach einem der vorangehenden Ansprüche, wobei das eine Monomer bzw. eines, mehrere oder sämtliche der mehreren polymerisierbaren Monomere der Monomerenkomponente (b2) ausgewählt ist bzw. sind aus der Gruppe bestehend aus Triethylenglykoldimethacrylat (TEDMA), Urethandimethacrylat

(7,7,9-Trimethyl-4,13-dioxo-3,14-dioxa-5,12-diazahexadecan-1,16-dioxydimethacrylat (UDMA)), Glycerindimethacrylat (GlyDMA) und Bisphenol-A-Glycidyl-Methacrylat (Bis-GMA).

**6.** Zusammensetzung zur Anwendung nach einem der vorherigen Ansprüche, wobei die Zusammensetzung so ausgestaltet ist, dass sie zu einem Versiegelungsmaterial aushärtbar ist, das eine Abrasion, bestimmt nach der 3-Medien-Abrasions-Methode nach ACTA, von weniger als 80 μm, bevorzugt weniger als 70 μm, besonders bevorzugt weniger als 60 μm aufweist.

**7.** Zusammensetzung zur Anwendung nach einem der vorangehenden Ansprüche, herstellbar durch ein Verfahren mit den folgenden Schritten:

(a) Bereitstellen oder Herstellen einer kolloidalen Lösung anorganischer Partikel in Wasser,
(b) Austauschen des Wassers gegen ein mit Wasser vollständig mischbares organisches Lösungsmittel,
(c) vor oder nach Schritt (b) Modifizieren der Oberfläche der anorganischen Partikel, so dass Partikel resultieren, die mittels organischer Strukturelemente oberflächenmodifiziert sind, wobei die organischen Strukturelemente mit den Monomeren unter Bildung kovalenter Bindungen umsetzbar sind,
(d) teilweises oder vollständiges Austauschen des mit Wasser vollständig mischbaren organischen Lösungsmittels gegen das organische Bindemittelsystem umfassend ein oder mehrere polymerisierbare Monomere, so dass die Zusammensetzung nach einem der vorangehenden Ansprüche resultiert.

**8.** Zusammensetzung nach einem der vorangehenden Ansprüche, zusätzlich umfassend einen Fluoreszenzfarbstoff.

**9.** Zusammensetzung nach einem der vorangehenden Ansprüche, zur Anwendung in einem Verfahren zur Bestimmung von Veränderungen des Infiltrationszustands bzw. der Versiegelung mittels Fluoreszenz.

**10.** Verfahren zum Erfassen von Daten einer Fluoreszenzmessung an einem Zahn, mit folgenden Schritten:

- Bestrahlen einer infiltrierten und/oder versiegelten Zahnfläche mit einer Anregungsstrahlung, welche die Emission von Fluoreszenzstrahlung bewirkt, wobei die Zahnfläche mit einer stofflich in einem der vorangehenden Ansprüche definierten Zusammensetzung infiltriert und/oder versiegelt ist, wobei der Infiltrant in der infiltrierten Zahnfläche bzw. die Versiegelung auf der Zahnfläche so ausgestaltet ist, dass er bzw. sie sowohl für die die Anregungsstrahlung als auch für die emittierte Fluoreszenzstrahlung durchlässig ist,
- Erfassen von entsprechenden Fluoreszenzstrahlungs-Daten.

**11.** Verfahren nach Anspruch 10, mit folgendem zusätzlichen Schritt zur Vorbereitung einer Kariesdiagnostik und/oder zur Bestimmung von Veränderungen des Infiltrationszustands eines Zahnes und/oder zur Bestimmung von Veränderungen der Versiegelung an einem Zahn eines Patienten:

- Vergleich der erfassten Fluoreszenzstrahlungs-Daten mit Normdaten, ohne Wechselwirkung mit dem Patienten.

**12.** Verfahren nach Anspruch 11, wobei der Vergleich automatisiert mittels elektronischer Datenverarbeitung erfolgt.

**13.** Verfahren nach einem der Ansprüche 10 bis 12, wobei die besagten Schritte mit einem zeitlichen Abstand von einem Monat oder mehr zwischen den Bestrahlungsschritten wiederholt werden.

**14.** Kit (a) zum Infiltrieren und/oder Versiegeln von Zahnhartsubstanz und (b) (i) zur Detektion von Karies im infiltrierten Bereich bzw. unter der Versiegelung mittels Fluoreszenz und/oder (ii) zur Bestimmung von Veränderungen des Infiltrationszustands eines Zahnes und/oder zur Bestimmung von Veränderungen der Versiegelung an einem Zahn eines Patienten, umfassend:

- eine Zusammensetzung nach einem der Ansprüche 1 bis 9,
- eine Vorrichtung zur Detektion von aus der Zahnhartsubstanz und/oder der Versiegelung und/oder dem Infiltranten im infiltrierten Bereich emittierter Fluoreszenzstrahlung.

## Claims

1. Composition comprising:

(a) a filler system consisting of or comprising inorganic filler particles surface-modified by means of organic structural elements, in which the organic structural elements are convertible with an organic bonding system with formation of covalent bonds, in which the filler particles of the composition have a mean particle size in the range between 1 nm and 150 nm,
(b) an organic bonding system comprising one or more polymerizable monomers,

in which the filler system is dispersed in the organic bonding system (b),
for use in a process for the infiltration and/or sealing of hard tooth substance and for the detection, by means of fluorescence, of caries in the infiltrated region or under the sealing,
in which the total amount of the organic bonding system (b) comprises
(b1) one, two or more monomers chosen from the group consisting of compounds (monomers) of the structure $Q(Y_xG_e)_b$, in which the following applies:

- Q represents a saturated or olefinically unsaturated polyalicyclic structural element chosen from the group consisting of bicyclic, tricyclic, tetracyclic, pentacyclic and hexacyclic hydrocarbon radicals, in which one, two or more of the hydrogen atoms of this polyalicyclic structural element Q not substituted by substituents $Y_xZ_e$ are optionally substituted by alkyl groups, alkoxy groups, halogen atoms or trifluoromethyl groups,
- b is a natural number chosen from the group of the natural numbers 1, 2, 3 and 4,
- each Z represents a structural element which, independently of possible additional structural elements Z, is chosen from the group consisting of

$-O-(C=O)-CH=CH_2$, $-O-(C=O)-C(CH_3)=CH_2$,

$-(C=O)-CH=CH_2$, $-(C=O)-C(CH_3)=CH_2$

$-CH=CH_2$, $-C(C_H3)=CH_2$ and $-O-CH=CH_2$,

- each index e is a natural number which, independently of possible additional indices e, is chosen from the group of the natural numbers 1, 2, 3 and 4,
- each index x represents, independently of possible additional indices x, 0 or 1,
- each Y represents, in the structure $Q(Y_xZ_e)_b$ with x = 1, a structural element which bonds the polyalicyclic structural element Q with e structural elements Z, in which each Y is chosen independently of possible additional structural elements Y.

2. Composition for use according to Claim 1, in which the total amount of the organic bonding system (b) comprises

(b2) one, two or more polymerizable monomers, in which the polymerizable monomer(s) are not any compounds (monomers) of the structure $Q(Y_xZ_e)_b$ defined above.

3. Composition for use according to either of the preceding claims, **characterized in that** one, two or more compounds of the structure $Q(Y_xZ_e)_b$ of the monomer component (b1) exhibit a tricyclo[5.2.1.0$^{2,6}$]decane or tricyclo[5.2.1.0$^{2,6}$]decene structural element and Z is preferably chosen from the group consisting of $-O-(C=O)-CH=CH_2$ and $-O-(C=O)-C(CH_3)=CH_2$; preferably, Z represents the $-O-(C=O)-C(CH_3)=CH_2$ group.

4. Composition for use according to one of the preceding claims, **characterized in that** component (b1) comprises or consists of bis(methacryloyloxymethyl)-tricyclo[5.2.1.0$^{2,6}$]decane and/or bis(acryloyloxymethyl)tricyclo[5.2.1.0$^{2,6}$]decane.

5. Composition for use according to one of the preceding claims, in which the one monomer or one, more or all of the several polymerizable monomers of the monomer component (b2) is or are chosen from the group consisting of triethylene glycol dimethacrylate (TEDMA), urethane dimethacrylate (7,7,9-trimethyl-4,13-dioxo-3,14-dioxa-5,12-diazahexadecane-1,16-diyl dimethacrylate (UDMA)), glycerol dimethacrylate (GlyDMA) and bisphenol A-glycidyl methacrylate (Bis-GMA).

**6.** Composition for use according to one of the preceding claims, in which the composition is so arranged that it can be cured to give a sealing material which exhibits an abrasion, determined according to the 3-media abrasion method according to ACTA, of less than 80 μm, preferably of less than 70 μm, particularly preferably of less than 60 μm.

**7.** Composition for use according to one of the preceding claims, which can be prepared by a process with the following stages:

(a) making available or preparing a colloidal solution of inorganic particles in water,
(b) exchanging the water against an organic solvent which is completely miscible with water,
(c) before or after stage (b), modifying the surface of the inorganic particles so that particles result which are surface-modified by means of organic structural elements, in which the organic structural elements are convertible with the monomers with formation of covalent bonds,
(d) partially or completely exchanging the organic solvent which is completely miscible with water against the organic bonding system comprising one or more polymerizable monomers, so that the composition according to one of the preceding claims results.

**8.** Composition according to one of the preceding claims, additionally comprising a fluorescent dye.

**9.** Composition according to one of the preceding claims, for use in a process for the determination, by means of fluorescence, of changes in the infiltration condition or in the sealing.

**10.** Method for the recording of data of a fluorescence measurement on a tooth, with the following stages:

- irradiating an infiltrated and/or sealed tooth surface with an excitation radiation which brings about the emission of fluorescence, in which the tooth surface is infiltrated and/or sealed with a composition defined in material terms in one of the preceding claims, in which the infiltrant in the infiltrated tooth surface or the sealing on the tooth surface is so arranged that it is permeable both for the excitation radiation and for the fluorescence emitted,
- recording corresponding fluorescence data.

**11.** Method according to Claim 10, with the following additional stage for the preparation of a caries diagnostics and/or for the determination of changes in the infiltration condition of a tooth and/or for the determination of changes in the sealing on a tooth of a patient:

- comparing the recorded fluorescence data with standard data, without interaction with the patient.

**12.** Method according to Claim 11, in which the comparison is carried out automatically by means of electronic data processing.

**13.** Method according to one of Claims 10 to 12, in which the said stages are repeated with a time interval of a month or more between the irradiation stages.

**14.** Kit (a) for the infiltration and/or sealing of hard tooth substance and (b) (i) for the detection, by means of fluorescence, of caries in the infiltrated region or under the sealing and/or (ii) for the determination of changes in the infiltration condition of a tooth and/or for the determination of changes in the sealing on a tooth of a patient, comprising:

- a composition according to one of Claims 1 to 9,
- an apparatus for the detection of fluorescence emitted from the hard tooth substance and/or the sealing and/or the infiltrant in the infiltrated region.

## Revendications

**1.** Composition comprenant :

(a) un système d’agent de charge consistant en ou comprenant des particules d’agent de charge inorganiques modifiées en surface au moyen d’éléments structuraux organiques, dans laquelle les éléments structuraux organiques peuvent réagir avec un système de liant organique en formant des liaisons covalentes, dans laquelle les particules d’agent de charge de la composition possèdent une taille moyenne de particules dans la plage

entre 1 nm et 150 nm,
(b) un système de liant organique comprenant un ou plusieurs monomères polymérisables,

dans laquelle le système d'agent de charge est dispersé dans le système de liant organique (b),
pour son utilisation dans un procédé pour l'infiltration et/ou le scellement de la matière dentaire dure et pour la détection de caries dans la zone infiltrée ou sous le scellement, par fluorescence,
dans laquelle la quantité totale du système de liant organique (b) comprend
(b1) un, deux ou plusieurs monomères choisis dans le groupe consistant en composés (monomères) de structure $Q(Y_xZ_e)_b$, dans laquelle :

- Q désigne un élément structural polyalicyclique saturé ou oléfiniquement insaturé choisi dans le groupe consistant en radicaux hydrocarbures bicycliques, tricycliques, tétracycliques, pentacycliques et hexacycliques, dans laquelle facultativement, un, deux ou plusieurs des atomes d'hydrogène non substitués par des substituant $Y_xZ_e$ de cet élément structural polyalicyclique Q sont substitués par des groupes alkyle, des groupes alcoxy, des atomes d'halogène ou des groupes trifluorométhyle,
- b est un nombre naturel choisi dans le groupe des nombres naturels 1, 2, 3 et 4,
- chaque Z désigne un élément structural qui est choisi indépendamment d'autres éléments structuraux Z éventuels, dans le groupe consistant en

$$-O-(C=O)-CH=CH_2,\ -O-(C=O)-C(CH_3)=CH_2,$$

$$-(C=O)-CH=CH_2,\ -(C=O)-C(CH_3)=CH_2$$

$$-CH=CH_2,\ -C(CH_3)=CH_2 \text{ et } -O-CH=CH_2,$$

- chaque indice e est un nombre naturel qui est choisi indépendamment d'autres indices e éventuels, dans le groupe des nombres naturels 1, 2, 3 et 4,
- chaque indice x désigne indépendamment d'autres indices éventuels x, 0 ou 1,
- chaque Y désigne dans la structure $Q(Y_xZ_e)_b$, où x = 1, un élément structural qui relie l'élément structural polyalicyclique Q à e éléments structuraux Z, dans laquelle chaque Y est choisi indépendamment d'autres éléments structuraux Y éventuels.

**2.** Composition pour son utilisation selon la revendication 1, dans laquelle la quantité totale du système de liant organique (b) comprend
(b2) un, deux ou plusieurs monomères polymérisables, dans laquelle le ou les monomères polymérisables ne sont pas des composés (monomères) de la structure $Q(Y_xZ_e)_b$ définie précédemment.

**3.** Composition pour son utilisation selon l'une des revendications précédentes, **caractérisée en ce qu'**un, deux ou plusieurs composés de la structure $Q(Y_x2_e)_b$ des composants monomères (b1) présentent un élément structural tricyclo[5.2.1.0$^{2,6}$]-décane ou tricyclo[5.2.1.0$^{2,6}$]-décène et Z est choisi de préférence dans le groupe consistant en $-O-(C=O)-CH=CH_2$ et $-O-(C=O)-C(CH_3)=CH_2$, de préférence Z désigne le groupe $-O-(C=O)-C(CH_3)=CH_2$.

**4.** Composition pour son utilisation selon l'une des revendications précédentes, **caractérisée en ce que** le composant (b1) comprend ou consiste en bis(méthacryloyloxyméthyl)tricyclo[5.2.1.0$^{2,6}$]-décane et/ou bis(acryloyloxyméthyl)tricyclo[5.2.1.0$^{2,6}$]-décane.

**5.** Composition pour son utilisation selon l'une des revendications précédentes, dans laquelle le monomère ou l'un, plusieurs ou tous parmi les multiples monomères polymérisables des composants monomères (b2) est ou sont choisi (s) dans le groupe consistant en triéthylèneglycoldiméthacrylate (TEDMA), uréthane-diméthacrylate (7,7,9-triméthyl-4,13-dioxo-3,14-dioxa-5,12-diazahexadécane-1,16-dioxydiméthacrylate (UDMA)), glycérinediméthacrylate (glyDMA) et bisphénol-A-glycidyl-méthacrylate (bis-GMA).

**6.** Composition pour son utilisation selon l'une des revendications précédentes, dans laquelle la composition est conçue de telle sorte qu'elle puisse être durcie en un matériau de scellement qui présente une abrasion, déterminée selon la méthode d'abrasion à 3 milieux selon l'ACTA, inférieure à 80 $\mu$m, de préférence inférieure à 70 $\mu$m, de manière particulièrement préférée inférieure à 60 $\mu$m.

**7.** Composition pour son utilisation selon l'une des revendications précédentes, pouvant être produite par un procédé

avec les étapes suivantes :

(a) préparation ou production d'une solution colloïdale de particules inorganiques dans l'eau,
(b) échange de l'eau contre un solvant organique complètement miscible avec l'eau,
(c) avant ou après l'étape (b), modification de la surface des particules inorganiques, de sorte qu'il en résulte des particules qui sont modifiées en surface au moyen d'éléments structuraux organiques, dans laquelle les éléments structuraux organiques peuvent réagir avec les monomères en formant des liaisons covalentes,
(d) échange partiel ou complet du solvant organique complètement miscible avec l'eau contre le système de liant organique comprenant un ou plusieurs monomères polymérisables, de sorte qu'il en résulte la composition selon l'une des revendications précédentes.

**8.** Composition selon l'une des revendications précédentes, comprenant en outre un colorant fluorescent.

**9.** Composition selon l'une des revendications précédentes pour son utilisation dans un procédé de détermination de modifications de l'état d'infiltration ou de scellement au moyen de la fluorescence.

**10.** Procédé de saisie de données d'une mesure de fluorescence sur une dent, avec les étapes suivantes :

- irradiation d'une surface dentaire infiltrée et/ou scellée avec un rayonnement d'excitation qui entraîne l'émission de rayonnement fluorescent, dans lequel la surface dentaire est infiltrée et/ou scellée avec une composition définie au niveau de sa substance dans l'une des revendications précédentes, dans lequel l'infiltrant dans la surface dentaire infiltrée ou le scellement sur la surface dentaire est conçu de telle sorte qu'il ou elle soit perméable aussi bien au rayonnement d'excitation qu'au rayonnement fluorescent émis,
- saisie de données de rayonnements fluorescents correspondantes.

**11.** Procédé selon la revendication 10, avec l'étape supplémentaire suivante pour la préparation d'un diagnostic de carie et/ou la détermination de modifications de l'état d'infiltration d'une dent et/ou la détermination de modifications du scellement sur une dent d'un patient, de :

- comparaison des données de rayonnement fluorescent saisies avec des données normales sans interaction avec le patient.

**12.** Procédé selon la revendication 11, dans lequel la comparaison s'effectue automatiquement au moyen d'un traitement électronique des données.

**13.** Procédé selon l'une des revendications 10 à 12, dans lequel lesdites étapes sont répétées à un intervalle temporel d'un mois ou plus entre les étapes de rayonnement.

**14.** Kit (a) pour l'infiltration et/ou le scellement de matière dentaire et (b) (i) pour la détection de caries dans la zone infiltrée ou sous le scellement, au moyen d'une fluorescence et/ou (ii) pour la détermination de modifications de l'état d'infiltration d'une dent et/ou pour la détermination de modifications du scellement sur une dent d'un patient, comprenant :

- une composition selon l'une des revendications 1 à 9,
- un dispositif de détection du rayonnement fluorescent émis à partir de la matière dentaire et/ou du scellement et/ou de l'infiltrant dans la zone infiltrée.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 7796243 B2
- US 20100227296 A1
- US 20070021670 A1
- DE 3031249 C2
- DE 4200741 C2
- DE 9317984 U1
- US 7596253 B
- DE 29704185 U1
- DE 19541686 B4
- DE 19825021 A1
- DE 19709500 C1
- DE 20209441 U1
- DE 10227128 A1
- DE 60316699 T2
- DE 19619067
- DE 102005052294 A1
- DE 9417470 U1
- DE 10133451 A1
- DE 2301067
- US 6573312 B2
- US 20050288387 A1
- US 20090047633 A1
- EP 0969789 B1
- US 6899948 B2
- US 6572693 B1
- WO 2007028159 A2
- WO 0130307 A1
- US 20060063853 A1
- US 20070166450 A1
- US 20100016464 A1
- WO 2005094757 A1
- EP 1307173 B1
- DE 202006020483 U1
- DE 202006020480 U1
- DE 202006020479 U1
- DE 202006020477 U1
- DE 202006020476 U1
- EP 2023884 A1
- EP 1854445 A1
- EP 2145613 A1
- EP 2151229 A2
- US 5936006 A
- US 6194481 B
- US 6593395 B2
- DE 2405578 A1
- US 20020065337 A
- DE 19508586 A1
- WO 0069392 A
- US 6387981 B
- DE 60012775 T2
- DE 102006044520 A1
- EP 2110414 A1
- JP 7206740 A
- EP 1112995 B1
- EP 0049631 B1
- DE 10352260 B3
- EP 0023686 B1
- DE 3522006 A1
- DE 3338077 A1
- EP 0143613 B1
- DE 3703120 A1
- WO 2009065873 A2
- EP 0209700 A2
- DE 3941629 A1
- DE 19903177
- DE 4416857
- EP 0074708 A
- EP 1084131 A
- US 4514342 A
- DE 2828381
- US 4148988 A
- EP 0684033 A
- EP 0684034 A
- EP 1148060 A
- EP 0909761 A
- EP 1148071 A
- EP 1346717 A
- EP 1721949 A1
- DE 102006019092 A1
- DE 3941629 C2
- DE 102006019092
- DE 60116142
- US 4772530 A
- US 4954414 A
- US 4874450 A
- US 5055372 A
- US 5057393 A
- EP 1720506 A
- EP 1839640 A
- DE 1495520
- WO 02092021 A
- WO 02092023 A
- EP 0059451 A
- EP 0783880 B1
- DE 10119831 A1
- EP 1563821 A1

**In der Beschreibung aufgeführte Nicht-Patentliteratur**


- **S. ALBIN et al.** Laser Induced Fluorescence of Dental Caries. Proc SPIE, 1988, vol. 907, 96-98
- **E. DE JOSSELIN DE JONG et al.** A new Method for in vivo Quantification of Changes in Initial Enamel Caries with Laser Fluorescence. *Caries Research,* 1995, 2-7
- Effects of composite fissure sealants on IR laser fluorescence measurements. *Lasers Med Sci,* 2008, vol. 23, 133-139
- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II
- **REINHARDT KJ.** *Phillip Journal,* 1997, vol. 3-4, 101-104
- *J. Dent. Suppl.,* 1994, vol. 1 (22), 21-27